(19)

Europäisches
Patentamt

European
Patent Office

Office européen
des brevets

(11) **EP 4 549 591 A1**

(12) **EUROPEAN PATENT APPLICATION**
published in accordance with Art. 153(4) EPC

(43) Date of publication:
**07.05.2025 Bulletin 2025/19**

(21) Application number: **23831932.1**

(22) Date of filing: **29.06.2023**

(51) International Patent Classification (IPC):
*C12Q 1/70* (2006.01)          *C12Q 1/6806* (2018.01)
*C12N 15/10* (2006.01)

(52) Cooperative Patent Classification (CPC):
**C12N 15/10; C12N 15/86; C12Q 1/6806; C12Q 1/70**

(86) International application number:
**PCT/KR2023/009153**

(87) International publication number:
**WO 2024/005574 (04.01.2024 Gazette 2024/01)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **29.06.2022 KR 20220080073**

(71) Applicants:
• **Seoul National University R&DB Foundation
Seoul 08826 (KR)**
• **Institute for Basic Science
Yuseong-gu
Daejeon 34126 (KR)**

(72) Inventors:
• **KIM, Vic Narry
Seoul 08826 (KR)**
• **SEO, Jenny
Seoul 05501 (KR)**
• **JUNG, Soo-Jin
Seoul 05268 (KR)**

(74) Representative: **AWA Sweden AB
Matrosgatan 1
Box 5117
200 71 Malmö (SE)**

Remarks:
The complete document including Reference
Table(s) and the Sequence Listing(s) can be
downloaded from the EPO website

(54) **METHOD FOR SCREENING REGULATORY ELEMENT FOR INCREASING MRNA TRANSLATION, NOVEL REGULATORY ELEMENT RESULTING FROM METHOD, AND USE THEREOF**

(57)    The present disclosure relates to a method of screening a regulatory element for enhancing mRNA translation, a novel regulatory element resulting from the method, and uses thereof. Through the screening method of the present disclosure, a novel regulatory element capable of enhancing mRNA translation may be obtained. Furthermore, the novel regulatory element may increase the expression of a target protein and as such, may be applied to various fields, depending on the intended use of the target protein.

EP 4 549 591 A1

【FIG. 2】

## Description

### Technical Field

The present disclosure relates to a method of screening a regulatory element for enhancing mRNA translation, a novel regulatory element resulting from the method, and uses thereof.

### Background Art

Viruses have evolved diverse mechanisms to hijack cellular gene expression machinery, and research in this area has contributed greatly to advances in RNA biology and biotechnology. For instance, the 7-methyl guanosine cap, internal ribosome entry site, and RNA triple helix were first discovered from reovirus, poliovirus, and Kaposi's sarcoma-associated herpesvirus, respectively. Human immunodeficiency virus (HIV) is known to utilize the transactivation response region (TAR) and the rev-response element (RRE) to recruit cellular factors for viral transcription and RNA export, respectively (Vaishnav, et al., New Biol., 1991, 3, 142-150; Dingwall, et al., EMBO J., 1990, 9, 4145-4153). Hepatitis B virus (HBV) relies on its post-transcriptional regulatory element (PRE) to bring host nucleotidyl transferases, which stabilize viral transcripts (Kim, et al., Nat. Struct. Mol. Biol., 2020, 27, 581-588; Huang, et al., Mol. Cell. Biol., 1993, 13, 7476-7486).

However, these discoveries were made through low-throughput analyses of pathogenic viruses, which represent only a small fraction of the entire virome. To date, 6,828 viral species have been named, and the NCBI Genome database contains 14,775 complete viral genome sequences (O'Leary, et al., Nucleic Acids Res., 2016, 44, D733-D745). Recent metagenomics studies based on deep sequencing have detected hundreds of thousands of additional viral sequences from environmental and animal samples (Neri, et al., Cell, 2022, 185, 4023-4037). Despite the vast number of available sequences, those without clinical or industrial relevance remain largely unexplored. Therefore, the rapidly growing collection of viral sequences presents a significant challenge for functional annotation, demanding more effective strategies to interpret viral sequence data.

### Detailed Description of the disclosure

### Technical Problem

The present inventors developed a method for screening regulatory elements for enhancing mRNA translation using viral sequence data, and used this method to discover novel regulatory elements, and uses thereof.

### Technical Solution to Problem

An objective of the present disclosure is to provide a method of screening a regulatory element for enhancing RNA stability and/or mRNA translation.

Another objective of the present disclosure is to provide a regulatory element for enhancing RNA stability and/or mRNA translation.

Another objective of the present disclosure is to provide a construct, vector, or recombinant host cell, which includes a gene of a target protein and the regulatory element, preferably located in a 3' UTR of the gene.

Another objective of the present disclosure is to provide a composition including the construct, vector, or recombinant host cell.

Another objective of the present disclosure is to provide a method of preparing a target protein, the method including: culturing the recombinant host cell; and separating a target protein.

Another objective of the present disclosure is to provide a method of preparing an mRNA construct, the method including: in vitro transcribing a construct by using the construct or vector as a template; and recovering a transcribed mRNA construct.

Another objective of the present disclosure is to provide a use of the construct, vector, recombinant host cell, or composition for enhancing RNA stability and/or mRNA translation.

Another objective of the present disclosure is to provide a use of the construct, vector, recombinant host cell, or composition for preventing or treating a disease.

Another objective of the present disclosure is to provide a use of the construct, vector, recombinant host cell, or composition for preparing an mRNA construct or a target protein.

## Advantageous Effects of Disclosure

Through the screening method of the present disclosure, a novel regulatory element capable of enhancing mRNA translation may be obtained. Furthermore, the novel regulatory element may increase the expression of a target protein and as such, may be applied to various fields, depending on the intended use of the target protein

## Brief Description of Drawings

FIGS. 1A to 1E relate to a viromic screen for identifying regulatory RNA elements.

FIG. 1A shows the total species count and average genome size after screening viruses capable of infecting humans. The total species count and average genome size of each family are indicated by gray bars. The total species count and the average portion of the genome covered in the library are indicated by colored bars.

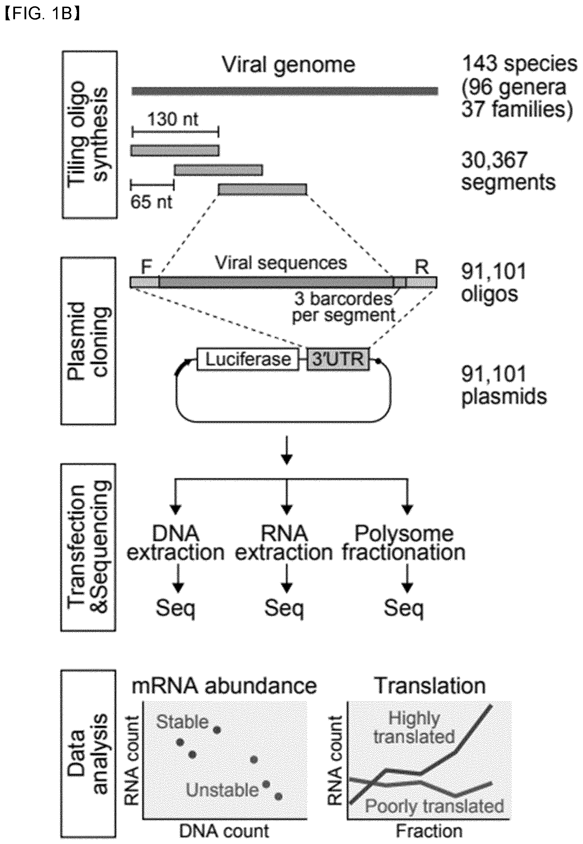

FIG. 1B is a schematic representation of the experimental design and procedure for the viromic screen. A total of 30,367 segments, each 130-nt in length, were selected in 65-nt tiling steps and linked with three different barcodes, generating 91,101 oligos in total. The oligos were cloned into the 3' UTR of the firefly luciferase construct. Next, the pool of plasmids was transfected into HCT116 cells. To quantify the RNA stability effects, reporter DNA and RNA were extracted, amplified by PCR, and sequenced. For polysome profiling, five fractions were collected using sucrose gradient centrifugation, and the reporter RNAs were sequenced.

FIG. 1C is a graph showing RNA abundance ranked by order. The RNA abundance score was calculated as the log2 ratio (the read fraction of RNA divided by the read fraction of DNA). Positive controls (HCMV 1E, WPRE), negative controls (HCMV 1Em), a self-cleaving ribozyme from hepatitis delta virus, and viral miRNAs are indicated.

FIG. 1D shows the polysome profiling results of viral reporter mRNAs. The colors indicate the relative abundance of RNA in each fraction. Twenty clusters were generated using hierarchical clustering and sorted by the read ratio between heavy polysome and free mRNA.

FIG. 1E shows the RNA distribution patterns in representative clusters.

FIG. 2 relates to the validation of viral regulatory elements.

FIG. 2A is a graph comparing the effects on RNA abundance (X-axis) and translation (Y-axis).

FIG. 2B investigates the validity of 16 selected segments through luciferase activity. K1-K16 (indicated by light blue dots in FIG. 2A) were individually cloned into dual-luciferase reporters. Ctrl indicates the reporter without the K elements and was used for normalization. Data are represented as mean $\pm$ standard error of the mean (SEM) (n = 8 biological replicates). * indicates $p < 0.05$, ** indicates $p < 0.01$, with a two-tailed Student's t-test performed.

FIG. 2C shows the genomic structure of Saffold virus (NC_009448.2, left) and Aichi virus 1 (NC_001918.1, right) and the genome coordinates of the K4 and K5 elements represented on each virus.

FIG. 2D shows luciferase activity from the UTR reporters. * indicates $p < 0.05$, with a two-sided Student's t-test performed.

FIG. 2E shows luciferase activity from truncated K5 reporters, with 120-K5 (8132-8251, 120-nt) and 110-K5 (8142-8251, 110-nt) representing truncated forms of K5. Data are represented as mean $\pm$ SEM (n = 3). * indicates $p < 0.05$, with a two-sided Student's t-test performed.

FIGS. 3A to 3E pertain to characteristics of K5 element.

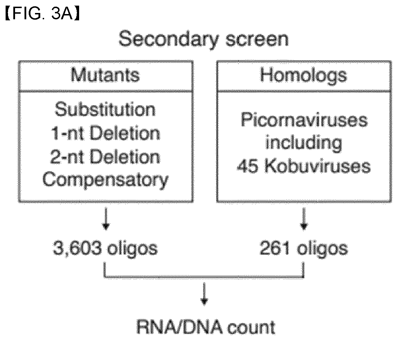

FIG. 3A shows a schematic diagram of the secondary screen covering the K5 variants and homologs. The homologous elements were derived from the 3' terminal 130-nt segments of 88 picornaviruses. RNA stability was measured as in FIG. 1B.

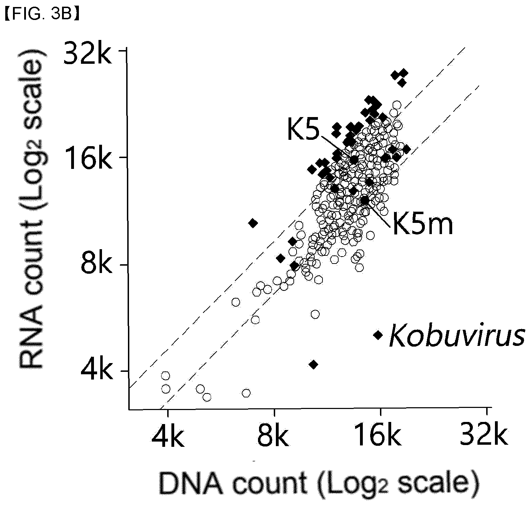

FIG. 3B presents results from the secondary screen. DNA count (X-axis) and RNA count (Y-axis) were measured by sequencing. K5 (red), K5m (dark red), and its homologous segments from kobuviruses (pink) are indicated.

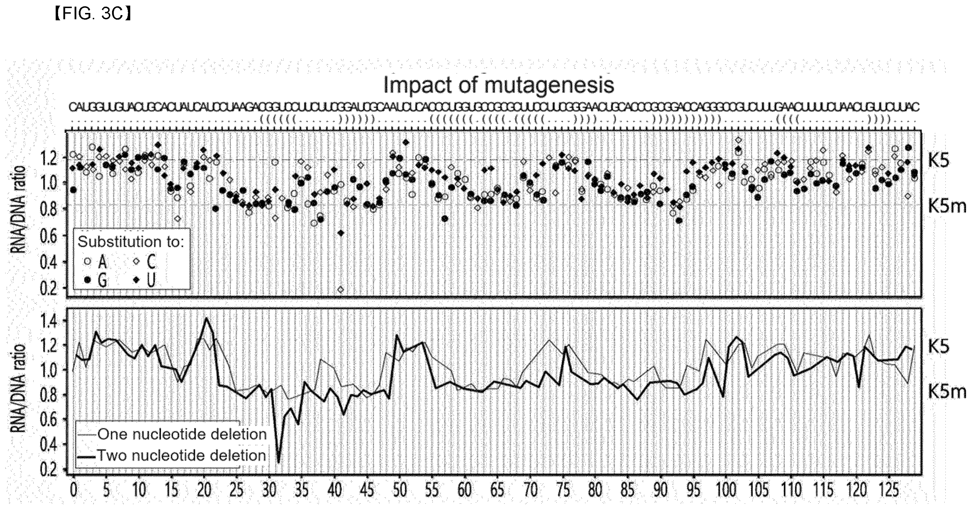

FIG. 3C shows results from the secondary screen using the mutants of K5, showing the RNA/DNA ratio measured with substitution mutants (top) and the ratio quantified after one or two nucleotide deletions (bottom). RNA/DNA ratio of the results from K5 and K5m is indicated by horizontal lines. Data are represented as mean $\pm$ SEM error bars for substitution and shading for deletion (n = 3).

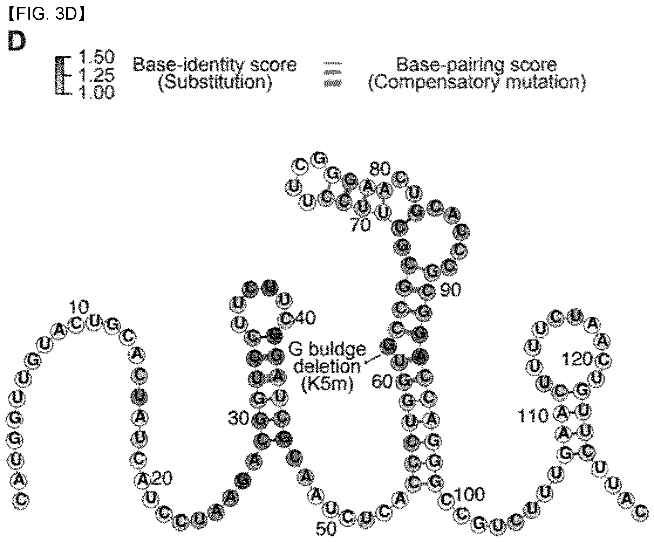

FIG. 3D shows a predicted secondary structure of K5. The base-identity score (indicated in magenta) and base-pairing score (indicated by the width of the blue lines between the paired bases) were measured from the secondary screen.

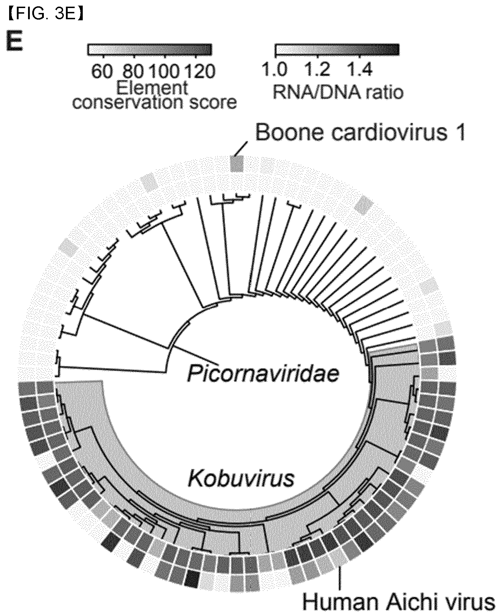

FIG. 3E depicts a cladogram of the Picornaviridae 3' UTR sequences used in the screen. The Kobuvirus genus is highlighted with a red shade. The element conservation score (red boxes) indicates the degree of sequence homology to the K5 element from human Aichi virus. The RNA stabilizing effect is presented with green boxes.

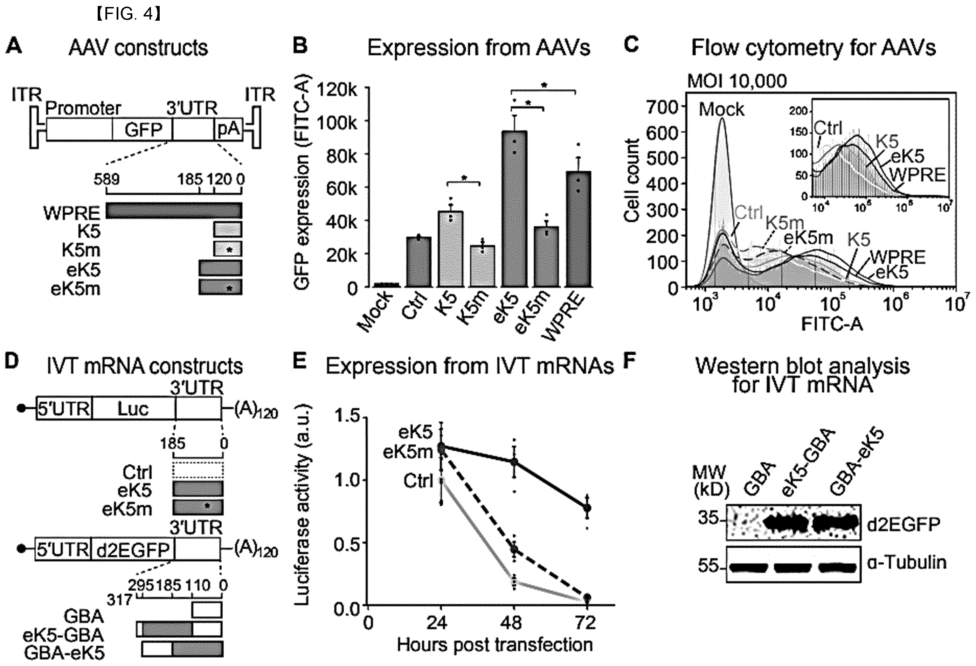

FIG. 4 demonstrates that K5 enhances gene expression from AAV vectors and synthetic mRNA.

FIG. 4A shows a schematic of the AAV constructs containing the K5 element or WPRE. The deletion of a G-bulge, which impairs K5 activity, is indicated with an asterisk.

FIG. 4B shows GFP expression from the rAAV constructs containing K5 or WPRE, transduced to HeLa cells at 10,000 moi. Data are normalized by the mock value and represented as mean $\pm$ SEM (n = 3). * indicates p < 0.05, with a two-sided Student's t-test performed.

FIG. 4C shows the expression of GFP in HeLa cells infected with rAAVs, confirmed by flow cytometry.

FIG. 4D provides a schematic of the firefly luciferase-encoding IVT mRNAs with or without eK5 and its mutants (top) and d2EGFP IVT mRNA constructs harboring the alpha-globin UTR (GBA) and/or K5 (bottom).

FIG. 4E shows luciferase expression from synthetic mRNAs transfected to HeLa cells. Data are normalized by the Ctrl (24 hpt) value and represented as mean $\pm$ SEM (n = 3). * indicates p < 0.05, with a two-sided Student's t-test performed.

FIG. 4F shows the results of western blotting performed on HeLa cells transfected with the d2EGFP mRNA reporters at 72 hour post-transfection.

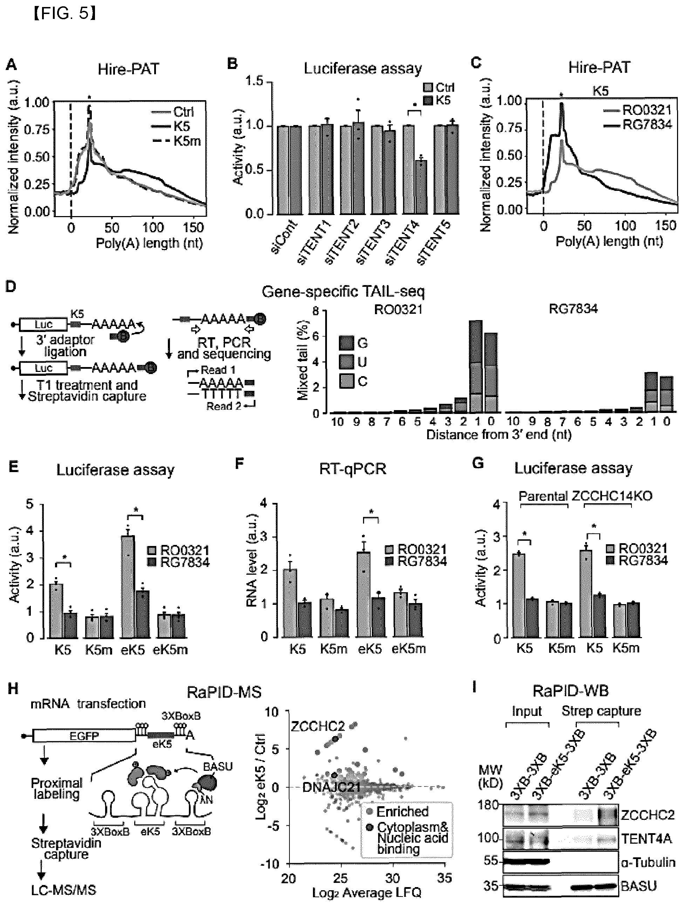

FIG. 5 shows that K5 induces mixed tailing by TENT4.

FIG. 5A depicts poly(A) length distribution measured by Hire-PAT. The normalized intensity (arbitrary unit, a.u.) represents the percentile of the reads, which applies to all subsequent Hire-PAT analyses. HeLa cells were transfected with the control, K5 reporter, or its mutant K5m plasmid. A side product of PCR serving as a size marker is indicated by an asterisk.

FIG. 5B shows the knockdown effects of terminal nucleotidyl transferases on K5 activity as measured by luciferase expression from the control and K5 reporter. Note that closely related paralogs were depleted together for TENT3 (TENT3A/TUT4/ZCCHC11 and TENT3B/TUT7/ZCCHC6), TENT4 (TENT4A/PAPD7/TRF4-1/TUT5 and TENT4B/-PAPD5/TRF4-2/TUT3), and TENT5 (TENT5A, TENT5B, TENT5C, and TENT5D). Data are normalized by the control siRNA (siCont) value for each reporter construct and represented as mean $\pm$ SEM (n = 3). * indicates p < 0.05, with a two-sided Student's t-test performed.

FIG. 5C shows the Poly(A) length distribution of K5 reporter mRNAs measured by Hire-PAT. HeLa cells were treated with the TENT4 inhibitor RG7834 or its R-isomer RO0321. A side product of PCR is indicated by an asterisk.

FIG. 5D depicts gene-specific TAIL-seq used to count non-adenosine residues within the 3' end positions of poly(A) tails of the K5-containing reporter in HeLa cells. The mixed tailing percentage of each position is represented by the distance from the 3' end.

FIG. 5E shows luciferase activity in HeLa cells transfected with the K5 and eK5 plasmids in the presence of RO0321 or RG7834. Data are normalized against the reporter without the K5 element (Ctrl) at each condition and represented as mean $\pm$ SEM (n = 3). * indicates p < 0.05, with a two-sided Student's t-test performed.

FIG. 5F presents the RT-qPCR results of HeLa cells transfected with the K5 and eK5 plasmids in the presence of RO0321 or RG7834. Data are normalized against the reporter without the K5 element (Ctrl) at each condition and represented as mean $\pm$ SEM (n = 3). * indicates p < 0.05, with a two-sided Student's t-test performed.

FIG. 5G shows the results of luciferase assay of K5 reporters in HCT116 parental cells and ZCCHC14 KO cells. Data are normalized by the reporter without the K5 element (Ctrl) at each condition and represented as mean $\pm$ SEM (n = 3). * indicates p < 0.05, with a two-sided Student's t-test performed.

FIG. 5H presents the results of mass-spectrometry analysis following the RaPID (RNA-protein interaction detection) experiment with eK5. The 3xBoxB sequence without the eK5 element was used as a negative control. Light blue dots indicate proteins enriched in two or more replicates (log2FC > 1). A pseudovalue of 100,000 was added to missing LFQ values. DNAJC21 and ZCCHC2 are proteins with cytoplasmic localization and nucleic acid GO term.

FIG. 5I shows the results of western blot following the RaPID (RNA-protein interaction detection) experiment with eK5. The 3xBoxB sequence without the eK5 element was used as a negative control. A pseudovalue of 100,000 was added to missing LFQ values. DNAJC21 and ZCCHC2 are proteins with cytoplasmic localization and nucleic acid GO term.

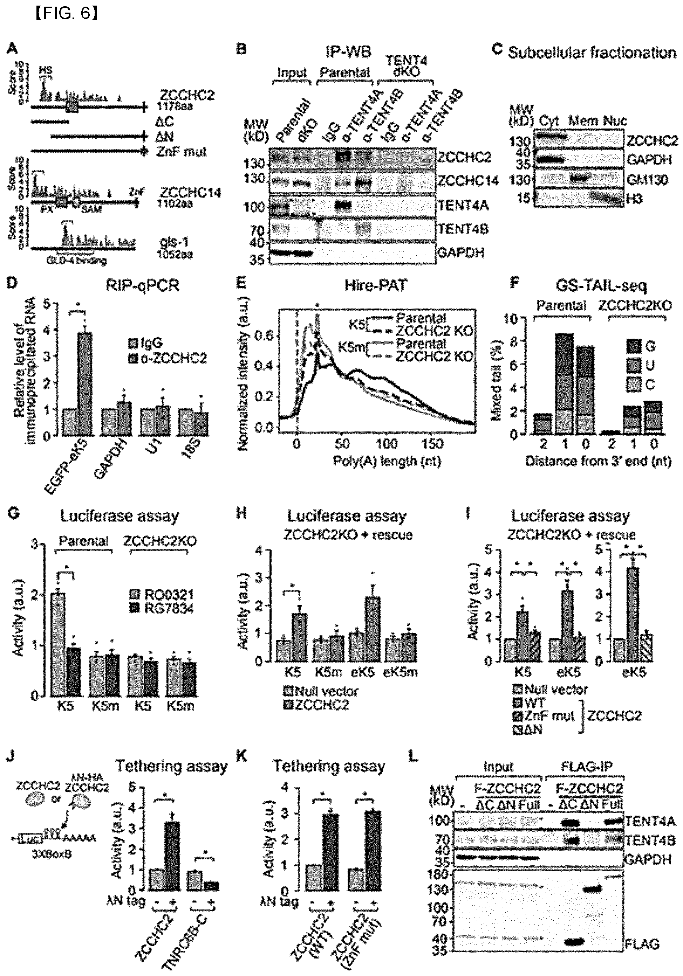

FIG. 6 illustrates the function of ZCCHC2 as a host factor for K5.

FIG. 6A shows the domain structure of ZCCHC2 in comparison with ZCCHC14 and C. elegans gls-1. The amino acid similarity score calculated among the three proteins is indicated above each domain structure. The region of highest similarity among these proteins is indicated with red brackets. The ZCCHC2 mutants, $\Delta$C (1-375 aa), $\Delta$N (201-1,178 aa), and ZnF mutants used in FIG. 6I, K, and L are also shown below the ZCCHC2 structure.

FIG. 6B depicts the interaction between ZCCHC2 and TENT4, demonstrated by co-immunoprecipitation with anti-TENT4A and anti-TENT4B in the presence of RNase A using lysates from HeLa parental and TENT4 double KO cells. Proteins were visualized by western blotting. ZCCHC14 and TENT4A were analyzed on different gels with the same amounts of samples. Cross-reacting bands are indicated by asterisks.

FIG. 6C shows the localization of ZCCHC2, examined by subcellular fractionation followed by western blotting with the corresponding antibodies. GM130 was analyzed on a different gel with the same amounts of samples.

FIG. 6D presents the RT-qPCR results after immunoprecipitation with anti-ZCCHC2 antibody in HeLa cells stably expressing the EGFP mRNA with eK5 in its 3' UTR. Immunoprecipitation with normal rabbit IgG was used for a control and normalization. The EGFP-eK5 mRNA was specifically precipitated with anti-ZCCHC2 antibody, unlike other RNAs (GAPDH, U1 snRNA, and 18S rRNA). Data are normalized against the EGFP-eK5 (IgG) qPCR value and represented as mean $\pm$ SEM (n = 3). * indicates p < 0.05, with a two-sided Student's t-test performed.

FIG. 6E shows the Poly(A) tail length distribution of K5 and K5m reporter mRNAs as measured by Hire-PAT assay in HeLa parental cells and HeLa ZCCHC2 KO cells. A side product of PCR serving as a size marker is indicated by an asterisk.

FIG. 6F shows the non-adenosine frequency within the 3' last three positions of poly(A) tails of the K5 reporter mRNAs in HeLa parental cells and ZCCHC2 KO cells, as measured by gene-specific TAIL-seq.

FIG. 6G shows luciferase expression in parental HeLa cells and ZCCHC2 KO cells transfected with the K5 reporters. Cells were treated with the TENT4 inhibitor RG7834 or its R-isomer RO0321. Data are normalized against the reporter without the K5 element (Ctrl) at each condition and represented as mean $\pm$ SEM (n = 3). * indicates p < 0.05, with a two-sided Student's t-test performed.

FIG. 6H shows the structure of HeLa ZCCHC2 KO cells with ectopic expression of wild-type ZCCHC2. Data are normalized against the reporter without the K5 element (Ctrl) at each condition and represented as mean $\pm$ SEM (n = 3). * indicates p < 0.05, with a two-sided Student's t-test performed.

FIG. 6I shows the structure of wild-type ZCCHC2, ZCCHC2 zinc-finger mutant, and ZCCHC2 $\Delta$N construct. Data are normalized against the reporter without the K5 element (Ctrl) at each condition and represented as mean $\pm$ SEM (n=4 (left), n=3 (right)).

FIG. 6J presents the results of tethering assay in which the ZCCHC2 protein with or without a $\lambda$N tag was co-expressed with 3xBoxB luciferase reporter mRNA in HeLa cells. The C-terminal silencing domain (716-1,028 amino acids) of TNRC6B protein was used as a control.

Data are normalized against the value of the wild-type sample and represented as mean $\pm$ SEM (n = 3). * indicates p < 0.05, with a two-sided Student's t-test performed.

FIG. 6K shows the results of tethering assay in which the ZCCHC2 zinc-finger mutant was active being artificially tethered to the reporter mRNA. The ZCCHC2 zinc-finger mutant was active when it was artificially tethered to the reporter mRNA. Data are normalized against the value of the wild-type sample and represented as mean $\pm$ SEM (n = 3). * indicates p < 0.05, with a two-sided Student's t-test performed.

FIG. 6L shows the results where FLAG-tagged ZCCHC2 proteins (F-ZCCHC2) were transiently expressed in HeLa ZCCHC2 knockout cells, immunoprecipitated with an anti-FLAG antibody, and analyzed by western blotting. Full-length ZCCHC2 protein and its truncated mutants ($\Delta$C, $\Delta$N) were compared for their ability to interact with TENT4 proteins. TENT4A and GAPDH were detected on the same gel, whereas the other proteins were analyzed on separate gels with the same amounts of samples. Cross-reacting bands are indicated by asterisks.

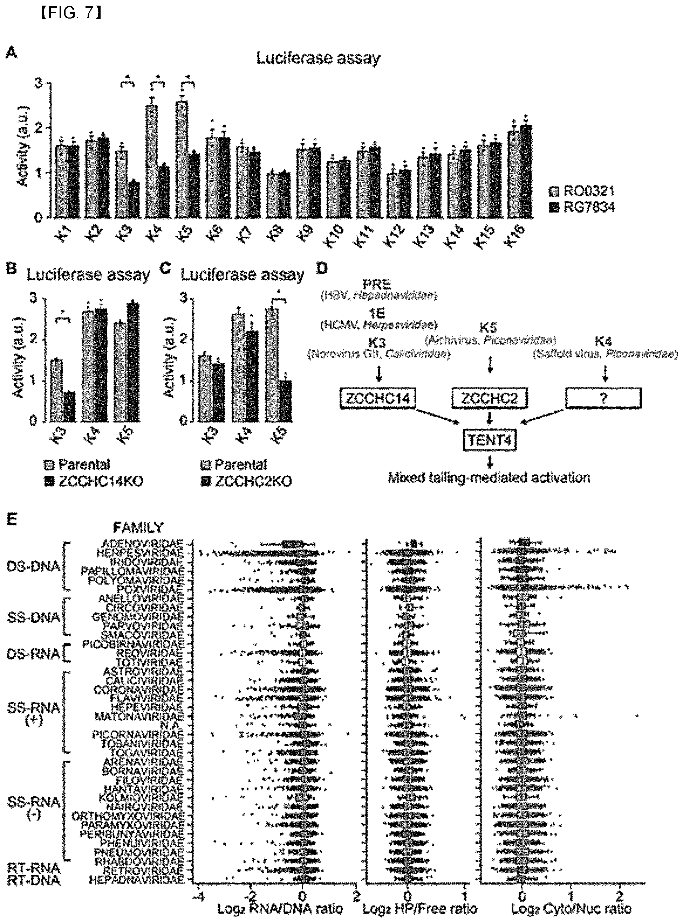

FIG. 7 shows a broad distribution of regulatory RNAs across the virosphere.

FIG. 7A shows a luciferase reporter assay for the K1 to K16 elements in HCT116 cells in the presence of RO0321 or RG7834.

FIG. 7B presents the results of a luciferase assay performed on parental HCT116 cells and ZCCHC14 KO cells transfected with the K3, K4, and K5 reporters. Data are normalized against the reporter without K5 (Ctrl) at each condition and represented as mean $\pm$ SEM (n = 3). * indicates p < 0.05, with a two-sided Student's t-test performed.

FIG. 7C provides the results of a luciferase assay performed on parental HeLa cells and ZCCHC2 KO cells transfected with K3, K4, and K5 reporters. Data are normalized against the reporter without K5 (Ctrl) at each condition and represented as mean $\pm$ SEM (n = 3). * indicates p < 0.05, with a two-sided Student's t-test performed.

FIG. 7D provides a schematic model of viruses exploiting mixed tailing. PRE, 1E, and K3 were from HBV, HCMV, and Norovirus, respectively, and depended on ZCCHC14 to recruit TENT4. K4 from Saffold virus relied on TENT4 but was independent of ZCCHC14 and ZCCHC2.

FIG. 7E shows a broad distribution of RNA elements controlling RNA abundance (left), translation (middle), and subcellular localization (right) in viral families.

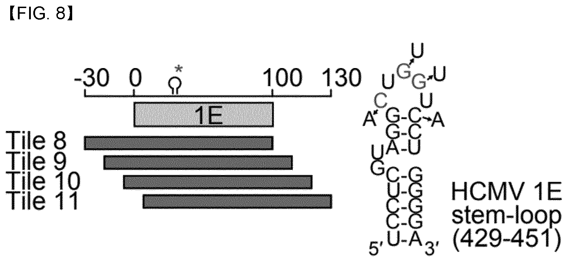

FIG. 8 is a schematic of the tiles containing the HCMV 1E element and loop mutations.

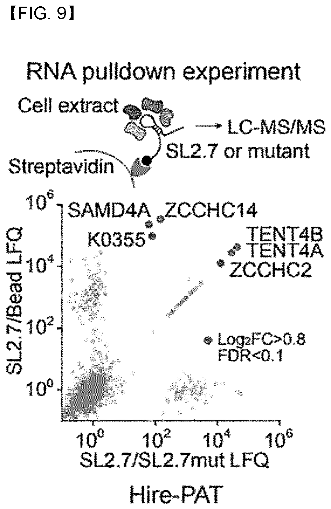

FIG. 9 shows the results of mass spectrometry analysis performed after RNA pull-down using SL2.7 RNA as a bait that recruits the TENT4-ZCCHC14 complex. The SL2.7 mutant (X-axis) and the "bead only" controls (Y-axis) were used for normalization. Blue dots indicate proteins significantly enriched in SL2.7 samples (Log2FC > 0.8 and FDR < 0.1). A pseudovalue of 100,000 was added to missing LFQ values. HEK293T cell lysate was used for the RNA pull-down (n = 2). The SAMD4 proteins bind to the RNA through their SAM domains but do not have an enhancing activity on SL2.7. K0355 is known to interact with SAMD4B.

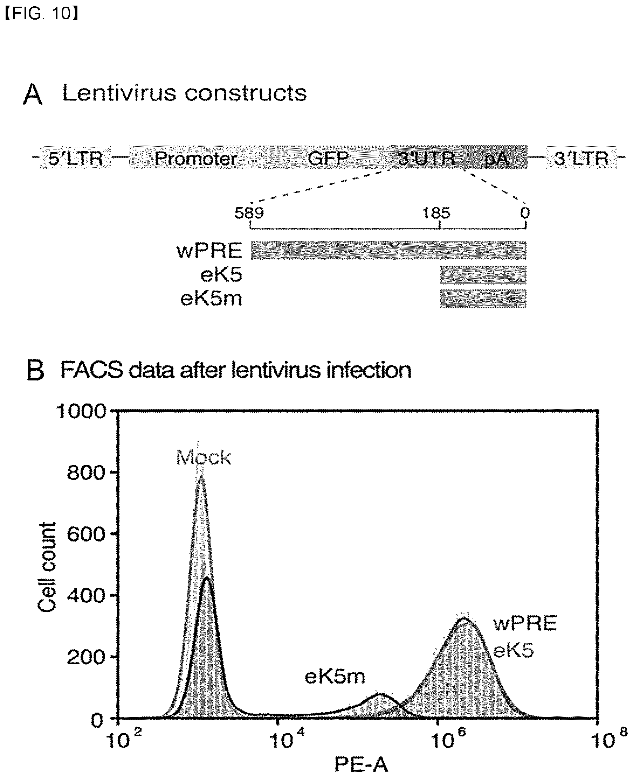

FIG. 10 demonstrates that K5 enhances gene expression from lentiviral vectors and synthetic mRNA.

FIG. 10A provides a schematic of a lentiviral construct containing the K5 element or WPRE. The deletion of a G-bulge, which impairs K5 activity, is indicated with an asterisk.

FIG. 10B shows the expression of GFP in HeLa cells infected with the lentivirus, confirmed by flow cytometry.

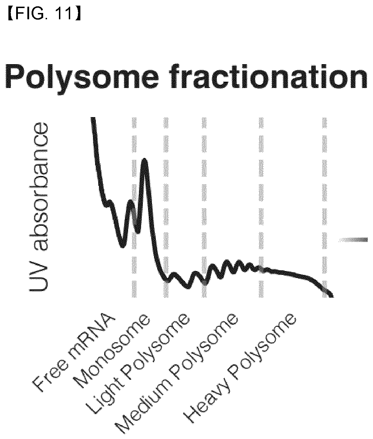

FIG. 11 shows a polysome fractionation graph.

**Best Mode for Disclosure**

Each description and embodiment disclosed in the present application may be applied to other descriptions and embodiments presented herein. In other words, all combinations of the various elements disclosed herein fall within the scope of the present application. Moreover, the scope of the present application shall not be considered limited by any specific descriptions provided below. Moreover, a person of ordinary skill in the art would be able to recognize or identify numerous equivalents to the specific aspects of the present application only through routine experimentation. Such equivalents are intended to be encompassed within the scope of the present application.

An aspect of the present disclosure relates to a method of screening a regulatory element for enhancing RNA stability and/or mRNA translation. The screened regulatory element may enhance RNA stability and/or mRNA translation, thereby increasing the expression of a target protein.

Specifically, the method may be a method of screening a regulatory element for enhancing RNA stability and/or mRNA translation and include:

(a) preparing a plurality of oligonucleotides by tiling a viral genome;
(b) preparing a pool of vectors, each including one of the oligonucleotides, wherein each vector includes a reporter gene and includes one of the oligonucleotide in a 3' UTR thereof;
(c) introducing each vector into a cell;
(d) fractionating the polysomes of the cell into free mRNA, monosome, light polysome (LP), medium polysome (MP), and heavy polysome (HP), performing sequencing, and calculating, for each oligonucleotide, a value of Equation (1) and a mean ribosome load (MRL):

$$\text{Equation (1)} = \text{Log2 (HP / free mRNA)}$$

$$\text{-Mean ribosome load (MRL)} =$$

$$1 \times p(\text{Monosome}) + 2.5 \times p(\text{LP}) + 6 \times p(\text{MP}) + 11 \times p(\text{HP})$$

where p(X) is a proportion of sequencing reads for each fraction X, and
(e) selecting, as a regulatory element for enhancing mRNA translation, an oligonucleotide for which the value of Equation (1) exceeds 0.2 and the MRL exceeds 4.5.

The viral genomes used in the present application may be obtained from known databases (e.g., NCBI).

The tiling in the process (a) may be a method used in the art to analyze genomic characteristics, which involves dividing the genomic sequence into segments of a certain size (sliding window) to generate a plurality of segments, wherein the window is shifted by a specific displacement (shift) size from the first position of the previous segment to create each subsequent segment. For example, the size of the sliding window may be 100 nt to 500 nt, and the displacement may be 1 nt to 500 nt, but are not limited thereto. The sizes of the sliding window and the displacement may be appropriately selected those skilled in the art.

One or more barcode sequences may be added to the plurality of segments. Specifically, by adding one barcode sequence from each of two or more different types downstream of a single segment, two or more oligonucleotides may be generated per segment. That is, in the present disclosure, the plurality of oligonucleotides may include one or more barcode sequences.

In process (b), the plurality of oligonucleotides may be individually introduced into a vector, thereby producing a plurality of vectors (i.e., a pool of vectors). At this stage, the oligonucleotides may be introduced into the 3' UTR of the reporter gene within the vector.

In the present disclosure, the reporter may be luciferase, a fluorescent protein, β-galactosidase, chloramphenicol acetyltransferase, or aequorin, but is not limited thereto.

In the present disclosure, methods for introducing vectors into cells encompass any method of introducing nucleic acids into cells (e.g., transfection or transformation) and may be performed by selecting appropriate standard techniques known in the art depending on the cell type. For example, methods such as electroporation, calcium phosphate (CaPO4) precipitation, calcium chloride (CaCl2) precipitation, microinjection, polyethylene glycol (PEG) method, DEAE-dextran method, cationic liposome method, and lithium acetate-DMSO method may be used, without being limited thereto.

In process (d), a method of isolating and fractionating polysomes from the cell into which a vector has been introduced may be performed by selecting an appropriate standard technique known in the art.

In an embodiment, process (d) may include lysing the cell into which a vector has been introduced, and fractionating polysomes by centrifugation into free mRNA, monosome, LP, MP, and HP, but is not limited thereto.

Additionally, after extracting free mRNA, monosome, LP, MP, and HP from each fraction, performing sequencing to obtain each read value, and using the obtained read values as a basis, values of Equation (1) and MRL may be determined for each oligonucleotide (i.e., each segment of the viral genome).

An oligonucleotide for which the calculated value of Equation (1) exceeds 0.2 and the value of the MRL exceeds 4.5 may be selected as a regulatory element for enhancing mRNA translation.

In addition, if the regulatory element for enhancing mRNA translation of the present disclosure also meets the condition that the value of Equation (2) exceeds 0.5, the regulatory element may further enhance RNA stability:

$$\text{Equation (2)} = \text{Log}_2 \ (\text{RNA/DNA}).$$

In this case, the RNA/DNA ratio refers to the ratio of RNA and DNA isolated and/or sequenced from the cell into which a vector has been introduced in the process (d) (for example, a sequencing read ratio).

Under these circumstances, it is possible to screen for a regulatory element that enhance both RNA stability and mRNA translation. Specifically, the screening method may further include: (d)' isolating DNA and RNA from the cell into which a vector has been introduced in process (c), and calculating the value of Equation (2) for each oligonucleotide; and (e)' selecting, as a regulatory element for enhancing RNA stability, an oligonucleotide for which the value of Equation (2) exceeds 0.5. At this stage, processes (d)' and (e)' may be performed simultaneously with processes (d) and (e), respectively, or may be performed as processes separate from processes (d) and (e).

Additionally, in an embodiment, process (d)' may include extracting and isolating DNA and RNA and/or treating the isolated RNA with DNase I to remove vector DNA, but is not limited thereto.

Additionally, based on the isolated DNA and RNA, the value of Equation (2) may be determined for each oligonucleotide (i.e., for each segment of the viral genome). For example, after reverse-transcribing the isolated RNA to obtain cDNA, amplifying the DNA, cDNA, and the original vector pool by PCR, and then performing sequencing, the value of Equation (2) for each oligonucleotide may be determined, but is not limited thereto.

Another aspect of the present disclosure relates to a regulatory element for enhancing mRNA translation that has been screened by the aforementioned screening method. This regulatory element may additionally enhance RNA stability and may enhance protein expression by enhancing RNA stability and/or mRNA translation.

Specifically, the regulatory element for enhancing mRNA translation may be a regulatory element for which the value of Equation (1) exceeds 0.2 and the MRL exceeds 4.5, but is not limited thereto. For example, the value of Equation (1) and the MRL for the regulatory element may be obtained through a method including:

(i) preparing a vector that includes a reporter gene and the regulatory element in the 3' UTR thereof;
(ii) introducing the vector into a cell; and
(iii) fractionating polysomes of the cell into free mRNA, monosome, LP, MP, and HP, and determining the values of Equation (1) and MRL for each oligonucleotide.

In an embodiment, the regulatory element for enhancing mRNA translation further meets the condition that the value of Equation (2) exceeds 0.5, and may thereby further enhance RNA stability, but is not limited thereto. In this case, the value of Equation (2) may be obtained through a method including:

(i) preparing a vector that includes a reporter gene and the regulatory element in the 3' UTR thereof;
(ii) introducing the vector into a cell; and
(iii) isolating DNA and RNA from the cell to obtain the value of Equation (2).

In an embodiment, the regulatory element of the present disclosure may include (i) a nucleotide sequence of any one of SEQ ID NOs: 20 and 79 to 93 (K5, K1-K4, K6-K16) or an RNA nucleotide sequence thereof; or (ii) a nucleotide sequence having at least 50 %, 55 %, 60 %, 65 %, 70 %, 75 %, 80 %, 85 %, 90 %, 95 %, 96 %, 97 %, 98 %, or 99 % homology or identity thereto, but is not limited thereto.

In an embodiment, the regulatory element of the present disclosure may include: (i) the nucleotide sequence of a segment of the Saffold virus genome (NCBI Reference Sequence: NC_009448.2) or an RNA nucleotide sequence thereof; (ii) a nucleotide sequence having at least 50 %, 55 %, 60 %, 65 %, 70 %, 75 %, 80 %, 85 %, 90 %, 95 %, 96 %, 97 %, 98 %, or 99 % homology or identity thereto; or (iii) a homolog thereof.

In the present disclosure, the segment may include more than 120 and up to 190, 130 to 180, 130, or 180 consecutive nucleotides in the 5' direction from the nucleotide at position 8060 within the Saffold virus genome, but is not limited thereto. For example, the segment may consist of the nucleotide sequence of SEQ ID NO: 82 (K4).

Additionally, the homolog may include a nucleotide sequence within the 3' UTR of a cardiovirus genus and having at least 70 %, 75 %, 80 %, 85 %, 90 %, 95 %, 96 %, 97 %, 98 %, or 99 % homology or identity to nucleotides 7952 to 7988 of the Saffold virus genome. For example, the homolog may include the nucleotide sequence of SEQ ID NO: 187 or an RNA nucleotide sequence thereof; or a nucleotide sequence having at least 50 %, 55 %, 60 %, 65 %, 70 %, 75 %, 80 %, 85 %, 90 %, 95 %, 96 %, 97 %, 98 %, or 99 % homology or identity thereto, but is not limited thereto.

The nucleotide sequence within the 3' UTR of a cardiovirus genus may be obtained from known databases (e.g., NCBI, etc.).

However, in the present disclosure, even when a 'regulatory element comprising/including the nucleotide sequence of a specific sequence number' or a 'regulatory element having the nucleotide sequence of a specific sequence number' is described, it is apparent that if regulatory elements, in which some sequences are deleted, modified, substituted, or added with respect to the nucleotide sequence of the specific sequence number, possess the same or equivalent function as the regulatory element with the specific sequence number, they can also be used in this application.

For example, it is apparent that if regulatory elements with non-functional sequences added to the internal or terminal regions of a sequence of the regulatory element with the specific sequence number, or with some sequences deleted from the internal or terminal regions of the sequence of the regulatory element with the specific sequence number, have the same or equivalent function as the regulatory element with the specific sequence number, they also fall within the scope of this application.

Homology and identity refer to the degree of relatedness between two given nucleotide sequences and can be expressed as a percentage. The terms homology and identity can often be used interchangeably.

Whether any two sequences have homology, similarity, or identity can be determined, for example, by using known computer algorithms such as the "FASTA" program with default parameters, as in Pearson et al (1988)[Proc. Natl. Acad. Sci. USA 85]: 2444. Alternatively, such determination can be made using the Needleman-Wunsch algorithm, as performed by the Needleman program of the EMBOSS package (EMBOSS: The European Molecular Biology Open Software Suite, Rice et al., 2000, Trends Genet. 16: 276-277) (version 5.0.0 or later), or other tools such as the GCG program package (Devereux et al., Nucleic Acids Research 12: 387 (1984)), BLASTP, BLASTN, FASTA (Atschul et al., J. Mol. Biol. 215: 403 (1990); Guide to Huge Computers, Martin J. Bishop, Ed., Academic Press, San Diego, 1994; and Carillo et al., SIAM J. Applied Math 48: 1073 (1988)). For example, homology, similarity, or identity of sequences can be determined using BLAST from the National Center for Biotechnology Information, or ClustalW.

In addition, the nucleic acid sequence described in (ii) may include a sequence of any one of SEQ ID NO: 20 and SEQ ID NOs: 79 to 93, incorporating one or more substitutions, deletions, or a combination thereof, or an RNA nucleotide sequence thereof, but is not limited thereto. For example, the altered nucleotide may be one or more nucleotides among nucleotides 1 through 14.

The regulatory element of the present disclosure, by interacting with TENT4, may induce poly(A) tail elongation, poly(A) tail stability increase via mixed tailing, or both.

Another aspect of the present disclosure relates to a construct including a gene of a target protein and the regulatory element of the present disclosure, preferably located in a 3' UTR of the gene. In detail, the construct may be a DNA construct or an mRNA construct.

In the present disclosure, the target protein is not limited as long as RNA stability and/or mRNA translation can be enhanced by the regulatory element of the present disclosure, but may be selected from a reporter, a bioactive peptide, an antigen, or an antibody or a fragment thereof.

In the present disclosure, the bioactive polypeptide may be selected from a hormone, a cytokine, a cytokine-binding protein, an enzyme, a growth factor, or an insulin, but is not limited thereto.

In the present disclosure, the antigen may be selected from a vaccine antigen, a tumor-associated antigen, or an allergy antigen, but is not limited thereto.

In an embodiment, the construct of the present disclosure may further include one or more barcode sequences, forward adapter sequences, reverse adapter sequences, poly(A) tail sequences, or a combination thereof, but is not limited thereto.

In an embodiment, the construct of the present disclosure may further include a promoter sequence, wherein the target protein may be operably linked to the promoter sequence, but is not limited thereto.

In an embodiment, the construct of the present disclosure may further include 5' terminal repeat sequences and 3' terminal repeat sequences from a virus selected from the group consisting of adeno-associated virus, adenovirus, alphavirus, retrovirus (e.g., gamma retrovirus and lentivirus), parvovirus, herpesvirus, and SV40, but is not limited thereto.

In an embodiment, the mRNA construct of the present disclosure may further include a 5' UTR, a 3' UTR, a poly(A) tail sequence, or a combination thereof, but is not limited thereto.

Another aspect of the present disclosure relates to a vector including the construct or a pool of the vector.

In the present disclosure, the term "vector" refers to a genetic construct containing a nucleotide sequence that encodes a target protein operably linked to appropriate regulatory sequences, enabling the expression of the target protein in a suitable host. The regulatory sequences may include a promoter capable of initiating transcription, any operator sequences for regulating such transcription, a sequence encoding an appropriate mRNA ribosome-binding site, and a sequence regulating the termination of transcription and translation, but are not limited thereto. The vector, once introduced into an appropriate host cell, may be replicated or function independently of the host genome, or may be integrated into the genome itself.

In the present disclosure, the vector is not particularly limited as long as it can be expressed in a host cell, and may be introduced into a host cell using any vector known in the art. Examples of commonly used vectors include a plasmid, a cosmid, a virus, and a bacteriophage, whether in their natural states or recombinant forms.

In addition, the term "operably linked" as used herein means that a promoter sequence that initiates and mediates the transcription of a gene encoding a target protein is functionally linked to the sequence of the gene..

Another aspect of the present disclosure relates to a recombinant host cell including the construct or vector.

In the present disclosure, the host cell includes any cell capable of expressing a target protein and encompasses cells that have undergone a natural or artificial genetic modification. In addition, the host cell includes eukaryotic and prokaryotic cells and may specifically be a eukaryotic cell or a cell derived from a mammal (e.g., human), but is not limited thereto.

Another aspect of the present disclosure relates to a composition including the construct, vector, or recombinant host cell. In the present disclosure, the construct, vector, recombinant host cell, or a composition including the same may express a target protein in vitro, in vivo, or ex vivo.

In an embodiment, the composition, when administered to an individual, may provide a target protein to the individual by the construct, vector, or recombinant host cell, and depending on the use of the target protein provided, may exhibit a preventative or therapeutic effect for a disease (e.g., infectious disease). Therefore, the composition may be a pharmaceutical composition but is not limited thereto.

In addition, in an embodiment, using the construct, vector, or recombinant host cell, the mRNA construct or target protein of the present disclosure may be prepared in vitro or ex vivo. Therefore, the composition may be a composition for preparing the mRNA construct or target protein of the present disclosure, but is not limited thereto.

For example, if the target protein is a vaccine antigen, the construct, vector, recombinant host cell, or the composition itself may be used as a vaccine, or may be used to prepare a vaccine antigen.

In an embodiment, the construct or vector of the present disclosure may further include a gene encoding TENT4, or a combination thereof, or the recombinant host cell or composition of the present disclosure may further include TENT4 or a gene encoding the same; or a combination thereof, to induce poly(A) tail elongation, poly(A) tail stability increase, or both, through interactions with TENT4, thereby enhancing RNA stability or mRNA translation, but are not limited thereto.

Another aspect of the present disclosure relates to a composition including TENT4 interacting with the regulatory element, or a gene encoding the same.

The TENT4 may induce poly(A) tail elongation, poly(A) tail stability increase via mixed tailing, or both, through interactions with the regulatory element, thereby enhancing RNA stability or mRNA translation. Therefore, the composition may increase the expression of the target protein of the present disclosure in vitro, in vivo, or ex vivo.

In an embodiment, to express the target protein, the composition may further include the construct, vector, and/or recombinant host cell of the present disclosure, or TENT4 or a gene encoding the same may be included in the construct, vector, and/or recombinant host cell of the present disclosure.

In an embodiment, depending on the use of a target protein whose in vivo expression is enhanced by the composition, the composition may exhibit a preventative or therapeutic effect for a disease. Therefore, the composition may be a pharmaceutical composition but is not limited thereto.

Further, in an embodiment, the composition may be used to prepare the mRNA construct or target protein of the present disclosure in vitro or ex vivo. Therefore, the composition may be a composition for preparing the mRNA or target protein of the present disclosure, but is not limited thereto.

For example, if the target protein is a vaccine antigen, the composition may increase the expression of the vaccine antigen in vivo, allowing the composition to be used as a vaccine composition, or the composition may be used to produce a vaccine antigen in vitro or ex vivo.

Another aspect of the present disclosure relates to a method for preparing a target protein, the method including: culturing the recombinant host cell; and recovering the target protein.

In the present disclosure, the method of preparing a target protein by using the recombinant host cell may be carried out using a method widely known in the art. In detail, the culturing may be carried out continuously in a batch process, fed-batch process, or repeated fed-batch process, but is not limited thereto. The medium used for culturing may be appropriately selected by a person skilled in the art, depending on the host cell. In detail, the recombinant host cell of the present disclosure may be cultured under aerobic or anaerobic conditions in a conventional medium containing an appropriate carbon source, nitrogen source, phosphorus source, inorganic compound, amino acid, and/or vitamin, with adjustments to temperature, pH, and the like.

The method of preparing a target protein may further include an additional process after the culturing. The additional process may be appropriately selected depending on the use of the target protein.

In detail, the method of preparing a target protein may include, after the culturing: recovering the target protein from one or more materials selected from the recombinant host cell, a dried material of the recombinant host cell, an extract of the recombinant host cell, a culture of the recombinant host cell, a supernatant of the culture, or a lysate of the recombinant host cell.

The method may further include lysing the recombinant host cell prior to or simultaneously with the recovering. The lysis of the recombinant host cell may be carried out by a method commonly used in the technical field to which the present disclosure pertains, such as lysis buffer, sonication, heat treatment, or French press. In addition, the lysing may include an enzymatic reaction, which involves cell wall/cell membrane degrading enzymes, nucleases, nucleic acid transferases, and/or proteases, etc., but is not limited thereto.

In the present disclosure, dried material of the recombinant host cell may be prepared by drying cells that have accumulated a target substance, but is not limited thereto.

In the present disclosure, extract of the recombinant host cell may refer to a remaining substance after separating the cell wall/cell membrane from the cell. In detail, the extract of the recombinant host cell may refer to the components obtained by lysing the cell, excluding the cell wall/cell membrane. The cell extract contains the target protein and may also contain, other than the target protein, one or more components from proteins, carbohydrates, nucleic acids, and fibers from the cell, but is not limited thereto.

In the present disclosure, the recovering may recover the target protein using an appropriate method known in the art (e.g., centrifugation, filtration, anion exchange chromatography, crystallization, and HPLC).

In the present disclosure, the recovering may include a purification process. The purification process may involve isolating only the target protein from the cell and purifying the target protein. Through the purification process, the purified target protein may be prepared.

Another aspect of the present disclosure relates to a method of preparing an mRNA construct, the method including: in vitro transcribing the construct or vector; and recovering a transcribed mRNA construct.

The transcription and recovery methods may employ suitable methods known in the art.

In an embodiment, the method may further include treating with DNase I after transcription to remove the DNA of the construct or vector used as a template; and/or washing, but is not limited thereto.

Another aspect of the present disclosure relates to a use of the construct, vector, recombinant host cell, or composition for enhancing RNA stability and/or mRNA translation.

Another aspect of the present disclosure relates to a use of the construct, vector, recombinant host cell, or composition for preventing or treating a disease.

Another aspect of the present disclosure relates to a use of the construct, vector, recombinant host cell, or composition for preparing a target protein.

### Mode for Disclosure

Hereinbelow, the present invention will be described in greater detail with reference to experimental examples and examples. These examples are provided only to illustrate the present invention and therefore, should not be construed as limiting the scope of the present invention.

### [Experimental Examples]

### 1.Cell line culturing

All cell lines used in the present disclosure tested mycoplasma-negative. HeLa cells (gift from C.-H. Chung at Seoul National University and authenticated by ATCC (STR profiling)), Lenti-X 293T cells (Clontech, 632180), and 293AAV cells (Cell Biolabs, AAV-100) were cultured in DMEM containing 10 % FBS (Welgene, S001-01). HCT116 cells (ATCC, CCL-247) were cultured in McCoy's 5A (Welgene, LM 005-01) containing 10 % FBS.

### 2. Oligo design for viromic screens

Genomic sequences of viruses that can infect humans as hosts were retrieved from NCBI Virus Genome Browser (retrieved 2020-01-10, 804 sequences, 504 viruses). Additional information on each virus was retrieved from the GenBank file from NCBI Nucleotide. Based on sequence similarity and virus classification, 143 representative viral species were selected, and woodchuck hepatitis virus was added as a control. For the tiling of RNA viruses, the whole genome of the sequences in positive-sense orientation was used for tiling. For DNA viruses, the sequences of the 3' UTR of coding transcripts and the whole sequences of non-coding RNAs were used for oligo design. If the UTR is not annotated, UTR was predicted based on the poly(A) signal (PAS) annotation. If the PAS is not annotated, PAS was predicted using Dragon PolyA Spotter ver. 1.2 within the range of 800 bp from the stop codon. If the PAS cannot be predicted, the 390-bp region downstream of the stop codon was taken for tiling. After determining the genomic region for tiling, oligos were designed with sliding windows of 130-nt with a 65-nt shift size. When a window contains the SacI or NotI restriction sites which were later used for cloning, the window was made to end at the restriction site, thereby creating a shorter segment. The next segment starts at the restriction site, thereby preventing cleavage of the segment by SacI or NotI during plasmid construction. Thus, the screen may miss some viral elements that contain the restriction site sequences. Also, the design may miss some elements that are longer than 65 nt. For instance, elements with a size of 100 nt have a probability of being missed by approximately 50 %.

Three barcodes of 7-bp random sequences with at least 3 hamming distances were added to each oligo sequence. As controls, the 1E segments and their stem-loop mutants were added to the library. In addition, human hepatitis B virus PRE and its corresponding stem-loop mutants were included as controls. Positive and negative controls were tiled separately. In total, 30,367 segments and 91,101 oligos were designed.

For the secondary screening, five classes of K5 mutants were designed. (1) For single-nucleotide substitution, the base at each position was converted into the other three base types throughout K5. (2) For single-nucleotide deletion, the base at each position was removed. (3) For two-nucleotide deletion, two consecutive nucleotides for all positions were deleted. (4) To examine the significance of base-pairing, the secondary structure was predicted from 6 different RNA secondary prediction software and 38 predicted base-pairs were collected and mutated (AT/TA/GC/CG/GU/UG/del) in a way to preserve the base pair. (5) Two bases randomly selected in predicted loops were mutated to create different combinations. In addition, the homologs of K5 were screened by including 88 homologous elements from other picornaviruses (including 45 from the genus *Kobuvirus*). When the homology was ambiguous, the 3'-most 130-nt were used for oligo design. In total, the library for the secondary screening included 1,288 elements with 3 barcodes each, generating a total of 3,864 oligos.

### 3. Plasmid pool generation

Oligos of 170 nt in length (containing the forward adaptor sequence of 16 nt, the reverse adaptor sequence of 17 nt, and the barcode sequence of 7 nt) were synthesized from Synbio Technologies. NotI and SacI restriction sites were added by 6 cycles of PCR using Q5 High-Fidelity 2X Master Mix (NEB, M0492) and primers SacI-univ-F and NotI-univ-R. The amplified product was purified using 6 % Native PAGE gel, SYBRgold (Invitrogen, S11494) staining. The purified amplified product and pmirGLO-3XmiR-1 vector were digested with SacI-HF (NEB, R3156S) and NotI-HF (NEB, R3189S) and cloned into the 3' UTR of the firefly luciferase gene using T4 DNA ligase (NEB, M0202M). The ligation product was purified with Zymo Oligo Clean & Concentrator kit (Zymo Research, #D4061) and transformed into the Lucigen Endura ElectroCompetent cell (Lucigen, LU60242-2). Transformed bacteria were recovered at 37°C for 1 hour and then cultured with shaking at 30°C for 14 hours. The colony count was confirmed to be approximately 1E7. The primer sequences used are provided in Table 1.

[Table 1]

| qPCR primers | | SEQ. ID |
|---|---|---|
| qPCR-FireflyLuc-F | CCCATCTTCGGCAACCAGAT | 141 |
| qPCR-FireflyLuc-R | GTACATGAGCACGACCCGAA | 142 |
| qPCR-RenillaLuc-F | CTGGACGAAGAGCATCAGG | 143 |
| qPCR-RenillaLuc-R | TGATATTCGGCAAGCAGGCA | 144 |
| qPCR-EGFP-F | AAG CAG AAG AAC GGC ATC AA | 145 |
| qPCR-EGFP-R | GGG GGT GTT CTG CTG GTA GT | 146 |
| qPCR-TENT1-F | GTAACTACGCCCTGACCTTGCT | 147 |
| qPCR-TENT1-R | AGCCATCGACTTCCACCTGTTC | 148 |
| qPCR-TENT2-F | AGTTCGTCCGTTAGTGCTGGTG | 149 |
| qPCR-TENT2-R | GAGGGATGGAAGGATGGGTTCA | 150 |
| qPCR-TENT3B-F | AGGCACCAAGAGAAACGCCGAT | 151 |
| qPCR-TENT3B-R | CATAGAACCGCAGCAATTCCACC | 152 |
| qPCR-TENT4A-F | CCCACCACTTCCAGAACACT | 153 |
| qPCR-TENT4A-R | GCTTTCAAAGACGCAGTTCC | 154 |
| qPCR-TENT4B-F | TCGCAGATGAGGATTCG | 155 |
| qPCR-TENT4B-R | CTGCTCTCACGCCATTCT | 156 |
| qPCR-TENT5C-F | CCTTGAACAGCAGAGGAAGTTGG | 157 |
| qPCR-TENT5C-R | GGAGATGAGGTTCAGAGTCTGC | 158 |
| qPCR-GAPDH-F | CTCTCTGCTCCTCCTGTTCGAC | 159 |
| qPCR-GAPDH-R | TGAGCGATGTGGCTCGGCT | 160 |
| qPCR-U1-F | CCA TGA TCA CGA AGG TGG TTT | 161 |
| qPCR-U1-R | ATG CAG TCG AGT TTC CCA CAT | 162 |
| qPCR-18S-F | GTA ACC CGT TGA ACC CCA TT | 163 |
| qPCR-18R-R | CCA TCC AAT CGG TAG TAG CG | 164 |
| qPCR-ZCCHC2-F | GCACCCGGCTTTCTCCTTCCAC | 165 |
| qPCR-ZCCHC2-R | TGCACGGCTCTACCTCCACCTC | 166 |
| qPCR-TNRC6B-F | AAGGCCCAAACTGCACTGCACA | 167 |
| qPCR-TNRC6B-R | CACTTGGGGTTGCTGCAGGTGT | 168 |

| MPRA plasmid pool generation primers | | SEQ. ID |
|---|---|---|
| SacI-univ-F | tgataagcaGAGCTCACTGGCCGCTTCACTG | 169 |
| NotI-univ-R | tcgtgcttGCGGCCGCCGACGCTCTTCCGATCT | 170 |

(continued)

**MPRA library construction pirmers**

| | | SEQ. ID |
|---|---|---|
| MPRAlib_NN_R | GTT CAG AGT TCT ACA GTC CGA CGA TCN NCG ACG CTC TTC CGA TCT | 171 |
| MPRAlib_NNN_R | GTT CAG AGT TCT ACA GTC CGA CGA TCN NNCG ACG CTC TTC CGA TCT | 172 |
| MPRAlib_N_R | GTT CAG AGT TCT ACA GTC CGA CGA TCN CG ACG CTC TTC CGA TCT | 173 |
| MPRAlib_NN_F | GCC TTG GCA CCC GAG AAT TCC ANNgcaagatcgccgtgtaattc | 174 |
| MPRAlib_NNN_F | GCC TTG GCA CCC GAG AAT TCC ANNNgcaagatcgccgtgtaattc | 175 |
| MPRAlib_N_F | GCC TTG GCA CCC GAG AAT TCC ANgcaagatcgccgtgtaattc | 176 |

**in vitro RNA transciption (Luciferase)**

| | | SEQ. ID |
|---|---|---|
| T7promoter+gene_spe cific_F(Luciferase) | TAA TAC GAC TCA CTA TAG GGA GAG GGC TTT CG ACC TGC AGC CCA AGC | 177 |
| T120+gene_specific_R | mUmU[T*118]ATCAATGTATCTTATCATGTCTG | 178 |
| T7promoter+gene_spe cific_F(d2EGFP) | TAATACGACTCACTATAGGGAGAGGGAAATAAGA GAGAAAGAAGA | 179 |

**Hire-PAT PCR primer**

| | | SEQ. ID |
|---|---|---|
| Hire-PAT-FireflyLuc-F | GGACAAACCACAACTAGAATG | 180 |

**Gene Specific TAIL-seq PCR primer**

| | | SEQ. ID |
|---|---|---|
| GS-TAIL-seq-FireflyLuc-F | GTT CAG AGT TCT ACA GTC CGA CGA TCG GAC AAA CCA CAA CTA GAA TG | 181 |

**plasmid**

| | | |
|---|---|---|
| pAAV-CAG-GFP | AAV generation | addgene 37825 |
| pAdDeltaF6 | AAV generation | addgene 112867 |
| pAAV-DJ | AAV generation | cell biolabs, VPK-420-DJ |

**plasmid**

| plasmid | purpose | reference |
|---|---|---|
| pAAV-CAG-GFP control | AAV generation | |
| pAAV-CAG-GFP K5 | AAV generation | |
| pAAV-CAG-GFP eK5 | AAV generation | |
| pAAV-CAG-GFP K5m | AAV generation | |
| pAAV-CAG-GFP eK5m | AAV generation | |
| pmirGLO-3Xmir-1 | control | NSMB, 2020 |
| pmirGLO-3Xmir-1_K1 | validation | |
| pmirGLO-3Xmir-1_K2 | validation | |
| pmirGLO-3Xmir-1_K3 | validation | |
| pmirGLO-3Xmir-1_K4 | validation | |
| pmirGLO-3Xmir-1_K6 | validation | |
| pmirGLO-3Xmir-1_K7 | validation | |
| pmirGLO-3Xmir-1_K8 | validation | |
| pmirGLO-3Xmir-1_K9 | validation | |
| pmirGLO-3Xmir-1_K10 | validation | |
| pmirGLO-3Xmir-1_K11 | validation | |
| pmirGLO-3Xmir-1_K12 | validation | |
| pmirGLO-3Xmir-1_K13 | validation | |
| pmirGLO-3Xmir-1_K14 | validation | |
| pmirGLO-3Xmir-1_K15 | validation | |
| pmirGLO-3Xmir-1_K16 | validation | |
| pmirGLO-3Xmir-1_K5 | validation, luciferase, GS TAIL-seq, Hire-PAT | |
| pmirGLO-3Xmir-1_K5m | luciferase, Hire-PAT | |
| pmirGLO-3Xmir-1_eK5 | luciferase, Hire-PAT, ivt RNA binding assay | |
| pmirGLO-3Xmir-1_eK5m | luciferase, Hire-PAT, ivt RNA binding assay | |
| pmirGLO-3Xmir-1_wPRE | luciferase | NSMB, 2020 |
| pmirGLO-3Xmir-1_full UTR | validation | |
| pmirGLO-3Xmir-1_120-K5 | validation | |
| pmirGLO-3Xmir-1_110-K5 | validation | |
| pmirGLO-3Xmir-1_eK4 | validation | |
| pmirGLO-d2EGFP-GBA | IVT mRNA generation | |
| pmirGLO-d2EGFP-eK5-GBA | IVT mRNA generation | |
| pmirGLO-d2EGFP-GBA-eK5 | IVT mRNA generation | |
| pmirGLO-3xBoxB | Tethering | |

| **plasmid** | | SEQ.ID |
|---|---|---|
| pCK-MCS | Rescue | |
| pGK-MCS | Rescue | |
| pCK-TNRC6B-Cterm | Tethering | |
| pCK-lambdaN-HA-TEV-TNRC6b-Cterm | Tethering | |
| pGK-ZCCHC2 | Rescue, Tethering | |
| pGK-lambdaN-HA-TEV-ZCCHC2 | Tethering | |
| pGK-ZCCHC2 (Zinc-finger mutant) | Rescue, Tethering | |
| pGK-lambdaN-HA-TEV-ZCCHC2 (Zinc-finger mutant) | Tethering | |
| pCK-Flag-ZCCHC2 | Rescue, Co-immunoprecipitation | |
| pCK-Flag-ZCCHC2 (201-1178) | Rescue, Co-immunoprecipitation | |
| pCK-Flag-ZCCHC2 (1-375) | Rescue, Co-immunoprecipitation | |
| pSpCas9(BB)-2A-GFP-px458 | KO generation | addgene 48138 |
| BASU RaPID | RaPID | addgene 107250 |
| pCK-EGFP-3xBoxB | RaPID | |
| pCK-EGFP-3xBoxB-eK5-3xBoxB | RaPID | |
| **siRNAs** | | |
| siTENT1 | ON-TARGETplus SMART pool (Dharmacon) | |
| siTENT2 | ON-TARGETplus SMART pool (Dharmacon) | |
| siTENT3 A/B | ON-TARGETplus SMART pool (Dharmacon) | |
| siTENT4 A/B | ON-TARGETplus SMART pool (Dharmacon) | |
| siTENT5 A/B/C/D | ON-TARGETplus SMART pool (Dharmacon) | |
| **Genomic sequences of ZCCHC2 HeLa cells and ZCCHC14 KO HCT116 cells** | | **SEQ.ID** |
| Parental | ACCTCAGGACGGACTTACCG | 182 |
| ZCCHC2 KO allele 1 | ACCTCAGGACGGACT-ACCG | 183 |

| Genomic sequences of ZCCHC2 HeLa cells and ZCCHC14 KO HCT116 cells | | SEQ.ID |
|---|---|---|
| ZCCHC2 KO allele 2 | ACCTCAGGACGGACTtacgggataaggccggcttcatcaaga gacagctggtggaaacccggcagatcacaaagcacgtggcacagatc ctggactcccggatgaacactaagtacgacgagaatgacaagttgatcc gggaagtgaaagtgatcaccctgaagtccaagctggtgtccgatttccgg aaggatttccagttttacaaagtgcgcgagatcaacaactaccaccacg cccacgacgcctacctgaacgccgtcgtgggaaccgccctgatcaaaa agtaccctaagctggaaagcgagttcgtgtacggcgactacaaggtgta cgacgtgcggaagatgatcgccaagagcgagcaggaaatcggcaag gctaccgccaagtacttcttctacagcaacatcatgaactttttcaagaccg agaTACCG | 184 |
| ZCCHC2 KO allele 3 | ACCTCAGGACGGACTtacgggataaggccggcttcatcaaga gacagctggtggaaacccggcagatcacaaagcacgtggcacagatc ctggactcccggatgaacactaagtacgacgagaatgacaagctgatc cgggaagtgaaagtgatcaccctgaagtccaagctggtgtccgatttccg gaaggatttccagttttacaaagtgcgcgagatcaacaactaccaccac gcccacgacgcctacctgaacgccgtcgtgggaaccgccctgatcaaa agtaccctaagctggaaagcgagttcgtgtacggcgactacaaggtgta cgacgtgcggaagatgatcgccaagagcgagcaggaaatcggcaag gctaccgccaagtacttcttctacagcaacatcatgaactttttcaagaccg agaTACCG | 185 |
| Parental ZCCHC14 KO | CAAGTGGGCAGCGCGCCGCC CAA---------------------- | 186 |

## 4. Library construction

4E5 HCT116 cells were seeded one day before transfection for RNA stability screening. 1.5 $\mu$g of the plasmid pool was transfected by Lipofectamine 3000 (Invitrogen, L3000001) and p3000. RNA and DNA were extracted 48 hours post-transfection using the Allprep RNA/DNA Mini Kit (Qiagen, 80004), and RNA was treated with Recombinant DNase I (RNase-free) (TAKARA, 2270A) to remove remaining plasmid DNA. RNAs were reverse-transcribed using SSIV reverse transcriptase (Invitrogen, 18090010). The extracted DNA, cDNA obtained from RNA, and the original plasmid pool were amplified by 14 cycles of PCR, using mixed primers MPRAlib_N/NN/NNN_F and MPRAlib_N/NN/NNN_R (Table 1). 6 cycles of the second PCR were performed using Illumina index primers. The PCR amplicons were sequenced by next-generation sequencing using the Illumina Novaseq 6000 platform.

For nuclear/cytoplasmic fractionation screening, the cytoplasm was obtained using cytosol lysis buffer (0.15 $\mu$g/$\mu$l digitonin [Merck, D141], 150 mM NaCl, 50 mM HEPES [pH 7.0-7.6], 20 U/ml RNase inhibitor [Ambion, AM2696], 1X protease inhibitor [Calbiochem, 535140], 1X phosphatase inhibitor [Merck, P0044]). The library preparation steps were performed in the same manner as the RNA stability screening.

For polysome fractionation screening, a 10-50 % sucrose gradient was prepared using Gradient Master™ (Biocomp, B108-2). HCT116 cells, at three times the scale of RNA stability screening, were treated with 100 $\mu$g/ml cycloheximide for 1 minute at 37°C, then lysed with 150 $\mu$l of PEB (20 mM Tris-Cl pH 7.5, 100 mM KCI, 5 mM MgCl2, 0.5 % NP-40 [Merck, 74385]) containing 100 U/ml RNase inhibitor, 1X protease inhibitor, and 1X phosphatase inhibitor on ice for 10 minutes, and then centrifuged. The supernatant was layered onto the sucrose gradient and centrifuged at 36,000 rpm for 2 hours at 4°C using an SW41Ti rotor and a Beckman Coulter Ultracentrifuge Optima XE. Samples were collected in 0.25 ml fractions using a Biologic LP system coupled with a Model 2110 fraction collector (Bio-Rad, 7318303) and a Model EM-1 Econo UV detector (Bio-Rad). 0.75 ml of TRIzol™ LS Reagent (Life Technologies) was immediately added to each fraction. Free mRNA, monosome, light polysome (LP; 2-3 ribosomes), medium polysome (MP; 4-8 ribosomes), and heavy polysome (HP; 9 or more ribosomes) were separated based on the 254 nm absorbance trend and extracted using the Direct-Zol RNA Miniprep kit (Zymo Research, R2052).

The following library preparation steps were performed in the same manner as the RNA stability screening. The sequencing data are available in the Zenodo database under the following DOI identifiers: [https://doi.org/10.5281/zenodo.6777910] (Stability), https://doi.org/10.5281/zenodo.6717932 (Polysome), https://doi.org/10.5281/zenodo.6696870 (Secondary screening), https://doi.org/10.5281/zenodo.7773943 (Nuclear/cytoplasmic fractionation).

## 5. Data analysis

For all samples, reads were aligned to oligos using bowtie 2.2.6 with the parameter-local. Aligned reads were filtered to ensure a strict, unique match to the barcode. Statistical tests were performed with MPRAnalyze using the mpralm function. Technical performance was assessed using the Spearman correlation coefficient from the scipy module and histogram plots. Normalized counts were used for visualization. For polysome analysis, after variance stabilizing transformation using DESeq2, the relative distance of each fraction was calculated by subtracting the mean of the five fractions. The relative distance of each fraction was used to perform hierarchical clustering in the scipy module. For another translational quantification, Mean Ribosome Load (MRL) was calculated as follows:

$$1 \times p(Monosome) + 2.5 \times p(Light\ polysome) + 6 \times p(Medidum\ polysome) + 11 \times p(Heavy\ polysome)$$

p(X): the proportion of sequencing reads for X (each fraction).

For mRNA stability cutoff, $Log_2FC < -1$ and adjusted p-value<0.001 were used for negatively regulated elements, and $Log_2FC > 0.5$ and adjusted p-value<0.05 were used for positively regulated elements. $Log_2$(heavy polysome / free mRNA) > 0.2 and/or MRL > 4.5 were used for the translational activating element cutoff, and $Log_2$(heavy polysome / free mRNA) < -0.2 and/or MRL < 3.5 were used for the translational downregulating element cutoff.

For the second screening substitution data, the base-identity score of substitution and deletion was calculated as follows:

A / mean($Stability_x$, $Stability_y$, $Stability_z$) (for substitution, x, y, z : substituted nucleotides)
A / Stability for deletion (for deletion)
A: the stability of wildtype K5.

The base-pairing score for substitution data was calculated as follows.
mean(Stability of substitutions maintaining base pair)

- mean(Stability of substitutions disrupting base pair)

The pair-deletion score for deletion data was calculated as follows.
A / Stability for pairwise deletion

For the tree construction of picornaviruses, virus sequences retrieved from NCBI were aligned using ClustalOmega and visualized using FigTree v1.4.4. The conservation score was calculated as the number of identical nucleotides with the K5 element after multiple sequence alignment across the top 33 species. For RNA structure visualization, the structure was predicted using RNAfold and visualized using forna.

**6. Plasmid construction**

For validation experiment, the selected elements were PCR-amplified from the plasmid library pool and cloned into 3' UTR of firefly gene in pmirGLO-3XmiR-1 vector. For luciferase construct, K5 element (8122-8251: NC_001918.1) was amplified from the plasmid pool library, and an additional 55 bp and 110 bp were added by PCR amplification to create eK5 element (8067-8251: NC_001918.1) and full UTR (8012-8251: NC_001918.1), respectively. 120-K5 element (8132-8251: NC_001918.1), 110-K5 element (8142-8251: NC_001918.1), and K5m element (8122-8251,8185ΔG: NC_001918.1) were amplified from pmirGLO-3XmiR-1 K5 plasmid, and eK5m element (8067-8251: NC_001918.1) was amplified from pmirGLO-3XmiR-1 eK5 plasmid. K4 element (7931-8060: NC_009448.2) was amplified from the plasmid pool library, and an additional 50 bp was added by PCR amplification to make eK4 element (7881-8060: NC_009448.2). 1E element (414-463: RNA2.7) was amplified from pmirGLO-3XmiR-1 1E vector.

For AAV production, pAAV-CAG-GFP (Addgene, Plasmid #37825) plasmid was used as a template. K5 element (8122-8251: NC_001918.1), K5m element (8122-8251, 8185ΔG: NC_001918.1), eK5 element (8067-8251: NC_001918.1), and eK5m element (8067-8251, 8185ΔG: NC_001918.1) were amplified from pmirGLO-3XmiR-1 eK5 and eK5m plasmid and replaced WPRE sequence in pAAV-CAG-GFP plasmid by Gibson assembly. For control plasmid, WPRE sequence in 3' UTR of GFP gene in pAAV-CAG-GFP was eliminated by PCR-based amplification.

For d2EGFP plasmid construction, firefly luciferase gene from pmirGLO-3XmiR-1 vector was replaced by GBA 5' UTR, d2EGFP CDS, and GBA 3' UTR to make control plasmid. UTRs from luciferase constructs were amplified and inserted into this d2EGFP control vector.

For tethering and rescue construction, pmirGLO-3xBoxB was generated from pmirGLO-3xmir1-5xBoxB vector, and for pGK-ZCCHC2 construct, ZCCHC2 amplified from HCT116 cDNA was subcloned into pGK vector. Tethering constructs including ZCCHC2 ΔC (1-375 a.a) and ZCCHC2 ΔN (201 aa-1,178 a.a) constructs were generated by subcloning ZCCHC2 in pGK-TEV-HA-λN. To generate ZCCHC2 zinc-finger mutated version, first and second cysteines of the zinc-finger (CX2CX3GHX4C) were replaced with serine by mutagenesis PCR. For TNRC6B C-term constructs, C-term region (716-1,028 a.a) of TNRC6B gene was amplified from HCT116 cDNA and was subcloned into pGK and pGK-TEV-HA-λN vector by Gibson assembly.

For RaPID experiment, EGFP CDS, 3xBoxB sequence, and eK5 sequence were amplified from d2EGFP, pmirGLO-3xBoxB, and pmirGLO-3xmir-1-eK5 plasmids, respectively, and subcloned into the pCK vector by Gibson assembly.

The list of plasmids generated by this method is shown in Table 1.

**7. Luciferase assay and transfection**

Luciferase assay was performed as follows. For luciferase reporter assay by Lipofectamine 3000, 2E5 of HeLa or HCT116 cells on a 24-well plate were transfected with 100 ng of pmirGLO-3XmiR-1 plasmid on Day 0, and harvested on Day 2. For knockdown experiment, 100 ng of the pmirGLO-3XmiR-1 K5 plasmid and 40 nM of siRNAs (Dharmacon siRNA smartpool) were co-transfected using Lipofectamine 3000 for each target gene. For ZCCHC2 structure experiment, 50 ng of the pmirGLO-3XmiR-1 plasmid and 60 ng of pGK-null, pGK-ZCCHC2, or pGK-ZCCHC2 zinc-finger mutant construct were co-transfected. For tethering experiment, 50 ng of pmirGLO-3xBoxB plasmid and 60 ng of pGK-ZCCHC2 wild-type/mutant constructs were co-transfected, with or without λN-HA-TEV flag. For the luciferase assay, cells were lysed and analyzed using the Dual-luciferase reporter assay system (Promega) according to the manufacturer's instructions.

**8. RT-qPCR**

RNA was extracted by RNeasy Mini Kit (Qiagen, 74106), treated with DNase (Qiagen, 79254), and reverse-transcribed with Primescript RTmix (Takara, RR036A). mRNA levels were measured with SYBR Green assays (Life Technologies, 4367659) and StepOnePlus Real-Time PCR System (Applied Biosystems) or QuantStudio 3 (Applied Biosystems). The list of RT-qPCR primers is shown in Table 1.

### 9. AAV generation and purification

AAV generation and purification were performed as follows. 293 AAV cell lines (Cell Biolabs, #AAV-100) were cultured in DMEM with 10 % FBS, 0.1 mM MEM Non-essential Amino Acids (NEAA), and 2 mM L-glutamine. For producing AAVs carrying GFP proteins, the 293 AAV cells were seeded overnight in a 150-mm petri dish and when the confluence reached 70 %, pAAV-CAG-GFP plasmid variants (Addgene, 37825) along with pAdDelta6F6 (Addgene, 112867) and pAAVDJ (Cell Biolabs, VPK-420-DJ) plasmids were co-transfected with Lipofectamine 3000 and p3000. After 72 hours of transfection, the cells were harvested and resuspended in 2.5 ml of serum-free DMEM. Then, cell lysis was performed through 4 rounds of freezing/thawing (30-min freezing in ethanol/dry ice and 15-min thawing in 37°C water bath, in each cycle). AAV supernatants were collected after centrifugation at 10,000Xg for 10 minutes at 4°C. After purifying the AAVs using the ViraBind™ AAV Purification Kit (Cell Biolabs), viral titers were measured using the QuickTiter™ AAV Quantitation Kit (Cell Biolabs) according to the manufacturer's instructions. For transduction, HeLa cells were seeded in a 12-well plate and infected by AAV with 2,000 and 10,000 moi along with mock infection with PBS as a control. After 5 days of infection, the GFP signal was detected using a flow cytometer (BD Accuri C6 Plus).

### 10. Preparation of *in vitro* transcribed RNA

For *in vitro* transcribed RNAs, DNA templates were prepared by PCR using a forward primer (T7 promoter + gene_specific_F) and a reverse primer (T120 + gene_specific_R, with two nucleotides of 2'-O-Methylated deoxyuridine at the 5' end). 250 ng of DNA templates was *in vitro* transcribed using the mMESSAGE mMACHINE™ T7 Transcription Kit (Invitrogen, AM1344) and Components (7.5 mM ATP/CTP/UTP [NEB, N0450S] each, 1.5 mM GTP, and 6 mM CleanCap® Reagent AG (3' OMe) [TriLink Biotechnologies]). The DNA templates were removed using Recombinant DNase I (RNase-free) and cleaned up using the RNeasy MiniElute Cleanup Kit (Qiagen, 74204). The primers used for *in vitro* transcription template preparation are shown in Table 1.

### 11. Preparation and Analysis of mRNA transfected samples

2E5 of HeLa cells on a 12-well plate were transfected with in vitro transcribed RNAs using Lipofectamine MessengerMax. For samples transfected with luciferase mRNA, the cells were lysed and analyzed by Dual-luciferase reporter assay system according to the manufacturer's instructions. For d2EGFP samples, the cells were lysed in RIPA lysis and extraction buffer (Thermo, 89901), which contains 1X protease inhibitor and 1X phosphatase inhibitor, on ice for 10 minutes and then centrifuged. The samples were boiled with 5X SDS buffer and loaded on Novex SDS-PAGE gel (10-20 %) using the ladder (Thermo, 26616). The gel was transferred to a methanol-activated PVDF membrane (Millipore), then blocked with PBS-T containing 5 % skim milk, followed by probing with primary antibodies and washing three times with PBS-T. Anti-EGFP (1:3,000, CAB4211, Invitrogen), and anti-alpha-TUBULIN (1:300, Abcam, ab52866) were used as the primary antibodies. Anti-mouse or anti-rabbit HRP-conjugated secondary antibodies (Jackson ImmunoResearch Laboratories) were incubated for 1 hour and washed 3 times with PBS-T. Chemiluminescence was conducted with West Pico or Femto Luminol reagents (Thermo), and the signals were detected by ChemiDoc XRS+ System (Bio-Rad). For d2EGFP samples, the GFP signals were detected by a flow cytometer (BD Accuri C6 Plus).

### 12. Hire-PAT assay

Hire-PAT assay and signal processing of capillary electrophoresis data were performed as described in the literature (Kim et al., Nat. Struct. Mol. Biol., 2020, 27, 581-588). Poly(A) site of the firefly luciferase gene was used as confirmed by Sanger sequencing in the referenced literature, and forward PCR primers for the poly(A) site are listed in Table 1.

### 13. Gene-specific TAIL-seq

To measure the poly(A) tail length distribution upon RG7834 treatment, HeLa cells were transfected with the pmirGLO-3XmiR-1 plasmid containing the K5 element in the 3' UTR of firefly luciferase treated with RO0321 (Glixx Laboratories Inc, GLXC-11004) or RG7834 (Glixx Laboratories Inc, GLXC-221188), and harvested within two days. To compare the poly(A) tail length distribution between parental cells and ZCCHC2 knockout, parental cells and ZCCHC2 knockout cells were prepared in the same way as the RG7834-treated sample. To perform gene-specific TAIL-seq, rRNA-depleted total RNAs (Truseq Strnd Total RNA LP Gold, Illumina, 20020599) were ligated to the 3' adapter and partially fragmented by RNase T1 (Ambion). After purification on a Urea-PAGE gel (300-1500 nt), the RNA was reverse transcribed and amplified by PCR. For PCR amplification of the firefly luciferase gene, GS-TAIL-seq-FireflyLuc-F was used as the forward primer. The libraries were sequenced on the Illumina platform (Miseq) using the PhiX control library v.2 (Illumina) containing a spike-in mixture, with a paired-end run (51X251 cycles). The TAIL-seq sequencing data have been deposited in the Zenodo database with the identifier DOI:10.5281/zenodo.6786179.

The TAIL-seq was analyzed using Tailseeker v.3.1.5. For each transcript, genes were identified by mapping read 1 to the firefly luciferase construct sequence and the human transcriptome using bowtie2.2.6. Next, the corresponding poly(A) tail length and modifications at the 3' end were extracted using read 2. The mixed tailing ratio was calculated from transcripts with poly(A) tails longer than 50 nt.

## 14. Preparation of TENT4, ZCCHC2 and ZCCHC14 knockout cells

TENT4 dKO cells were prepared using the same method as described in the literature by Kim et al. In addition, ZCCHC2 and ZCCHC14 knockout cell lines were also prepared according to the method described in the literature by Kim et al. HeLa cells in a 6-well plate and HCT116 cells in a 24-well plate were transfected with 300 ng of the pSpCas9(BB)-2A-GFP-px458 plasmid (Addgene #48138) containing sgRNA targeting ZCCHC2 (ACCTCAGGACGGACTTACCG, PAM sequence: TGG) and sgRNA targeting ZCCHC14 (CAAGTGGGCAGCGCGCGCCGCC [SEQ ID NO: 97], PAM sequence: CGG), respectively, using Metafectene (Biontex, T020). After single-cell screening, knockout strains were confirmed by Sanger sequencing and western blot analysis. The parental and modified genome sequences are listed in Table 1, with the inserted sequences highlighted in red.

## 15. RNA proximity labeling assay

RaPID (RNA-protein interaction detection) assay was performed as follows. In detail, a BASU-expressing stable HeLa cell line was generated by transducing lentiviral delivery constructs produced from Lenti-X 293T (Clontech, 632180) and the BASU RaPID plasmid (Addgene #107250). 1E7 cells from a 150 mm plate were transfected with 40 μg of RNA synthesized above, using Lipofectamine mMAX (Life Technologies, LMRNA015). After 16 hours, the cells were treated with 200 μM biotin (Sigma, B4639) for 1 hour. The treated cells were lysed on ice for 10 minutes using RIPA lysis and extraction buffer (Thermo, 89901) containing 1X protease inhibitor and 1X phosphatase inhibitor, followed by centrifugation. The lysate was incubated with Pierce streptavidin beads (Thermo, 88816) at 4°C overnight with rotation. The beads were washed three times with wash buffer 1 (1 % SDS containing 1 mM DTT, protease, and phosphatase inhibitor cocktails), was washed once with wash buffer 2 (0.1 % Na-DOC, 1 % Triton X-100, 0.5 M NaCl, 50 mM HEPES pH 7.5, 1 mM DTT, 1 μM EDTA containing protease and phosphatase inhibitor cocktails), and then washed once with wash buffer 3 (0.5 % Na-DOC, 150 mM NaCl, 0.5 % NP-40, 10 mM Tris-HCl, 1 mM DTT, 1 μM EDTA containing protease and phosphatase inhibitor cocktails).

For western blot, proteins were eluted using Elution buffer (1.5x Laemmli sample buffer, 0.02 mM DTT, 4 mM Biotin) and analyzed by western blot using anti-ZCCHC2 (1:250, Atlas Antibodies, HPA040943), anti-TENT4A (1:500, Atlas Antibodies, HPA045487), anti-alpha-TUBULIN (1:300, Abcam, ab52866), anti-HA (1:2000, Invitrogen, 715500) primary antibodies. For LC-MS/MS analysis, the samples were washed six times with digestion buffer (50 mM Tris, pH 8.0) at 37°C for 1 minute. After washing, the protein-bound beads were incubated at 37°C for 1 hour in 180 μL of digestion buffer containing 2 μL of 1 M DTT, followed by the addition of 16 μL of 0.5 M IAA and further incubation at 37°C for 1 hour. Then, 2 μL of 0.1 g/L trypsin was added, and the resulting mixture was incubated overnight at 37°C. The remaining detergents were removed using HiPPR (Thermo, 88305) and washed with ZipTip C18 resin (Millipore, ZTC18S960) prior to LC-MS/MS analysis.

LC-MS/MS analysis was carried out using an Orbitrap Eclipse Tribrid (Thermo) coupled with a nanoAcquity system (Waters). The capillary analytical column (75 μm i.d. × 100 cm) and trap column (150 μm i.d. × 3 cm) were packed with 3 μm of Jupiter C18 particles (Phenomenex). The LC flow was set to 300 nL/min with a 60-minute linear gradient ranging from 95 % solvent A (0.1 % formic acid (Merck)) to 35 % solvent B (100 % acetonitrile, 0.1 % formic acid). Full MS scans (m/z 300-1,800) were acquired at 120k resolution (m/z 200). High-energy collision-induced dissociation (HCD) fragmentation occurred at 30 % normalized collision energy (NCE) with 1.4th precursor isolation window. MS2 scans were acquired at a resolution of 30k.

MS/MS raw data were analyzed using MSFragger1 (v3.7), IonQuant2 (v1.8.10), and Philosopher3 (v4.8.1) integrated into FragPipe (v18.0). For label-free protein identification and quantification, a built-in FragPipe workflow (LFQ-MBR) was used with trypsin specified as the enzyme. The target-decoy database (including contaminants) was generated using FragPipe from the Swiss-Prot human database (October 2022). The combined_protein.tsv file was used for further analysis. For the enrichment cutoff, a Log2FC greater than 1, based on at least two replicate experiments, was used.

## 16. Co-immunoprecipitation (co-IP) and western blotting

For co-IP experiment, parental cells and TENT4 dKO cells on a 150 μl plate were lysed on ice for 20 minutes using Buffer A (100 mM KCl, 0.1 mM EDTA, 20 mM HEPES [pH 7.5], 0.4 % NP-40, 10 % glycerol) containing 1 mM DL-Dithiothreitol (DTT), 1X protease inhibitor, and RNase A (Thermo, EN0531), and then centrifuged. For immunoprecipitation, 12.5 $\mu$g of antibody (NMG, anti-TENT4A, and anti-TENT4B) conjugated to protein A and G sepharose beads (1:1 mixture, total 20 $\mu$l) was used with 1 mg of the lysates. After incubation at 4°C for 2 hours, the beads were washed, boiled in 20 $\mu$l of 2X SDS buffer, and loaded onto a 4-12 % (Novex) SDS-PAGE gel with the ladder (Thermo, 26616 and 26619). For domain co-IP experiment, full-length ZCCHC2, truncated construct of ZCCHC2, and negative construct having FLAG tag were transfected in ZCCHC2KO cells, and the cells were lysed within 2 days. 10 $\mu$l of ANTI-FLAG® M2 Affinity Gel (Merck, A2220-10ML) were added to 1 mg of the lysates and immunoprecipitation was performed for 2 hr incubation at 4 °C. For the input sample, 50 $\mu$g of cell lysates were used. After the gel transferring to a methanol-activated PVDF membrane (Millipore), the membrane was blocked with PBS-T containing 5 % skim milk, probed with primary antibodies, and washed three times with PBS-T. Anti-ZCCHC2 (1:250, Atlas HPA040943), anti-ZCCHC14 (1:1,000, Bethyl Laboratories, A303-096A), anti-TENT4A (1:500, Atlas Antibodies, HPA045487), anti-TENT4B (1:500, lab-made), anti-GAPDH (1:1,000, Santa Cruz, sc-32233), and anti-FLAG (1:1,000, Abcam, ab1162) were used as the primary antibodies. Anti-mouse or anti-rabbit HRP-conjugated secondary antibodies (Jackson ImmunoResearch Laboratories) were incubated for 1 hour and washed 3 times with PBS-T. Chemiluminescence was conducted with West Pico or Femto Luminol reagents (Thermo, 34580 and 34095), and the signals were detected by ChemiDoc XRS+ System (Bio-Rad).

### 17. Re-analysis of RNA pulldown-LC-MS/MS data

MS/MS data were processed using MaxQuant v.1.5.3.30 with default settings and the human Swiss-Prot database v.12/5/2018, applying a 0.8 % FDR cutoff at the protein level.

Among the MaxQuant output files, MaxLFQ intensity values were extracted from the proteingroups.txt file. After adding a pseudo-value of 10,000 to MaxLFQ intensity values, Limma was performed and significant genes were filtered by Log2FC>0.8 and FDR<0.1.67.

### 18. Domain conservation analysis

Using the UniProt Align tool, ZCCHC2 (Q9C0B9), ZCCHC14 (A0A590UJW6), and GLS-1 (Q8I4M5) were aligned, and conservation scores for the three proteins were calculated

### 19. RNA immunoprecipitation

For ZCCHC2 immunoprecipitation, a stable HeLa cell line expressing EGFP with the K5 element in the 3' UTR was generated by transducing lentiviral vectors produced from Lenti-X 293T (Clontech, 632180) cells according to the constructs. In addition, the cells were lysed by treatment on ice for 30 minutes with lysis buffer (20 mM HEPES pH 7.6 [Ambion, AM9851 and AM9856], 0.4 % NP-40, 100 mM KCl, 0.1 mM EDTA, 10 % glycerol, 1 mM DTT, 1X Protease inhibitor [Calbiochem, 535140]), followed by centrifugation to obtain the cell lysate. As a negative control, 10 $\mu$g of normal rabbit IgG (Cell Signaling, 2729S) was used, and for ZCCHC2 immunoprecipitation, 10 $\mu$g of ZCCHC2 antibody (Atlas, HPA040943) was used. After antibodies being conjugated to protein A magnetic beads (Life Technologies, 10002D), 1 mg of cell lysates were incubated with antibody-conjugated beads for 2 hours and then washed with wash buffer (the same lysis buffer but with 0.2% NP-40). After adding 5 ng of firefly luciferase mRNA to each sample as a spike-in used for normalization, RNAs were purified by TRIzol reagent (Life Technologies) and used for RT-qPCR. The RT-qPCR primers are shown in Table 1.

### 20. Subcellular fractionation

Subcellular fractionation was conducted as follows. In detail, to obtain cytoplasmic fraction, cells were lysed in 200 $\mu$l of cytoplasmic lysis buffer (0.2 $\mu$g/$\mu$l digitonin [Merck, D141], 150 mM NaCl, 50 mM HEPES [pH 7.0-7.6], 0.1 mM EDTA, 1 mM DTT, 20 U/ml RNase inhibitor, 1X Protease inhibitor, 1X Phosphatase inhibitor). For the membrane and nuclear fractions, a subcellular protein fractionation kit (Thermo Scientific, 78840) was used according to the manufacturer's instructions. Anti-GM130 (1:500, BD Bioscience, 610822) and anti-Histone (1:2000, Cell Signaling, 4499) were used as the primary antibodies.

The reagents and resources used in the experimental examples of the present disclosure are shown in Table 2 below.

[Table 2]

| REAGENT or RESOURCE | SOURCE | IDENTIFIER |
|---|---|---|
| Antibodies | | |
| Mouse polyclonal anti-GAPDH | Santa Cruz | Cat#sc-32233; RRID: AB_627679 |

EP 4 549 591 A1

(continued)

| REAGENT or RESOURCE | SOURCE | IDENTIFIER |
|---|---|---|
| Antibodies | | |
| Rabbit polyclonal anti-ZCCHC2 | Atlas | Cat#HPA040943; RRID: AB_10795496 |
| Rabbit polyclonal anti-ZCCHC14 | Bethyl Laboratories | Cat#A303-096A; RRID: AB_10895018 |
| Mouse monoclonal anti-GM130 | BD Bioscience | Cat#610822; RRID: AB_398141 |
| Rabbit monoclonal anti-Histone (H3) | cell signalling | Cat#4499; RRID: AB_10544537 |
| Rabbit polyclonal anti-FLAG | abcam | Cat#ab1162; RRID: AB_298215 |
| Rabbit polyclonal anti-TENT4A | Atlas | Cat#HPA045487; RRID: AB_2679346 |
| Mouse polyclonal anti-TENT4B | Kim et al | N/A |
| Rabbit polyclonal anti-eGFP | Invtrogen | Cat#CAB4211; RRID: AB_10709851 |
| Rabbit monoclonal anti-$\alpha$-Tubulin | abcam | Cat#ab52866; RRID: AB_869989 |
| Rabbit polyclonal anti-HA | Invitrogen | Cat#71-5500; RRID: AB_87935 |
| Bacterial and virus strains | | |
| pAAV-CAG-GFP | Addgene | Cat#37825 |
| pAAV-CAG-GFP (no WPRE) | This study | N/A |
| pAAV-CAG-GFP-K5 | This study | N/A |
| pAAV-CAG-GFP-K5m | This study | N/A |
| pAAV-CAG-GFP-eK5 | This study | N/A |
| pAAV-CAG-GFP-eK5m | This study | N/A |
| pVVV-DJ | Addgene | Cat#104963 |
| pAdDeltaF6 | Addgene | Cat#112867 |
| psPAX | This study | N/A |
| pMD2.G | This study | N/A |
| pLENTI EGFP K5 | This study | N/A |
| Endura Electrocompetent cell | Lucigen | Cat#LU60242-2 |
| Chemicals, peptides, and recombinant proteins | | |
| RO0321 | Glixx Laboratories Inc | Cat#GLXC-11004 |
| RG7834 | Glixx Laboratories Inc | Cat#GLXC-221188 |
| Cycloheximide | Sigma-Aldrich | Cat#C4859-1ML |
| Critical commercial assays | | |
| DMEM | WELGENE | Cat#LM001-05 |
| McCoy's 5A Medium | WELGENE | Cat#LM005-1 |
| FBS | WELGENE | Cat#S001-01 |
| Q5® High-Fidelity 2X Master Mix | NEB | Cat#M0492 |
| Notl-HF | NEB | Cat#R3189S |
| Sacl-HF | NEB | Cat#R3156S |
| T4 DNA Ligase | NEB | Cat#M0202M |
| Zymo Oligo Clean & Concentrator kit | Zymo Research | Cat#D4061 |

23

(continued)

| Critical commercial assays | | |
|---|---|---|
| SYBRgold | Invitrogen | Cat#S11494 |
| Lipofectamine 3000 Transfection Reagent | Invitrogen | Cat#L3000001 |
| Allprep RNA/DNA Mini Kit | Qiagen | Cat#80004 |
| Recombinant DNase I | TAKARA | Cat#2270A |
| SSIV reverse transciptase | Invitrogen | Cat#18090010 |
| Digitonin | Merck | Cat#D141 |
| SUPERas In RNase Inhibitor | Ambion | Cat#AM2696 |
| Protease inhibitor | Calbiochem | Cat#535140 |
| Phosphatase inhibitor | Merck | Cat#P0044 |
| D(+)-Sucrose | Acros Organics | Cat#AC419760050 |
| Gradient Master™ | Biocomp | Cat#B108-2 |
| SW41Ti rotor | Beckman coulter | Cat#331362 |
| Beckman Coulter Ultracentrifuge Optima XE | Beckman coulter | Cat#A94471 |
| Biologic LP system with Model 2110 fraction collector | Bio-Rad | Cat#7318303 |
| EM-1 Econo UV detector | Bio-Rad | Cat#7318162 |
| TRIzol™ LS Reagent | Life Technologies | Cat#10296-028 |
| TRIzol | Life Technologies | Cat#15596-018 |
| Direct-Zol RNA Miniprep kit | Zymo Research | Cat#R2052 |
| Dual-luciferase reporter assay system | Promega | Cat#E4550 |
| RNeasy Mini Kit | Qiagen | Cat#74106 |
| DNase | Qiagen | Cat#79254 |
| Primescript RTmix | Takara | Cat#RR036A |
| SYBR Green | Life Technologies | Cat#4367659 |
| StepOnePlus Real-Time PCR System | Applied Biosystems | Cat#4376599 |
| QuantStudio 3 | Applied Biosystems | Cat#A28132 |
| MiSeq Reagent Kit v2 (300-cycles) | Illumina | Cat#15033412 |
| Truseq Strnd Total RNA LP Gold | Illumina | Cat#20020599 |
| PhiX control v3 kit | Illumina | Cat#FC-110-3001 |
| AAV Quantitation kit | cell biolabs | Cat#VPL-145 |
| AAV purification kit | cell biolabs | Cat#VPK-140 |
| BD Accuri C6 Plus flow cytometer | BD accuri | Cat#660517 |
| mMESSAGE mMACHINE™ T7 Transcription Kit | Invitrogen | Cat#AM 1344 |
| CleanCap(R) Reagent AG (3' OMe) | TriLink Biotechnologies | Cat#N-7413-10 |
| NTPs | NEB | Cat#N0450S |

(continued)

| Critical commercial assays | | |
|---|---|---|
| RNeasy MiniElute Cleanup Kit | Qiagen | Cat#74204 |
| RIPA lysis and extraction buffer | Thermo | Cat#89901 |
| Novex WedgeWell 10-20%Tris-Glycine Mini Gels | Invitrogen | Cat#XP10202BOX |
| Novex WedgeWell 4-12% Tris-Glycine Mini Gels | Invitrogen | Cat#SP04122BOX |
| Protein ladder | Thermo | Cat#26616 |
| Protein ladder | Thermo | Cat#26619 |
| PVDF | Millipore | Cat#88518 |
| poly(A) Tail-Length Assay kit | Affymetrix | Cat#76455 |
| T4 RNA ligase 2, truncated KQ | NEB | Cat#M0373L |
| RNase T1 | Thermo Scientific | Cat#EN0541 |
| Dynabead M-280 | Thermo Scientific | Cat#11204D |
| poly(A) Polymerase, Yeast | Thermo Scientific | Cat#74225Z25KU |
| Metafectene | Biontex | Cat#T020 |
| Lipofectamine mMAX | Life Technologies | Cat#LMRNA015 |
| Biotin | Sigma | Cat#B4639 |
| Pierce streptavidin beads | Thermo | Cat#88816 |
| HiPPR | Thermo | Cat#88305 |
| ZipTip C18 resin | Millipore | Cat#ZTC18S960 |
| Orbitrap Eclipse Tribrid | Thermo | Cat#FSN04-10000 |
| RNase A | Thermo | Cat#EN0531 |
| ANTI-FLAG® M2 Affinity Gel | Merck | Cat#A2220-10ML; RRID: AB_10704031 |
| HEPES | Ambion | Cat#AM9851 |
| HEPES | Ambion | Cat#AM9856 |
| Normal rabbit IgG | Cell Signaling | Cat#2729S |
| Protein A magnetic beads | Life Technologies | Cat#10002D |
| Subcellular protein fractionation kit | Thermo Scientific | Cat#78840 |
| SuperSignal West Pico PLUS Chemiluminescent | Thermo Scientific | Cat#34580 |
| SuperSignal West Pico femto Chemiluminescen | Thermo Scientific | Cat#34905 |
| ChemiDoc XRS+ System | Bio-Rad | Cat#1708265 |
| Deposited data | | |
| Analysis code | This study | https://github.com/Jen2Seo /viromics-screen-MPRA |
| MPRA - RNA abundance | This study | 10.5281/zenodo.6777910 |
| MPRA - polysome fractionation | This study | 10.5281/zenodo.6717932 |
| MPRA - Secondary mutagenesis | This study | 10.5281/zenodo.6696870 |

(continued)

| Deposited data | | |
|---|---|---|
| MPRA - Nucleocytoplasmic frac-tionation | This study | 10.5281/zenodo.7773943 |
| Gene-specific TAIL-seq | This study | 10.5281/zenodo.6786179 |
| RaPID mass spectrometry | This study | PXD041296 |
| RNA pull-down Mass spectrometry | Kim et. al. | PXD018061 |
| Experimental models: Cell lines | | |
| Human/HCT116 | ATCC | Cat#CCL-247 |
| Human/293AAV | Cell biolabs | Cat#AAV-100 |
| Human/Lenti-X293T | Clontech | Cat#632180 |
| Oligonucleotides | | |
| The oligonucleotides used in this study were listed in Table 1 | This study | N/A |
| MPRA screening oligos | Synbio Technologies | Sequence information in https://github.com/Jen2 Seo/ viromics-screen-MPRA/ |
| Recombinant DNA | | |
| The plasmids used in this study were listed in Table 1 | This study | N/A |
| Software and algorithms | | |
| Bowtie2.2.6 | Langmead and Salzberg | http://bowtie-bio.sourceforge.net/bowtie2/ index.shtml |
| mpra-package (MPRAnalyze) | Ashauach et al. | https://rdrr.io/bioc/mpra/man /mpra-package.html |
| SciPy 1.4.1 | Virtanen et al. | https://www.scipy.orq/; RRID: SCR_008058 |
| Tailseeker 3.1.5 | Chang et al. | https: //github.com/hyeshik/ta ilseeker |
| Dragon PolyA spotter ver. 1.2 | Kalkatawi et al. | https://mybiosoftware.com/d ragon-polya-spotter-1-1-predictor-polya-motifs-human-genomic-dna-sequences.html |
| RNAFold | Gruber et al. | http://rna.tbi.univie.ac.at//c gi-bin/RNAWebSuite/RNAfold. cqi?PAGE=3&ID=0LRrlcG16 z&r=57 |
| IPKnot | Sato et al. | https://github.com/satoken/ip knot |
| RNAstructure | Reuter et al. | https://rna.urmc.rochester.e du/RNAstructure.html |
| CENTROIDFOLD | Sato et al. | https://www.ncrna.org/centro idfold/ |
| CONTRAfold | Do et al. | https://bio.tools/contrafold |
| Contextfold | Zakov et al. | https://www.cs.bgu.ac.il/~ne gevcb/contextfold/ |
| DESeq2 | Love etl al. | https://bioconductor.org/pa ckages/release/-bioc/html/D ESeq2.html |
| ClustalOmega | Sievers et al. | https://www.ebi.ac.uk/Tools/ msa/clustalo/ |
| FigTree v1.4.4 | Rambaut and Drummond | http://tree.bio.ed.ac.uk/softw are/figtree/ |
| forna | Kerpedjev et al. | https:/lbio.tools/forna |
| MaxQuant v.1.5.3.30 | Cox and Mann | https://www.maxquant.org/ |
| Limma | Smyth, G.K. | http://bioconductor.org/pack ages/release/-bioc/html/limm a.html |

(continued)

| Software and algorithms | | |
|---|---|---|
| UniProt Align tool | UniProt | https://www.uniprot.org/align |
| MSFragger1 v3.7 | Kong et al. | https://fragpipe.nesvilab.org/ |
| IonQuant2 v1.8.10 | Yu et al. | https://fragpipe.nesvilab.org/ |
| Philosopher3 v4.8.1 | da Veiga et al. | https://fragpipe.nesvilab.org/ |
| **Other** | | |
| Virus genome sequences | NCBI | https://www.ncbi.nlm.nih.gov /labs/virus/vssi/#/ |
| Swiss-Prot human database4 | Swiss-prot Group | https://www.uniprot.org/dow nloads |

**[Examples]**

**1.Viromic screens to identify regulatory RNA elements**

To build a library of viral RNA elements, a two-step approach was used due to the technical limitations of oligo synthesis: the initial screens were performed with human viruses, followed by expanding the secondary screen to include other related species. To identify viruses that can infect humans, the NCBI database, which currently annotates 502 human viral species that belong to 114 genera and 40 families, was used.

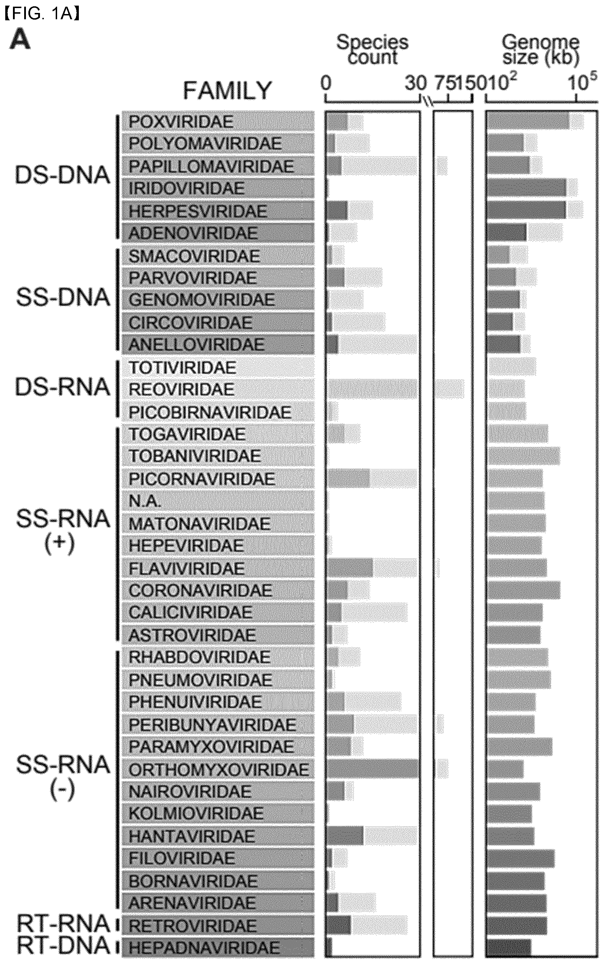

As shown in FIG. 1A and Table 3, after manual inspection, 143 species representing 96 genera and 37 families were selected, and the species with close sequence similarity and those that are either classified ambiguously or lacking clear evidence for human infection were excluded. The catalog of the present disclosure covers all seven groups of the Baltimore classification system. For RNA viruses, the whole-genome sequence was used. For DNA viruses, which generally have larger genomes, untranslated regions (UTRs) and non-coding genes were included.

【Table 3】

| Genome Type | Family | Genus | Name | Segment | RefSeq ID |
|---|---|---|---|---|---|
| DS-DNA | ADENO VIRIDAE | MASTADE NOVIRUS | HUMAN MASTADENOVIRUS A | GEN OME | NC_001460.1 |
| | HERPES VIRIDAE | CYTOMEG ALOVIRUS | HUMAN BETAHERPESVIRUS 5 (HHV-5; HCMV) | GEN OME | NC_006273.2 |
| | | LYMPHOC RYPTOVI RUS | HUMAN GAMMAHERPESVIRU S 4 (EPSTEIN-BARR VIRUS) | GEN OME | NC_007605.1 |
| | | RHADINO VIRUS | HUMAN GAMMAHERPESVIRU S 8 (KAPOSI'S SARCOMA- ASSOCIATED HERPESVIRUS) | GEN OME | NC_009333.1 |
| | | ROSEOLO VIRUS | HUMAN BETAHERPESVIRUS 6B (HHV-6B) | GEN OME | NC_000898.1 |
| | | SIMPLEXV IRUS | HUMAN ALPHAHERPESVIRUS | GEN OME | NC_001806.2 |

| | | 1 (HERPES SIMPLEX VIRUS 1) | | |
|---|---|---|---|---|
| | | HUMAN ALPHAHERPESVIRUS 2 (HERPES SIMPLEX VIRUS 2) | GEN OME | NC_001798.2 |
| | VARICELL OVIRUS | HUMAN ALPHAHERPESVIRUS 3 (HHV-3) | GEN OME | NC_001348.1 |
| IRIDOVI RIDAE | MEGALOC YTIVIRUS | INFECTIOUS SPLEEN AND KIDNEY NECROSIS VIRUS (ISKNV) | GEN OME | NC_003494.1 |
| PAPILLO MAVIRID AE | ALPHAPA PILLOMAV IRUS | HUMAN PAPILLOMAVIRUS TYPE 16 | GEN OME | NC_001526.4 |
| | BETAPAPI LLOMAVIR US | HUMAN PAPILLOMAVIRUS 5 | GEN OME | NC_001531.1 |
| | GAMMAP APILLOMA VIRUS | HUMAN PAPILLOMAVIRUS 4 | GEN OME | NC_001457.1 |
| | MUPAPILL OMAVIRU S | HUMAN PAPILLOMAVIRUS TYPE 63 | GEN OME | NC_001458.1 |
| | NUPAPILL OMAVIRU S | HUMAN PAPILLOMAVIRUS TYPE 41 | GEN OME | NC_001354.1 |
| POLYO MAVIRID AE | ALPHAPO LYOMAVI RUS | MERKEL CELL POLYOMAVIRUS | GEN OME | NC_010277.2 |
| | BETAPOL YOMAVIR US | JC POLYOMAVIRUS (JCPYV) | GEN OME | NC_001699.1 |
| | DELTAPO LYOMAVI RUS | HUMAN POLYOMAVIRUS 6 | GEN OME | NC_014406.1 |
| POXVIRI DAE | CENTAPO XVIRUS | NY_014 POXVIRUS | GEN OME | NC_035469.1 |

| | | MOLLUSC IPOXVIRUS | MOLLUSCUM CONTAGIOSUM VIRUS SUBTYPE 1 | GEN OME | NC_001731.1 |
|---|---|---|---|---|---|
| | | ORTHOPO XVIRUS | COWPOX VIRUS | GEN OME | NC_003663.2 |
| | | | VACCINIA VIRUS | GEN OME | NC_006998.1 |
| | | | VARIOLA VIRUS | GEN OME | NC_001611.1 |
| | | PARAPOX VIRUS | ORF VIRUS | GEN OME | NC_005336.1 |
| | | YATAPOX VIRUS | YABA-LIKE DISEASE VIRUS | GEN OME | NC_002642.1 |
| SS-DNA | SMACO VIRIDAE | HUCHISM ACOVIRU S | HUMAN ASSOCIATED HUCHISMACOVIRUS 1 | GEN OME | NC_039061.1 |
| | | PORPRIS MACOVIR US | HUMAN FECES SMACOVIRUS 2 | GEN OME | NC_039070.1 |
| | ANELLO VIRIDAE | ALPHATO RQUEVIR US | TORQUE TENO VIRUS 1 | GEN OME | NC_002076.2 |
| | | BETATOR QUEVIRU S | TORQUE TENO MINI VIRUS 1 | GEN OME | NC_014097.1 |
| | | GAMMAT ORQUEVI RUS | TORQUE TENO MIDI VIRUS 1 | GEN OME | NC_009225.1 |
| | | GYROVIR US | AVIAN GYROVIRUS 2 | GEN OME | NC_015396.1 |
| | CIRCOVI RIDAE | CIRCOVIR US | PORCINE CIRCOVIRUS 2 | GEN OME | NC_005148.1 |
| | | CYCLOVI RUS | HUMAN CYCLOVIRUS VS5700009 | GEN OME | NC_021568.1 |
| | GENOM OVIRIDA E | GEMYCIR CULARVIR US | GEMYCIRCULARVIRU S HV-GCV1 | GEN OME | NC_030447.1 |
| | PARVOV IRIDAE | BOCAPAR VOVIRUS | PRIMATE BOCAPARVOVIRUS 1 | GEN OME | NC_007455.1 |

| | | DEPENDO PARVOVIRUS | ADENO-ASSOCIATED VIRUS - 1 | GENOME | NC_002077.1 |
|---|---|---|---|---|---|
| | | ERYTHRO PARVOVIRUS | HUMAN PARVOVIRUS B19 | GENOME | NC_000883.2 |
| | | UNCLASSIFIED PARVOVIRINAE | PARVOVIRUS NIH-CQV | GENOME (PARTIAL) | NC_022089.1 |
| | | PROTOPARVOVIRUS | CUTAVIRUS | GENOME (PARTIAL) | NC_039050.1 |
| | | TETRAPARVOVIRUS | HUMAN PARVOVIRUS 4 G1 | GENOME | NC_007018.1 |
| DS-RNA | PICOBIRNAVIRIDAE | PICOBIRNAVIRUS | HUMAN PICOBIRNAVIRUS | SEGMENT 1 | NC_007026.1 |
| | | | | SEGMENT 2 | NC_007027.1 |
| | REOVIRIDAE | ORBIVIRUS | GREAT ISLAND VIRUS (GIV) | SEGMENT 1 | NC_014522.1 |
| | | | | SEGMENT 10 | NC_014531.1 |
| | | | | SEGMENT 2 | NC_014523.1 |
| | | | | SEGMENT 3 | NC_014524.1 |
| | | | | SEGMENT 4 | NC_014525.1 |

| | | | | SEGMENT 5 | NC_014526.1 |
|---|---|---|---|---|---|
| | | | | SEGMENT 6 | NC_014527.1 |
| | | | | SEGMENT 7 | NC_014528.1 |
| | | | | SEGMENT 8 | NC_014529.1 |
| | | | | SEGMENT 9 | NC_014530.1 |
| | | ORTHOREOVIRUS | MAMMALIAN ORTHOREOVIRUS 3 | SEGMENT L1 | NC_013225.1 |
| | | | | SEGMENT L2 | NC_013226.1 |
| | | | | SEGMENT L3 | NC_013229.1 |
| | | | | SEGMENT M1 | NC_013227.1 |
| | | | | SEGMENT M2 | NC_013228.1 |
| | | | | SEGMENT M3 | NC_013230.1 |
| | | | | SEGMENT S1 | NC_013231.1 |
| | | | | SEGMENT S2 | NC_013232.1 |

| | | | | SEGMENT S3 | NC_013233.1 |
|---|---|---|---|---|---|
| | | | | SEGMENT S4 | NC_013234.1 |
| | | ROTAVIRUS | ROTAVIRUS A | SEGMENT 1 | NC_011507.2 |
| | | | | SEGMENT 10 | NC_011504.2 |
| | | | | SEGMENT 11 | NC_011505.2 |
| | | | | SEGMENT 2 | NC_011506.2 |
| | | | | SEGMENT 3 | NC_011508.2 |
| | | | | SEGMENT 4 | NC_011510.2 |
| | | | | SEGMENT 5 | NC_011500.2 |
| | | | | SEGMENT 6 | NC_011509.2 |
| | | | | SEGMENT 7 | NC_011501.2 |
| | | | | SEGMENT 8 | NC_011502.2 |
| | | | | SEGMENT 9 | NC_011503.2 |

| | | | | SEGMENT 1 | NC_004211.1 |
|---|---|---|---|---|---|
| | | | | SEGMENT 10 | NC_004201.1 |
| | | | | SEGMENT 11 | NC_004200.1 |
| | | | | SEGMENT 12 | NC_004198.1 |
| | | | | SEGMENT 2 | NC_004217.1 |
| | | SEADORNAVIRUS | BANNA VIRUS STRAIN JKT-6423 | SEGMENT 3 | NC_004218.1 |
| | | | | SEGMENT 4 | NC_004219.1 |
| | | | | SEGMENT 5 | NC_004220.1 |
| | | | | SEGMENT 6 | NC_004221.1 |
| | | | | SEGMENT 7 | NC_004204.1 |
| | | | | SEGMENT 8 | NC_004203.1 |
| | | | | SEGMENT 9 | NC_004202.1 |
| | TOTIVIRIDAE | UNCLASSIFIED | TRICHOMONAS VAGINALIS VIRUS | GENOME | NC_003824.1 |

| | | TOTIVIRIDAE | | | |
|---|---|---|---|---|---|
| SS-POS-RNA | ASTROVIRIDAE | MAMASTROVIRUS | ASTROVIRUS MLB1 | GENOME | NC_011400.1 |
| | | UNCLASSIFIED ASTROVIRIDAE | HUMAN ASTROVIRUS | GENOME | NC_001943.1 |
| | CALICIVIRIDAE | NOROVIRUS | NOROVIRUS GI | GENOME | NC_001959.2 |
| | | | NOROVIRUS GII | GENOME | NC_039477.1 |
| | | | NOROVIRUS GV | GENOME | NC_008311.1 |
| | | SAPOVIRUS | SAPOVIRUS HU/DRESDEN/PJG-SAP01/DE | GENOME | NC_006269.1 |
| | | VESIVIRUS | VESICULAR EXANTHEMA OF SWINE VIRUS | GENOME | NC_002551.1 |
| | CORONAVIRIDAE | ALPHACORONAVIRUS | HUMAN CORONAVIRUS 229E | GENOME | NC_002645.1 |
| | | | HUMAN CORONAVIRUS NL63 (HCOV-NL63) | GENOME | NC_005831.2 |
| | | BETACORONAVIRUS | HUMAN CORONAVIRUS HKU1 (HCOV-HKU1) | GENOME | NC_006577.2 |
| | | | HUMAN CORONAVIRUS OC43 (HCOV-OC43) | GENOME | NC_006213.1 |
| | | | MIDDLE EAST RESPIRATORY SYNDROME-RELATED CORONAVIRUS (MERS-COV) | GENOME | NC_019843.3 |
| | | | SARS CORONAVIRUS TOR2 | GENOME | NC_004718.3 |

| | | | SEVERE ACUTE RESPIRATORY SYNDROME CORONAVIRUS 2 (SARS-COV-2) | GEN OME | NC_045512.2 |
|---|---|---|---|---|---|
| FLAVIVI RIDAE | FLAVIVIR US | | DENGUE VIRUS 1 | GEN OME | NC_001477.1 |
| | | | DENGUE VIRUS 2 | GEN OME | NC_001474.2 |
| | | | DENGUE VIRUS 3 | GEN OME | NC_001475.2 |
| | | | DENGUE VIRUS 4 | GEN OME | NC_002640.1 |
| | | | JAPANESE ENCEPHALITIS VIRUS | GEN OME | NC_001437.1 |
| | | | SAINT LOUIS ENCEPHALITIS VIRUS | GEN OME | NC_007580.2 |
| | | | TICK-BORNE ENCEPHALITIS VIRUS | GEN OME | NC_001672.1 |
| | | | WEST NILE VIRUS (WNV) | GEN OME | NC_001563.2 |
| | | | YELLOW FEVER VIRUS (YFV) | GEN OME | NC_002031.1 |
| | | | ZIKA VIRUS | GEN OME | NC_012532.1 |
| | HEPACIVI RUS | | HEPATITIS C VIRUS GENOTYPE 1 | GEN OME | NC_004102.1 |
| | | | HEPATITIS GB VIRUS B | GEN OME | NC_001655.1 |
| | PEGIVIRU S | | GB VIRUS C (GBV-HGV) | GEN OME | NC_001710.1 |
| | | | PEGIVIRUS A | GEN OME | NC_001837.1 |
| | PESTIVIR US | | BOVINE VIRAL DIARRHEA VIRUS 1 (BVDV-1) | GEN OME | NC_001461.1 |

| | | | | |
|---|---|---|---|---|
| HEPEVIRIDAE | ORTHOHEPEVIRUS | HEPATITIS E VIRUS | GENOME | NC_001434.1 |
| MATONAVIRIDAE | RUBIVIRUS | RUBELLA VIRUS | GENOME | NC_001545.2 |
| N.A. | HUSAVIRUS | HUSAVIRUS SP. | GENOME | NC_032480.1 |
| PICORNAVIRIDAE | CARDIOVIRUS | ENCEPHALOMYOCARDITIS VIRUS | GENOME | NC_001479.1 |
| | | SAFFOLD VIRUS | GENOME | NC_009448.2 |
| | COSAVIRUS | COSAVIRUS A | GENOME | NC_012800.1 |
| | ENTEROVIRUS | ENTEROVIRUS A | GENOME | NC_001612.1 |
| | | ENTEROVIRUS B | GENOME | NC_001472.1 |
| | | ENTEROVIRUS C | GENOME | NC_002058.3 |
| | | ENTEROVIRUS D | GENOME | NC_001430.1 |
| | | HUMAN RHINOVIRUS A1 (HRV-A1) | GENOME | NC_038311.1 |
| | | RHINOVIRUS B14 | GENOME | NC_001490.1 |
| | HEPATOVIRUS | HEPATOVIRUS A | GENOME | NC_001489.1 |
| | KOBUVIRUS | AICHI VIRUS 1 | GENOME | NC_001918.1 |
| | PARECHOVIRUS | PARECHOVIRUS A | GENOME | NC_001897.1 |
| | ROSAVIRUS | ROSAVIRUS A2 | GENOME | NC_024070.1 |
| | SALIVIRUS | SALIVIRUS A | GENOME | NC_012986.1 |
| TOBANIVIRIDAE | TOROVIRUS | BREDA VIRUS | GENOME | NC_007447.1 |
| TOGAVIRIDAE | ALPHAVIRUS | BARMAH FOREST VIRUS | GENOME | NC_001786.1 |

| | | | CHIKUNGUNYA VIRUS | GEN OME | NC_004162.2 |
|---|---|---|---|---|---|
| | | | EASTERN EQUINE ENCEPHALITIS VIRUS | GEN OME | NC_003899.1 |
| | | | SEMLIKI FOREST VIRUS | GEN OME | NC_003215.1 |
| | | | VENEZUELAN EQUINE ENCEPHALITIS VIRUS (VEEV) | GEN OME | NC_001449.1 |
| | | | WESTERN EQUINE ENCEPHALITIS VIRUS | GEN OME | NC_003908.1 |
| SS-NEG-RNA | ARENAV IRIDAE | MAMMAR ENAVIRU S | ARGENTINIAN MAMMARENAVIRUS | SEG MEN T L | NC_005080.1 |
| | | | | SEG MEN T S | NC_005081.1 |
| | | | LYMPHOCYTIC CHORIOMENINGITIS MAMMARENAVIRUS (LCMV) | SEG MEN T L | NC_004291.1 |
| | | | | SEG MEN T S | NC_004294.1 |
| | BORNA VIRIDAE | ORTHOBO RNAVIRU S | BORNA DISEASE VIRUS 1 (BODV-1) | GEN OME | NC_001607.1 |
| | FILOVIRI DAE | EBOLAVIR US | ZAIRE EBOLAVIRUS | GEN OME | NC_002549.1 |
| | | MARBUR GVIRUS | MARBURG MARBURGVIRUS | GEN OME | NC_001608.3 |
| | HANTAV IRIDAE | ORTHOHA NTAVIRUS | ANDES ORTHOHANTAVIRUS | SEG MEN T L | NC_003468.2 |
| | | | | SEG MEN T M | NC_003467.2 |

| | | | | |
|---|---|---|---|---|
| | | | SEGMENT S | NC_003466.1 |
| | | HANTAAN ORTHOHANTAVIRUS | SEGMENT L | NC_005222.1 |
| | | | SEGMENT M | NC_005219.1 |
| | | | SEGMENT S | NC_005218.1 |
| | | SEOUL ORTHOHANTAVIRUS | SEGMENT L | NC_005238.1 |
| | | | SEGMENT M | NC_005237.1 |
| | | | SEGMENT S | NC_005236.1 |
| | | SIN NOMBRE ORTHOHANTAVIRUS | SEGMENT L | NC_005217.1 |
| | | | SEGMENT M | NC_005215.1 |
| | | | SEGMENT S | NC_005216.1 |
| KOLMIOVIRIDAE | DELTAVIRUS | HEPATITIS DELTA VIRUS | GENOME | NC_001653.2 |
| NAIROVIRIDAE | ORTHONAIROVIRUS | CRIMEAN-CONGO HEMORRHAGIC FEVER ORTHONAIROVIRUS | SEGMENT L | NC_005301.3 |
| | | | SEGMENT M | NC_005300.2 |

| | | | | SEG MEN T S | NC_005302.1 |
|---|---|---|---|---|---|
| | | | NAIROBI SHEEP DISEASE VIRUS (NSDV) | SEG MEN T L | NC_034387.1 |
| | | | | SEG MEN T M | NC_034391.1 |
| | | | | SEG MEN T S | NC_034386.1 |
| ORTHO MYXOVI RIDAE | ALPHAINF LUENZAVI RUS | | INFLUENZA A VIRUS (A/NEW YORK/392/2004(H3N2 )) | SEG MEN T 1 | NC_007373.1 |
| | | | | SEG MEN T 2 | NC_007372.1 |
| | | | | SEG MEN T 3 | NC_007371.1 |
| | | | | SEG MEN T 4 | NC_007366.1 |
| | | | | SEG MEN T 5 | NC_007369.1 |
| | | | | SEG MEN T 6 | NC_007368.1 |
| | | | | SEG MEN T 7 | NC_007367.1 |
| | | | | SEG MEN T 8 | NC_007370.1 |
| | | | INFLUENZA A VIRUS (A/PUERTO RICO/8/1934(H1N1)) | SEG MEN T 1 | NC_002023.1 |

| | | | | | | |
|---|---|---|---|---|---|---|
| | | | | | SEGMENT 2 | NC_002021.1 |
| | | | | | SEGMENT 3 | NC_002022.1 |
| | | | | | SEGMENT 4 | NC_002017.1 |
| | | | | | SEGMENT 5 | NC_002019.1 |
| | | | | | SEGMENT 6 | NC_002018.1 |
| | | | | | SEGMENT 7 | NC_002016.1 |
| | | | | | SEGMENT 8 | NC_002020.1 |
| | | | BETAINFLUENZAVIRUS | INFLUENZA B VIRUS (B/LEE/1940) | SEGMENT 1 | NC_002204.1 |
| | | | | | SEGMENT 2 | NC_002205.1 |
| | | | | | SEGMENT 3 | NC_002206.1 |
| | | | | | SEGMENT 4 | NC_002207.1 |
| | | | | | SEGMENT 5 | NC_002208.1 |
| | | | | | SEGMENT 6 | NC_002209.1 |

| | | | | SEG MEN T 7 | NC_002210.1 |
|---|---|---|---|---|---|
| | | | | SEG MEN T 8 | NC_002211.1 |
| | GAMMAIN FLUENZA VIRUS | INFLUENZA C VIRUS (C/ANN ARBOR/1/50) | | SEG MEN T 1 | NC_006307.2 |
| | | | | SEG MEN T 2 | NC_006308.2 |
| | | | | SEG MEN T 3 | NC_006309.2 |
| | | | | SEG MEN T 4 | NC_006310.2 |
| | | | | SEG MEN T 5 | NC_006311.1 |
| | | | | SEG MEN T 6 | NC_006312.2 |
| | | | | SEG MEN T 7 | NC_006306.2 |
| | THOGOTO VIRUS | DHORI THOGOTOVIRUS | | SEG MEN T 1 | NC_034261.1 |
| | | | | SEG MEN T 2 | NC_034263.1 |
| | | | | SEG MEN T 3 | NC_034254.1 |
| | | | | SEG MEN T 4 | NC_034255.1 |

| | | | SEGMENT 5 | NC_034262.1 |
|---|---|---|---|---|
| | | | SEGMENT 6 | NC_034256.1 |
| PARAMYXOVIRIDAE | HENIPAVIRUS | HENDRA HENIPAVIRUS | GENOME | NC_001906.3 |
| | MORBILLIVIRUS | MEASLES MORBILLIVIRUS | GENOME | NC_001498.1 |
| | ORTHORUBULAVIRUS | HUMAN ORTHORUBULAVIRUS 2 | GENOME | NC_003443.1 |
| | | HUMAN PARAINFLUENZA VIRUS 4A | GENOME | NC_021928.1 |
| | | MUMPS ORTHORUBULAVIRUS | GENOME | NC_002200.1 |
| | PARARUBULAVIRUS | SOSUGA VIRUS | GENOME | NC_025343.1 |
| | RESPIROVIRUS | HUMAN RESPIROVIRUS 1 | GENOME | NC_003461.1 |
| | | HUMAN RESPIROVIRUS 3 | GENOME | NC_001796.2 |
| PERIBUNYAVIRIDAE | ORTHOBUNYAVIRUS | BUNYAMWERA VIRUS | SEGMENT L | NC_001925.1 |
| | | | SEGMENT M | NC_001926.1 |
| | | | SEGMENT S | NC_001927.1 |
| | | LA CROSSE VIRUS | SEGMENT L | NC_004108.1 |

| | | | | | |
|---|---|---|---|---|---|
| | | | | SEGMENT M | NC_004109.1 |
| | | | | SEGMENT S | NC_004110.1 |
| | | | OROPOUCHE VIRUS | SEGMENT L | NC_005776.1 |
| | | | | SEGMENT M | NC_005775.1 |
| | | | | SEGMENT S | NC_005777.1 |
| PHENUIVIRIDAE | BANDAVIRUS | SEVERE FEVER WITH THROMBOCYTOPENIA SYNDROME VIRUS | | SEGMENT L | NC_043450.1 |
| | | | | SEGMENT M | NC_043451.1 |
| | | | | SEGMENT S | NC_043452.1 |
| | PHLEBOVIRUS | RIFT VALLEY FEVER VIRUS | | SEGMENT L | NC_014397.1 |
| | | | | SEGMENT M | NC_014396.1 |
| | | | | SEGMENT S | NC_014395.1 |
| PNEUMOVIRIDAE | METAPNEUMOVIRUS | HUMAN METAPNEUMOVIRUS (HMPV) | | GENOME | NC_039199.1 |
| | ORTHOPNEUMOVIRUS | HUMAN ORTHOPNEUMOVIRUS (HRSV) | | GENOME | NC_001781.1 |

| | | LEDANTE VIRUS | LE DANTEC VIRUS | GENOME (PARTIAL) | NC_034443.1 |
|---|---|---|---|---|---|
| | RHABDOVIRIDAE | LYSSAVIRUS | RABIES LYSSAVIRUS | GENOME | NC_001542.1 |
| | | TIBROVIRUS | BAS-CONGO TIBROVIRUS | GENOME (PARTIAL) | NC_043067.1 |
| | | VESICULOVIRUS | CHANDIPURA VIRUS | GENOME | NC_020805.1 |
| RT-RNA | RETROVIRIDAE | BETARETROVIRUS | MOUSE MAMMARY TUMOR VIRUS | GENOME | NC_001503.1 |
| | | DELTARETROVIRUS | HUMAN T-CELL LEUKEMIA VIRUS TYPE I | GENOME | NC_001436.1 |
| | | | HUMAN T-LYMPHOTROPIC VIRUS 2 | GENOME | NC_001488.1 |
| | | GAMMARETROVIRUS | MOLONEY MURINE LEUKEMIA VIRUS (MOMLV) | GENOME | NC_001501.1 |
| | | LENTIVIRUS | HUMAN IMMUNODEFICIENCY VIRUS 1 (HIV-1) | GENOME | NC_001802.1 |
| | | | HUMAN IMMUNODEFICIENCY VIRUS 2 (HIV-2) | GENOME | NC_001722.1 |
| | | UNCLASSIFIED RETROVIRIDAE | HUMAN ENDOGENOUS RETROVIRUS K113 | GENOME | NC_022518.1 |
| | | SPUMAVIRUS | SIMIAN FOAMY VIRUS | GENOME | NC_001364.1 |
| RT-DNA | HEPADNAVIRIDAE | ORTHOHEPADNAVIRUS | HEPATITIS B VIRUS | GENOME | NC_003977.2 |
| | | | WOODCHUCK HEPATITIS VIRUS | GENOME | NC_004107.1 |

As shown in FIG. 1B, oligos for the screen were designed by tiling the viral genomes with a sliding window size of 130-nt and a step size of 65-nt, generating 30,367 segments in total. Each segment was prepared with three different barcodes for reliable detection. As positive controls, four segments harboring the "1E" element from lncRNA2.7 of human cytomegalovirus (HCMV) and one segment with woodchuck PRE (WPRE) from woodchuck hepatitis virus, known to enhance gene expression, were included (FIG. 8). As nonfunctional controls, the corresponding mutants (1Em) that contain inactivating mutations in the loop of 1E were used. After synthesis, the oligos were amplified by PCR and inserted into the 3' UTR of a luciferase reporter plasmid. The constructed library contained a total of 91,101 reporter plasmids, covering 30,367 segments from 143 human viruses and one woodchuck hepatitis virus.

For functional assessment, the plasmid pool was transfected into the human colon cancer cell line (HCT116) to quantify the impact of each element on gene expression (FIG. 1B). To monitor the effect on RNA abundance, both the plasmids and mRNAs were extracted, amplified, and sequenced to calculate the ratio between the read proportion of mRNA to the read proportion of transfected DNA ('RNA/DNA'). To search for translation-modulatory elements, sucrose gradient centrifugation was used to separate the cytoplasmic extract into five fractions (free mRNA, monosomes, light polysomes (LP), medium polysomes (MP), and heavy polysomes (HP)), and the extract was used for RNA extraction and sequencing to estimate translation efficiency for each UTR.

## 2. Identification of regulatory RNA elements

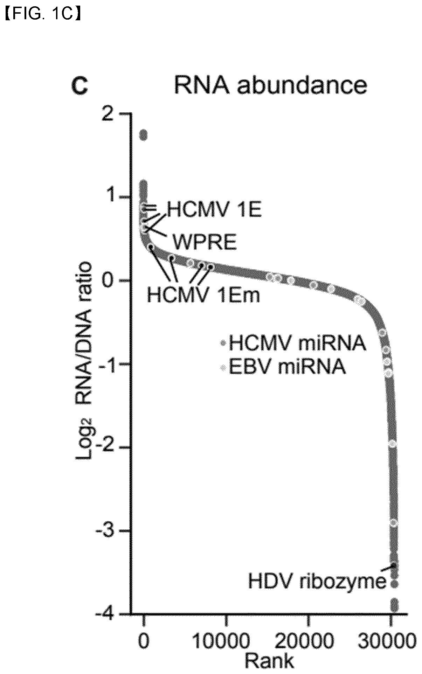

To determine the effect of 30,302 viral segments (30,190 segments with all three barcodes detected) on mRNA abundance, the following experiment was conducted. The experiment results were reproducible between quadruplicate experiments and between barcodes. In detail, the positive controls spanning 1E and WPRE increased mRNA levels relative to the 1E mutants (FIG. 1C). 245 upregulating segments and 628 downregulating segments were identified. As expected, segments that increased mRNA abundance included stem-loop alpha of human HBV, which is part of PRE known to enhance mRNA stability. Negative elements included RNAs cleaved by endonucleolytic enzymes, such as the self-cleaving ribozyme from hepatitis D virus (HDV), and microRNA loci from HCMV (also known as human betaherpesvirus 5) and Epstein-Barr virus, which are likely cleaved by DROSHA, resulting in reporter mRNA decay (FIG. 1C).

Thus, segments that stabilize RNA ($\text{Log}_2$(RNA/DNA) > 0.5, p-value < 0.05) or destabilize RNA ($\text{Log}_2$(RNA/DNA) < -1, p-value < 0.001) were effectively identified through this experiment (Tables 4 and 5). The 50 segments in Table 4 were found to exhibit excellent RNA abundance, with $\text{Log}_2$(RNA/DNA) values similar to or higher than those of the positive controls WPRE or HCMV 1E (FIG. 1C).

Segments that stabilize RNA

[Table 4]

| Rank | Virus Name | NCBI ID | Start | End | log2 RNA/DNA ratio | TILE ID | SEQ. ID |
|---|---|---|---|---|---|---|---|
| 1 | HUMAN_GAMMA HERPESVIRUS_ 4_(EPSTEIN-BARR_VIRUS) | NC_00 7605.1 | 88961 | 88832 | 1.7565 | TILE_ID_13 8-00443 | 1 |
| 2 | ENCEPHALOMY OCARDITIS_VIR US | NC_00 1479.1 | 196 | 325 | 1.7179 | TILE_ID_06 6-00004 | 2 |
| 3 | HUMAN_BETAH ERPESVIRUS_5_ (HHV-5_HCMV) | NC_00 6273.2 | 96273 | 96402 | 1.1516 | TILE_ID_14 3-00201 | 3 |
| 4 | ORF_VIRUS | NC_00 5336.1 | 1E+05 | 1E+05 | 1.1381 | TILE_ID_13 3-00301 | 4 |
| 5 | MOLLUSCUM_C ONTAGIOSUM_V IRUS_SUBTYPE _1 | NC_00 1731.1 | 2E+05 | 2E+05 | 1.1065 | TILE_ID_14 0-00299 | 5 |
| 6 | BORNA_DISEAS E_VIR US_1_(BO DV-1) | NC_00 1607.1 | 3368 | 3497 | 1.0742 | TILE_ID_07 6-00050 | 6 |
| 7 | HUSAVIRUS_SP. | NC_03 2480.1 | 6695 | 6824 | 1.0331 | TILE_ID_07 5-00103 | 7 |

(continued)

| Ra nk | Virus Name | NCBI ID | Start | End | log2 RNA/DNA ratio | TILE ID | SEQ. ID |
|---|---|---|---|---|---|---|---|
| 8 | HUMAN_GAMMA HERPES-VIRUS_ 4_(EPSTEIN-BARR_VIRUS) | NC_00 7605.1 | 89026 | 88897 | 1.0057 | TILE_ID_13 8-00442 | 8 |
| 9 | POSITIVE_CONT ROL(SL27) | GU937 742.2 | 110 | 240 | 0.9327 | TILE_ID_14 4-00012 | 9 |
| 10 | POSITIVE_CONT ROL(SL27) | GU937 742.2 | 100 | 230 | 0.894 | TILE_ID_14 4-00011 | 10 |
| 11 | SAINT_LOUIS_E NCEPHALITIS_VI RUS | NC_00 7580.2 | 10613 | 10742 | 0.8586 | TILE_ID_09 3-00163 | 11 |
| 12 | BREDA_VIRUS | NC_00 7447.1 | 7510 | 7639 | 0.857 | TILE_ID_12 3-00116 | 12 |
| 13 | POSITIVE_CONT ROL(SL27) | GU937 742.2 | 90 | 220 | 0.8544 | TILE_ID_14 4-00010 | 13 |
| 14 | HUMAN_CORON AVIRUS_OC43_( HCOV-OC43) | NC_00 6213.1 | 7281 | 7410 | 0.8456 | TILE_ID_12 8-00113 | 14 |
| 15 | SIN_NOMBRE_O RTHOHANTAVIR US | NC_00 5216.1 | 1561 | 1690 | 0.8431 | TILE_ID_02 4-00025 | 15 |
| 16 | MOLLUSCUM_C ONTAGIOSUM_V IRUS_SUBTYPE _1 | NC_00 1731.1 | 2E+05 | 2E+05 | 0.8089 | TILE_ID_14 0-00298 | 16 |
| 17 | HUMAN_BETAH ERPESVIRUS_5_ (HHV-5_HCMV) | NC_00 6273.2 | 4579 | 4450 | 0.7902 | TILE_ID_14 3-00440 | 17 |
| 18 | HUMAN_CORON AVIRUS_HKU1_( HCOV-HKU1) | NC_00 6577.2 | 15809 | 15938 | 0.7896 | TILE_ID_12 6-00243 | 18 |
| 19 | MARBURG_MAR BURGVIRUS | NC_00 1608.3 | 18484 | 18613 | 0.7854 | TILE_ID_12 0-00285 | 19 |
| 20 | AICHI_VIRUS_1 | NC_00 1918.1 | 8122 | 8251 | 0.7599 | TILE_ID_07 0-00126 | 20 |
| 21 | WEST_NILE_VIR US_(WNV) | NC_00 1563.2 | 8132 | 8261 | 0.7515 | TILE_ID_09 4-00124 | 21 |
| 22 | HUMAN_CORON AVIRUS_HKU1_( HCOV-HKU1) | NC_00 6577.2 | 7411 | 7540 | 0.75 | TILE_ID_12 6-00115 | 22 |
| 23 | SIMIAN_FOAMY_ VIRUS | NC_00 1364.1 | 2272 | 2401 | 0.7461 | TILE_ID_10 8-00035 | 23 |
| 24 | BUNYAMWERA_ VIRUS | NC_00 1925.1 | 5851 | 5980 | 0.7443 | TILE_ID_00 8-00173 | 24 |
| 25 | MOLLUSCUM_C ONTAGIOSUM_V IRUS_SUBTYPE _1 | NC_00 1731.1 | 72311 | 72182 | 0.7443 | TILE_ID_14 0-00585 | 25 |
| 26 | HUMAN_BETAH ERPESVIRUS_5_ (HHV-5_HCMV) | NC_00 6273.2 | 4644 | 4515 | 0.7434 | TILE_ID_14 3-00439 | 26 |
| 27 | COWPOX_VIRU S | NC_00 3663.2 | 29398 | 29269 | 0.7319 | TILE_ID_14 2-00551 | 27 |
| 28 | POSITIVE_CONT ROL(SL27) | GU937 742.2 | 60 | 190 | 0.7278 | TILE_ID_14 4-00007 | 28 |

(continued)

| Ra nk | Virus Name | NCBI ID | Start | End | log2 RNA/DNA ratio | TILE ID | SEQ. ID |
|---|---|---|---|---|---|---|---|
| 29 | ROTAVIRUS_A | NC_01 1500.2 | 1366 | 1495 | 0.716 | TILE_ID_00 1-00110 | 29 |
| 30 | POSITIVE_CONT ROL(SL27) | GU937 742.2 | 80 | 210 | 0.7121 | TILE_ID_14 4-00009 | 30 |
| 31 | BREDA_VIRUS | NC_00 7447.1 | 2375 | 2504 | 0.7104 | TILE_ID_12 3-00037 | 31 |
| 32 | HUMAN_CORON AVIRUS_HKU1_( HCOV-HKU1) | NC_00 6577.2 | 21139 | 21268 | 0.6988 | TILE_ID_12 6-00325 | 32 |
| 33 | HUMAN_CORON AVIRUS_HKU1_( HCOV-HKU1) | NC_00 6577.2 | 15744 | 15873 | 0.6912 | TILE_ID_12 6-00242 | 33 |
| 34 | HUMAN_ORTHO PNEUMOVIRUS_ (HRSV) | NC_00 1781.1 | 14950 | 15079 | 0.6905 | TILE_ID_11 0-00230 | 34 |
| 35 | VARIOLA_VIRUS | NC_00 1611.1 | 1E+05 | 1E+05 | 0.6873 | TILE_ID_13 9-00782 | 35 |
| 36 | COWPOX_VIRU S | NC_00 3663.2 | 2E+05 | 2E+05 | 0.6851 | TILE_ID_14 2-00982 | 36 |
| 37 | COWPOX_VIRU S | NC_00 3663.2 | 2E+05 | 2E+05 | 0.6775 | TILE_ID_14 2-00298 | 37 |
| 38 | JAPANESE_ENC EPHALITIS_VIRU S | NC_00 1437.1 | 10648 | 10777 | 0.6713 | TILE_ID_09 5-00164 | 38 |
| 39 | POSITIVE_CONT ROL(SL27) | GU937 742.2 | 50 | 180 | 0.6702 | TILE_ID_14 4-00006 | 39 |
| 40 | NY_014_POXVIR US | NC_03 5469.1 | 54907 | 54778 | 0.6645 | TILE_ID_14 1-00618 | 40 |
| 41 | HANTAAN_ORT HOHANTAVIRUS | NC_00 5219.1 | 3381 | 3510 | 0.658 | TILE_ID_01 8-00079 | 41 |
| 42 | HUMAN_CORON AVIRUS_NL63_( HCOV-NL63) | NC_00 5831.2 | 17641 | 17770 | 0.6571 | TILE_ID_12 2-00272 | 42 |
| 43 | SEVERE_ACUTE _RE- SPIRATORY _SYNDRO- ME_C ORONAVIRUS_2 _(SARS-COV-2) | NC_04 5512.2 | 5851 | 5980 | 0.6529 | TILE_ID_12 5-00091 | 43 |
| 44 | NY_014_POXVIR US | NC_03 5469.1 | 2E+05 | 2E+05 | 0.6523 | TILE_ID_14 1-00868 | 44 |
| 45 | HUMAN_CORON AVIRUS_HKU1_( HCOV-HKU1) | NC_00 6577.2 | 29054 | 29183 | 0.6522 | TILE_ID_12 6-00446 | 45 |
| 46 | HUMAN_CORON AVIRUS_HKU1_( HCOV-HKU1) | NC_00 6577.2 | 7671 | 7800 | 0.6517 | TILE_ID_12 6-00119 | 46 |
| 47 | HUMAN_CORON AVIRUS_NL63_( HCOV-NL63) | NC_00 5831.2 | 4551 | 4680 | 0.6468 | TILE_ID_12 2-00071 | 47 |
| 48 | HUMAN_RHINOV IRUS_A1_(HRV-A1) | NC_03 8311.1 | 6626 | 6755 | 0.6459 | TILE_ID_04 8-00102 | 48 |
| 49 | WOODCHUCK_H EPATITIS_VIRUS | NC_00 4107.1 | 1366 | 1495 | 0.6448 | TILE_ID_03 2-00022 | 49 |

(continued)

| Rank | Virus Name | NCBI ID | Start | End | log2 RNA/DNA ratio | TILE ID | SEQ. ID |
|---|---|---|---|---|---|---|---|
| 50 | HUMAN_CORON AVIR-US_HKU1_( HCOV-HKU1) | NC_00 6577.2 | 7476 | 7605 | 0.6418 | TILE_ID_12 6-00116 | 50 |

Segments that destabilize RNA

[Table 5]

| Rank | Virus Name | NCBI ID | Start | End | log2 RNA/DNA |
|---|---|---|---|---|---|
| 1 | HUMAN_BETAHERPES VIRUS 6B (HHV-6B) | NC_0008 98.1 | 8715 | 8586 | -3.9227 |
| 2 | HUMAN_BETAHERPES VIRUS 6B (HHV-6B) | NC_0008 98.1 | 8650 | 8521 | -3.8904 |
| 3 | HUMAN_GAMMAHERPE SVIRUS_4_(EPSTEIN-BARR_VIRUS) | NC_0076 05.1 | 96564 | 96693 | -3.8478 |
| 4 | HUMAN_ALPHAHERPE SVIRUS_2_(HERPES_SI MPLEX_VIRUS_2) | NC_0017 98.2 | 2443 | 2572 | -3.6327 |
| 5 | ORF_VIRUS | NC_0053 36.1 | 7275 | 7146 | -3.5236 |
| 6 | AICHI_VIRUS_1 | NC_0019 18.1 | 6696 | 6825 | -3.4538 |
| 7 | SALIVIRUS_A | NC_0129 86.1 | 6233 | 6362 | -3.4187 |
| 8 | HEPATITIS_DELTA_VIR US | NC_0016 53.2 | 651 | 780 | -3.415 |
| 9 | HUMAN_GAMMAHERPE SVIRUS_8_(KAPOSI'S_S ARCOMA-ASSOCIATED_HERPES VIRUS) | NC_0093 33.1 | 91041 | 90912 | -3.4138 |
| 10 | HUMAN_ALPHAHERPE SVIRUS_2_(HERPES_SI MPLEX_VIRUS_2) | NC_0017 98.2 | 138425 | 138554 | -3.3986 |
| 11 | ORF_VIRUS | NC_0053 36.1 | 117429 | 117558 | -3.3572 |
| 12 | HUMAN_GAMMAHERPE SVIRUS_4_(EPSTEIN-BARR_VIRUS) | NC_0076 05.1 | 273 | 402 | -3.3558 |
| 13 | SEVERE_FEVER_WITH _THROMBOCYTOPENIA SYNDROME_VIRUS | NC_0434 52.1 | 511 | 382 | -3.3294 |
| 14 | HEPATITIS_GB_VIRUS_ B | NC_0016 55.1 | 1301 | 1430 | -3.3131 |
| 15 | HUMAN_ALPHAHERPE SVIRUS_1_(HERPES_SI MPLEX_VIRUS_1) | NC_0018 06.2 | 124112 | 124241 | -3.3043 |
| 16 | HUMAN_GAMMAHERPE SVIRUS_4_(EPSTEIN-BARR_VIRUS) | NC_0076 05.1 | 923 | 1052 | -3.2867 |
| 17 | HUMAN_GAMMAHERPE SVIRUS_8_(KAPOSI'S_S ARCOMA-ASSOCIATED_HERPES VIRUS) | NC_0093 33.1 | 38265 | 38394 | -3.2081 |
| 18 | HUMAN_GAMMAHERPE SVIRUS_4_(EPSTEIN-BARR_VIRUS) | NC_0076 05.1 | 134293 | 134422 | -3.1646 |
| 19 | HUMAN_BETAHERPES VIRUS_5_(HHV-5_HCMV) | NC_0062 73.2 | 168911 | 168782 | -3.1588 |
| 20 | ORF_VIRUS | NC_0053 36.1 | 132605 | 132734 | -3.1438 |
| 21 | MOLLUSCUM_CONTAGI OSUM_VIRUS_SUBTYP E_1 | NC_0017 31.1 | 140576 | 140447 | -3.1427 |

(continued)

| Rank | Virus Name | NCBI ID | Start | End | log2 RNA/DNA |
|------|-----------|---------|-------|-----|--------------|
| 22 | GREAT_ISLAND_VIRUS _(GIV) | NC_0145 24.1 | 1303 | 1432 | -3.1262 |
| 23 | HUMAN_BETAHERPES VIRUS_5_(HHV-5_HCMV) | NC_0062 73.2 | 29277 | 29148 | -3.0852 |
| 24 | MOLLUSCUM_CONTAGI OSUM_VIRUS_SUBTYP E_1 | NC_0017 31.1 | 99789 | 99660 | -3.057 |
| 25 | PEGIVIRUS_A | NC_0018 37.1 | 3706 | 3835 | -3.0548 |

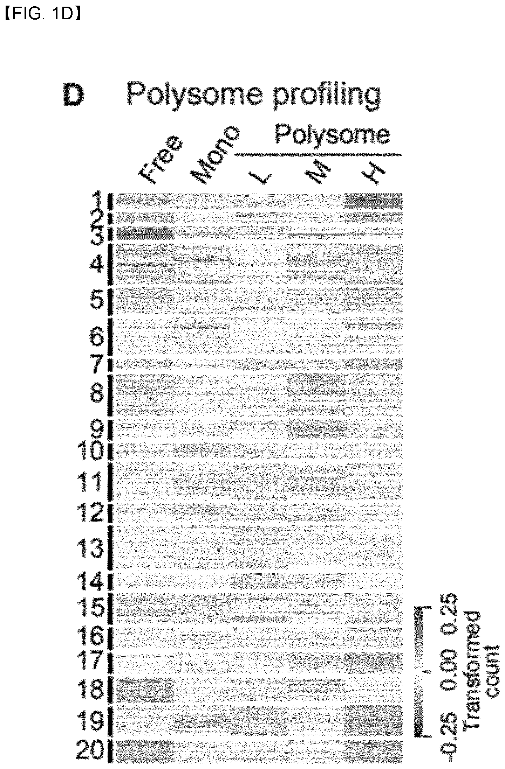

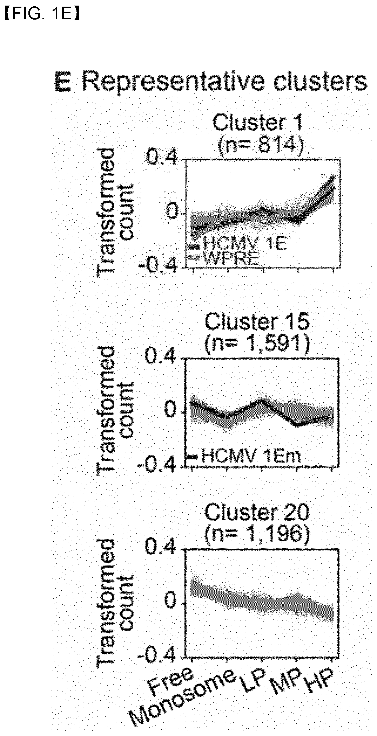

Also, the translational effects of 30,155 segments (29,786 segments with all three barcodes detected) were assessed using the polysome profiling-sequencing data (FIG. 1D). The WPRE and 1E, but not their mutants, were enriched in a heavy polysomal fraction, consistent with their positive effect on translation (FIG. 1E). Identifying 535 upregulating segments and 66 downregulating segments, translation efficiency was estimated using the read ratio between the heavy polysome and free mRNA fractions ($Log_2$(HP/free mRNA) > 0.2) (Table 6). The 30 segments in Table 6 were found to be enriched in the heavy polysome fraction, similar to the positive controls WPRE and HCMV 1E, confirming that they can increase mRNA translation (FIG. 1E).

[Table 6]

| Rank | Virus Name | NCBI ID | Start | End | log2 HP/Free RNA | TILE ID | SEQ. ID |
|------|-----------|---------|-------|-----|------------------|---------|---------|
| 1 | RUBELLA_VIRUS | NC_00 1545.2 | 6626 | 6755 | 0.99 | TILE_ID_08 5-00096 | 51 |
| 2 | RUBELLA_VIRUS | NC_00 1545.2 | 6691 | 6820 | 0.9414 | TILE_ID_08 5-00097 | 52 |
| 3 | HUMAN_ALPHAHE RPESVIRUS_2_(HE RPES_SIMPLEX_VI RUS_2) | NC_00 1798.2 | 1E+05 | 1E+05 | 0.8569 | TILE_ID_13 6-00311 | 53 |
| 4 | YELLOW_FEVER_V IRUS_(YFV) | NC_00 2031.1 | 9011 | 9140 | 0.733 | TILE_ID_09 2-00138 | 54 |
| 5 | HUMAN_GAMMAHE RPESVIRUS_8_(KA POSI'S_SARCOMA-ASSOCIATED_HER PESVIRUS) | NC_00 9333.1 | 90911 | 90782 | 0.6046 | TILE_ID_13 2-00719 | 55 |
| 6 | SAINT_LOUIS_ENC EPHALITIS_VIRUS | NC_00 7580.2 | 2492 | 2621 | 0.5745 | TILE_ID_09 3-00039 | 56 |
| 7 | NY_014_POXVIRUS | NC_03 5469.1 | 1E+05 | 1E+05 | 0.5405 | TILE_ID_14 1-00766 | 57 |
| 8 | GB_VIRUS_C_(GBV -HGV) | NC_00 1710.1 | 2633 | 2762 | 0.5389 | TILE_ID_08 0-00041 | 58 |
| 9 | MIDDLE_EAST_RE SPIRA-TORY_SYND ROME-RELA-TED_CORON AVIRUS_(MERS-COV) | NC_01 9843.3 | 13911 | 14040 | 0.5353 | TILE_ID_12 7-00215 | 59 |
| 10 | HUMAN_BETAHER PESVIRUS_5_(HHV -5_HCMV) | NC_00 6273.2 | 4579 | 4450 | 0.5305 | TILE_ID_14 3-00440 | 17 |
| 11 | MAMMALIAN_ORT HOREOVIRUS_3 | NC_01 3233.1 | 66 | 195 | 0.5258 | TILE_ID_01 2-00018 | 60 |

(continued)

| Rank | Virus Name | NCBI ID | Start | End | log2 HP/Free RNA | TILE ID | SEQ. ID |
|---|---|---|---|---|---|---|---|
| 12 | HUMAN_BETAHER PESVIRUS_5_(HHV -5_HCMV) | NC_00 6273.2 | 49953 | 49824 | 0.525 | TILE_ID_14 3-00553 | 61 |
| 13 | MOLLUSCUM_CON TAGIOSUM_VIRUS _SUBTYPE_1 | NC_00 1731.1 | 80070 | 80199 | 0.5206 | TILE_ID_14 0-00076 | 62 |
| 14 | INFECTIOUS_SPLE EN_AND_KIDNEY_ NECROSIS_VIRUS_ (ISKNV) | NC_00 3494.1 | 12399 | 12528 | 0.5117 | TILE_ID_13 0-00025 | 63 |
| 15 | DENGUE_VIRUS_1 | NC_00 1477.1 | 10548 | 10677 | 0.5086 | TILE_ID_09 0-00162 | 64 |
| 16 | AICHI_VIRUS_1 | NC_00 1918.1 | 8122 | 8251 | 0.5007 | TILE_ID_07 0-00126 | 20 |
| 17 | HUMAN_ASTROVIR US | NC_00 1943.1 | 3938 | 4067 | 0.4892 | TILE_ID_04 7-00061 | 65 |
| 18 | NOROVIRUS_GII | NC_03 9477.1 | 7208 | 7337 | 0.4867 | TILE_ID_06 1-00110 | 66 |
| 19 | SEVERE_ACUTE_R ESPIRATORY_SYN DROME_CORONAV IRUS_2_(SARS-COV-2) | NC_04 5512.2 | 17051 | 17180 | 0.4841 | TILE_ID_12 5-00263 | 67 |
| 20 | SAINT_LOUIS_ENC EPHALITIS_VIRUS | NC_00 7580.2 | 2947 | 3076 | 0.482 | TILE_ID_09 3-00046 | 68 |
| 21 | HUMAN_ASTROVIR US | NC_00 1943.1 | 6018 | 6147 | 0.4713 | TILE_ID_04 7-00093 | 69 |
| 22 | HUMAN_IMMUNOD EFICIENCY_VIRUS _1_(HIV-1) | NC_00 1802.1 | 2558 | 2429 | 0.4658 | TILE_ID_07 9-00238 | 70 |
| 23 | MOLLUSCUM_CON TAGIOSUM_VIRUS _SUBTYPE_1 | NC_00 1731.1 | 2E+05 | 2E+05 | 0.4643 | TILE_ID_14 0-00263 | 71 |
| 24 | HUMAN_CORONAV IRUS_HKU1_(HCOV -HKU1) | NC_00 6577.2 | 5071 | 5200 | 0.4545 | TILE_ID_12 6-00079 | 72 |
| 25 | GREAT_ISLAND_VI RUS_(GIV) | NC_01 4524.1 | 131 | 260 | 0.4473 | TILE_ID_00 2-00135 | 73 |
| 26 | GREAT_ISLAND_VI RUS_(GIV) | NC_01 4524.1 | 261 | 390 | 0.4422 | TILE_ID_00 2-00137 | 74 |
| 27 | INFLUENZA_C_VIR US_(C_ANN_ARBO R_1_50) | NC_00 6310.2 | 456 | 585 | 0.4402 | TILE_ID_00 7-00067 | 75 |
| 28 | HUMAN_BETAHER PESVIRUS_5_(HHV -5_HCMV) | NC_00 6273.2 | 2E+05 | 2E+05 | 0.4344 | TILE_ID_14 3-00424 | 76 |
| 29 | ASTROVIRUS_MLB 1 | NC_01 1400.1 | 2341 | 2470 | 0.4313 | TILE_ID_04 6-00037 | 77 |
| 30 | SEVERE_FEVER_ WITH_THROMBOC YTOPENIA_SYNDR OME_VIRUS | NC_04 3451.1 | 753 | 882 | 0.4303 | TILE_ID_01 5-00062 | 78 |

3. Validation of regulatory elements

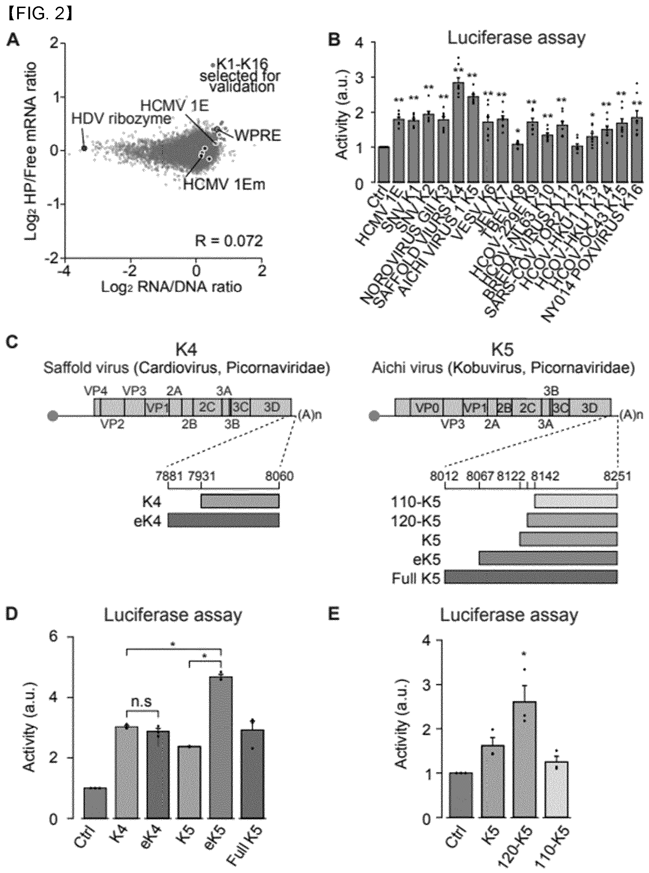

The very weak correlation between the estimated mRNA abundance and translational efficiency suggests that most viral elements influence either mRNA abundance or translation. Nevertheless, some segments were found to affect both aspects. For validation, 16 candidates, not previously studied, which enhanced both RNA abundance and translation were selected (FIG. 2A, Table 7; $Log_2$(HP/free mRNA) > 0.2 and MRL > 4.5). Using 3' UTR reporters and individual luciferase assays, it was confirmed that 15 out of 16 candidates increased luciferase expression with statistical significance ($p < 0.05$) (FIG. 2B).

[Table 7]

| Name | ID | log2 (HP/Free) | MRL | SEQ. ID |
|---|---|---|---|---|
| K1 | TILE_ID_024-00023\|SIN_NOMBRE_ ORTHOHANTAVIRUS | 0.3991 | 5.1267 | 79 |
| K2 | TILE_ID_024-00025\|SIN_NOMBRE_ ORTHOHANTAVIRUS | 0.2156 | 4.5407 | 80 |
| K3 | TILE_ID_061-001091\|NOROVIRUS_GII | 0.3133 | 4.7404 | 81 |
| K4 | TILE_ID_069-00123\|SAFFOLD_VIRUS | 0.4081 | 5.0198 | 82 |
| K5 | TILE_ID_070-00126\|AICHI_VIRUS_1 | 0.5007 | 4.8105 | 20 |
| K6 | TILE_ID_071-00125\|VESICULAR_ EXANTHEMA_OF_SWINE_VIRUS | 0.4166 | 5.0304 | 83 |
| K7 | TILE_ID_095-00164\|JAPANESE_ENCEPHALITIS_VIRUS | 0.3283 | 4.6157 | 84 |
| K8 | TILE_ID_097-00038\|TICK-BORNE_ ENCEPHALITIS_VIRUS | 0.3959 | 4.6477 | 85 |
| K9 | TILE_ID_121-00135\|HUMAN_ CORONAVIRUS_229E | 0.2846 | 4.6149 | 86 |
| K10 | TILE_ID_122-00243\|HUMAN_ CORONAVIRUS_NL63_(HCOV-NL63) | 0.3013 | 4.8697 | 87 |
| K11 | TILE_ID_123-00130\|BREDA_VIRUS | 0.3171 | 4.5876 | 88 |
| K12 | TILE_ID_124-00267\|SARS_ CORONAVIRUS_TOR2 | 0.2225 | 4.5144 | 89 |
| K13 | TILE_ID_126-00030\|HUMAN_ CORONAVIRUS_HKU1_(HCOV-HKU1) | 0.2366 | 4.7599 | 90 |
| K14 | TILE_ID_126-00421\|HUMAN_ CORONAVIRUS_HKU1_(HCOV-HKU1) | 0.2586 | 4.5642 | 91 |
| K15 | TILE_ID_128-00362\|HUMAN_ CORONAVIRUS_OC43_(HCOV-OC43) | 0.2393 | 4.5485 | 92 |
| K16 | TILE_ID_141-00071\|NY_014_POXVIRUS | 0.2049 | 4.5646 | 93 |

The K4 element from the 3' UTR of Saffold virus (GenBank: NC_009448.2, 7,931-8,060) and the K5 element from the 3' UTR of Aichi virus 1 (AiV-1) (GenBank: NC_001918.1, 8,122-8,251) were further investigated (FIG. 2C). Both viruses belong to the family *Picornaviridae*, which have a single-stranded, positive-sense RNA genome encoding a single polypeptide, and the viruses were proteolytically processed into multiple fragments.

Saffold virus and AiV-1 belong to the genus *Cardiovirus* and genus *Kobuvirus*, respectively, and are broadly distributed and poorly investigated viruses that cause relatively mild symptoms, including gastroenteritis.

To map the boundaries of the elements, the extended or truncated segments of K4 and K5 were examined. The extended 180-nt segment of K4 covering the entire 3' UTR of Saffold virus ("eK4," 7,881-8,060) showed similar effects to the original K4 segment, confirming that the 3' terminal 130 nt is sufficient to convey the activity of K4. However, the extended form of K5 ("eK5," 8,067-8,251, 185 nt) further enhanced luciferase expression, outperforming other elements, including the original K5, K4, and the extended K4 (eK4) (FIG. 2D). In addition, a 120-nt segment (8,132-8,251, SEQ ID NO: 95), which is shorter than K5, exhibited higher activity than K5. Notably, K5 ranked as one of the top 25 candidates in both the mRNA abundance and translation screens, suggesting that K5 is a particularly robust element. Truncation experiments on K5 showed that the element exceeding 110-nt at the 3' end (8142-8251) may constitute a minimal K5 element (FIG. 2E). The K5-containing segments increased mRNA levels, and more importantly, the protein levels were consistent with the screening data.

### 4. Characterization of the K5 element

To characterize K5 in more detail, a second round of high-throughput assay was performed on K5 mutants and homologs (FIGS. 3A and 3B). For mutagenesis, single-nucleotide substitutions, single-nucleotide deletions, and two-consecutive-nucleotide deletions were introduced to every position of the 130-nt K5 element (FIG. 3C). In addition, compensatory mutations were introduced that changed the sequences but preserved the predicted duplex structure. Additionally, the loops were substituted for a maximum of two randomly selected bases with different combinations. In total, 1,201 mutants were synthesized, each with three barcodes. After cloning and transfection, mRNA levels relative to the transfected DNA levels were measured to assess the effects of the mutations on mRNA abundance (FIG. 3B).

As shown in FIG. 3D, to quantify the contribution of the specific nucleotide sequence, a "base-identity score" was calculated using the single-base substitution data. Also, "base-pairing score" was calculated based on compensatory mutation data, which indicate the requirement for base pairing in the stem region. As a result, some mutations, particularly those in the first 14 nucleotides, resulted in a modest increase in the mRNA levels (FIG. 3C), suggesting an autoinhibitory activity, which is consistent with the truncation experiments (FIG. 2E). Further, the other variants increased mRNA levels similarly to or higher than K5 (FIG. 3C). In contrast, mutations to the first hairpin (including a pyrimidine-rich terminal loop) and the second hairpin (including a G bulge) substantially reduced mRNA levels, confirming that these hairpins are crucial for the K5 activity (FIG. 3D). These results were consistent with the results from deletion and compensatory mutants.

To investigate the phylogenetic distribution of K5, the 3' UTR segments from 88 picornavirus species (K5 and 87 other picornavirus elements) were included in the secondary screen. Among these picornavirus, 43 kobuvirus segments (Table 8; with at least 59 % homology to K5) upregulated mRNA levels further than the nonfunctional control K5m, which has a deletion in the G bulge in the second hairpin (FIGS. 3D and 3E; Table 8), and upregulated mRNA levels similarly to or higher than K5. This result indicates that K5 is conserved in the genus *Kobuvirus.* Some kobuvirus segments lacking the conserved 3' sequences were less active in our assay. This absence of the 3' sequences may be due to incomplete annotation in the database.

[Table 8]

| rank | des. | NC_id | RNA/DNA ratio | SEQ. ID |
|---|---|---|---|---|
| 1 | Canine kobuvirus US-PC0082, complete genome | JN088541.1 | 1.5851 | 98 |
| 2 | Canine kobuvirus isolate CaKoV AH-1/CHN/2019, complete genome | MN449341.1 | 1.5312 | 99 |
| 3 | Kobuvirus sp. strain 16317x87 polyprotein gene, complete cds | MF947441.1 | 1.5149 | 100 |
| 4 | Kobuvirus sewage Aichi gene for polyprotein, partial cds, strain: Y12/2004 | AB861494.1 | 1.5131 | 101 |
| 5 | Feline kobuvirus isolate 12D240, complete genome | KJ958930.1 | 1.4917 | 102 |
| 6 | Aichivirus A strain Wencheng-Rt386-2 polyprotein gene, complete cds | MF352432.1 | 1.4696 | 103 |
| 7 | Kobuvirus SZAL6-KoV/2011/HUN, complete genome | KJ934637.1 | 1.4508 | 104 |
| 8 | Canine kobuvirus CH-1, complete genome | JQ911763.1 | 1.4502 | 105 |
| 9 | Kobuvirus sp. strain 20724x43 polyprotein gene, partial cds | MF947446.1 | 1.4467 | 106 |
| 10 | Aichivirus A strain rat08/rAiA/HUN, complete genome | MN116647.1 | 1.4388 | 107 |
| 11 | Mouse kobuvirus M-5/USA/2010, complete genome | JF755427.1 | 1.4276 | 108 |
| 12 | Canine kobuvirus strain S272/16, complete genome | MN337880.1 | 1.4176 | 109 |
| 13 | Feline kobuvirus isolate FKV/18CC0718, complete genome | MK671315.1 | 1.4173 | 110 |
| 14 | Kobuvirus sewage Kathmandu isolate KoV-SewKTM, complete genome | JQ898342.1 | 1.4148 | 111 |
| 15 | Feline kobuvirus strain FeKoV/TE/52/IT/13, complete genome | KM091960.1 | 1.4074 | 112 |
| 16 | Aichi virus 1 strain PAK585 polyprotein gene, complete cds | MK372823.1 | 1.3919 | 113 |
| 17 | Canine kobuvirus strain UK003, complete genome | KC161964.1 | 1.3886 | 114 |
| 18 | Kobuvirus dog/AN211 D/USA/2009 polyprotein gene, complete cds | JN387133.1 | 1.3842 | 115 |
| 19 | Aichivirus A strain FSS693 polyprotein gene, complete cds | MG200054.1 | 1.3822 | 116 |

(continued)

| rank | des. | NC_id | RNA/DNA ratio | SEQ. ID |
|------|------|-------|---------------|---------|
| 20 | Kobuvirus sp. strain 20724x41 polyprotein gene, partial cds | MF947445.1 | 1.3768 | 117 |
| 21 | Aichivirus A7 isolate RtMruf-PicoV/JL2014-2 polyprotein gene, complete cds | KY432931.1 | 1.3722 | 118 |
| 22 | Feline kobuvirus isolate FKV/18CC0503, complete genome | MK671314.1 | 1.3677 | 119 |
| 23 | Canine kobuvirus strain CaKoV-26, complete genome | MH747478.1 | 1.3646 | 120 |
| 24 | Feline kobuvirus strain FK-13, complete genome | KF831027.1 | 1.3581 | 121 |
| 25 | Aichi virus strain D/VI2244/2004 polyprotein gene, complete cds | GQ927712.2 | 1.3519 | 122 |
| 26 | Aichi virus isolate Chshc7, complete genome | FJ890523.1 | 1.3312 | 123 |
| 27 | Aichi virus isolate Goiania/GO/03/01/Brazil, complete genome | DQ028632.1 | 1.3282 | 124 |
| 28 | Aichi virus strain D/VI2321/2004 polyprotein gene, complete cds | GQ927706.2 | 1.3236 | 125 |
| 29 | Canine kobuvirus 1 isolate 82 polyprotein mRNA, complete cds | KM068049.1 | 1.3129 | 126 |
| 30 | Aichi virus strain kvgh99012632/2010 polyprotein gene, complete cds | JX564249.1 | 1.2940 | 127 |
| 31 | Canine kobuvirus 1 isolate 75 polyprotein mRNA, complete cds | KM068050.1 | 1.2922 | 128 |
| 32 | Aichi virus strain D/VI2287/2004 polyprotein gene, complete cds | GQ927711.2 | 1.2717 | 129 |
| 33 | Aichi virus isolate BAY/1/03/DEU from Germany polyprotein gene, complete cds | AY747174.1 | 1.2121 | 130 |
| 34 | Canine kobuvirus isolate CaKoV_CE9_AUS_2012 polyprotein gene, complete cds | MH052678.1 | 1.1030 | 131 |
| 35 | Canine kobuvirus 1 isolate B103 polyprotein mRNA, complete cds | KM068051.1 | 1.0241 | 132 |
| 36 | Canine kobuvirus 1 isolate 12D049, complete genome | KF924623.1 | 0.9982 | 133 |
| 37 | Feline kobuvirus strain WHJ-1, complete genome | MF598159.1 | 0.9554 | 134 |
| 38 | Marmot kobuvirus strain HT9, complete genome | KY855436.1 | 0.9545 | 135 |
| 39 | Canine kobuvirus strain CU_101 polyprotein gene, complete cds | MK201777.1 | 0.9292 | 136 |
| 40 | Canine kobuvirus strain CU_716 polyprotein gene, complete cds | MK201779.1 | 0.9197 | 137 |
| 41 | Canine kobuvirus strain CU_53 polyprotein gene, complete cds | MK201776.1 | 0.8912 | 138 |
| 42 | Murine kobuvirus strain TF5WM polyprotein mRNA, partial cds | JQ408726.1 | 0.8689 | 139 |
| 43 | Canine kobuvirus isolate SMCD-59, complete genome | MF062158.1 | 0.8616 | 140 |
| - K5 : RNA/DNA ratio = 1.072033 | | | | |

Outside the *Kobuvirus* genus, most picornaviral 3' UTRs failed to increase mRNA abundance (FIG. 3E). However, there were some exceptions, notably, a segment (SEQ ID NO. 187; RNA/DNA ratio = 1.2433) of Boone cardiovirus 1 (NC_038305.1), which is related to Saffold virus that possesses the positive element K4 (RNA/DNA ratio = 1.514). Both viruses belong to the genus *Cardiovirus.* Thus, K4 and its homologous elements of cardioviruses may constitute another distinct group of conserved regulatory elements. In detail, the underlined nucleotide sequence (nucleotides 7952 to 7988 in NC_009448.2) in the nucleotide sequence of K4 has 78.38 % identity to the corresponding nucleotide sequence (underlined below) in a segment of Boone cardiovirus 1, which is its homolog. Therefore, it can be understood that a homolog, which is a nucleotide sequence within the 3' UTR of a cardiovirus and has at least 70% identity to the nucleotide sequence at positions 7952 to 7988 of the Saffold virus gene, can increase mRNA abundance, similar to K4.

K4

AACATCCTCTCGATCGGATCG<u>CAACGTGTTACCCAGGAATCCACTTGGGTG TACGCGG</u>CCGTTCTGACGTTGGAATTCTGTAGATGAAAGTTAGCTAGGAGCTTTTA ATTGGAAATGAGAACAAAAAAAA (SEQ ID NO: 82)

Underlined: 7952-7988 in NC_009448.2

Boone cardiovirus 1

TTCGGTTGAGCCCCCACCCGGTA<u>CAACGCTTTACCTTAGAAGCCACTAAGG TGTACGCGG</u>TCATCGGGGACCCCTCCTGGCCTTTGGTTTATTGGTGAATTACTAGT TCAGTTAGGTTTTGTTAGTTAGG (SEQ ID NO: 187)

### 5. Enhancement of Gene Expression from Vectors and Synthetic mRNAs by K5

To test whether K5 can function in other molecular contexts, a vector system based on adeno-associated virus (AAV), a single-stranded DNA virus belonging to the *Parvoviridae* family that enables efficient gene delivery with low toxicity for human gene therapy, was used. As shown in FIG. 4, WPRE enhanced gene expression in AAV 35, but its use in AAV was restricted due to its large size (~600 nt) and the limited packaging capacity of AAV (1.7-3 kb).

Minimal K5 (120 nt) or eK5 (185 nt) sequences, along with inactive mutants (K5m and eK5m) and WPRE, were evaluated as controls. These segments were inserted downstream of the EGFP-coding sequences within AAV vectors, and their impact on gene expression was measured (FIG. 4A). As shown in FIGS. 4B and 4C, both K5 and eK5 led to increased GFP expression from AAV vectors under two different transduction conditions. In particular, it was confirmed that the effect of eK5 (~3-fold) was superior to that of WPRE (~2-fold). This demonstrated that eK5 can significantly improve AAV vectors while saving their packaging space.

In addition, the above experiment was repeated using a lentiviral vector. As a result, it was confirmed that, similar to AAV vectors, eK5 also increased GFP expression when using the lentiviral vector (FIG. 10).

*In vitro* transcribed (IVT) mRNA represents another important platform for gene transfer, as exemplified by the COVID-19 vaccines. To test the effect of K5 on IVT mRNAs, luciferase-encoding mRNAs were synthesized with or without functional eK5, as shown in FIG. 4D. These mRNAs contained the cap-1 analog, 3' UTR sequences derived from the pmirGLO vector, and poly(A) tail of 120 nt. The mRNAs were transfected into HeLa cells and incubated up to 72 hours. As shown in FIG. 4E, in the absence of functional eK5, the luciferase levels rapidly declined over time, indicating a shorter lifespan of transfected mRNAs. However, when eK5 was included, the duration of expression drastically increased.

A similar observation was made with another set of IVT mRNAs containing the GFP coding sequences (d2EGFP) and the alpha-globin 3' UTR (GBA), widely used to stabilize mRNAs. As shown in FIGS. 4D and 4F, regardless of its position within the 3' UTR, the inclusion of eK5 substantially increased protein production from these alpha-globin 3' UTR-containing mRNAs. Based on these results, it was confirmed that K5 is active in all tested contexts, including plasmid, AAV vector, and synthetic mRNA, demonstrating its broad regulatory activity and therapeutic potential.

### 6. Induction of mixed tailing via TENT4 by K5

In the time-course experiment using synthetic mRNA transfection, the prolonged protein expression (FIG. 4E) confirmed that K5 acts, at least in part, by increasing mRNA stability in the cytoplasm. Eukaryotic mRNA stability is determined primarily at the deadenylation step. Thus, to understand the mechanism of K5, the poly(A) tail length was monitored using high-resolution poly(A) tail assay (Hire-PAT). Hire-PAT used G/I tailing followed by RT-PCR with a gene-specific forward primer and a reverse primer that binds to the junction between poly(A) and G/I sequences. As shown in FIG. 5A, it was confirmed that K5 increases the steady-state poly(A) tail length of the reporter mRNA. This implies a mechanism involving poly(A) tail regulation, via either inhibition of deadenylation or extension of the poly(A) tail, or both.

To test the possibility that this change involves tail extension catalyzed by terminal nucleotidyl transferases (TENTs), TENTs were depleted, and luciferase assays were performed with K5 reporter constructs. As shown in FIG. 5B, knockdown of TENT4 paralogs (TENT4A and TENT4B) specifically reduced K5 reporter expression, whereas the other TENTs (TENT1, TENT2, TENT3A/B [also known as TUT4 and TUT7], and TENT5A/B/C/D) failed to show significant impact on K5 activity. To further verify the involvement of TENT4, the chemical inhibitor of the TENT4 enzymes, RG7834, and its inactive control R-isomer RO0321 were used. As shown in FIG. 5C, the poly(A) tail of K5 reporter mRNA was shortened specifically by RG7834, confirming that TENT4 is indeed required for K5 function.

TENT4A (also known as PAPD7, TRF4-1, and TUT5) and TENT4B (also known as PAPD5, TRF4-2, and TUT3) extend poly(A) tails with the occasional incorporation of non-adenosine residues, a process known as "mixed tailing". The resulting mixed tail effectively impedes deadenylation, stabilizing the transcript, because the main deadenylase complex, CCR4-NOT, has a preference for adenosine residues. To investigate the direct involvement of mixed tails by measuring the frequency of mixed tails, a modified version of TAIL-seq (named as "gene-specific TAIL-seq(GS-TAIL-seq)") was developed. In detail, RNA was ligated to the 3' adapter conjugated with a biotin and partially fragmented. The 3' end fragments were enriched using streptavidin beads, reverse transcribed with primers binding to the adapter, and then amplified by PCR with a gene-specific forward primer. The sequencing data show that K5 reporter mRNA has non-adenosine residues mainly at terminal and penultimate positions, as expected for mixed tails. As shown in FIG. 5D, the frequency of mixed tailing was reduced after RG7834 treatment, confirming that K5 induces mixed tailing via TENT4. As shown in FIG. 5F, GS-TAIL-seq data also confirmed that the poly(A) tail of K5 reporter is shortened in RG7834-treated cells, corroborating the Hire-PAT data shown in FIG. 5C.

Moreover, as shown in FIG. 5E to 5G, the luciferase activity and mRNA abundance from the K5 and eK5 reporters decreased when RG7834 was added to HeLa and HCT116 cells. The inactive mutants of K5 and eK5 with a single G deletion (K5m and eK5m) were not significantly affected by RG7834, demonstrating the specificity. These results, taken together, support a mechanism where K5 acts through mixed tailing catalyzed by TENT4.

Interestingly, however, it was observed that K5 remains fully active in the absence of ZCCHC14, an adapter protein known to recruit TENT4 to viral RNAs. As shown in FIG. 5G, ZCCHC14 was found to be dispensable for K5 activity in both reporter expression and tail elongation. This lack of ZCCHC14 dependency suggested that there might be a different factor that recognizes K5.

## 7. Identification of a host factor (ZCCHC2) for K5

To identify the potential K5 adapters, the 'RNA-protein interaction detection (RaPID)' method was performed. As shown in FIG. 5H, an IVT mRNA containing eK5 and BoxB elements was transfected into cells stably expressing a λN peptide-fused biotin ligase, BASU. After 16 hours, cells were treated with biotin for 1 hour to allow BASU to biotinylate proteins associated with the bait, followed by cell lysis, streptavidin capture, and mass spectrometry of the biotinylated proteins. As shown in FIG. 5H, among the proteins enriched on the eK5-containing mRNAs compared over the control RNAs lacking eK5, two cytoplasmic proteins with nucleic acid-binding GO terms, ZCCHC2 and DNAJC21, were identified (FIG. 5H, Table 9).

[Table 9]

| ID | Entry | Gene Names | Gene Ontology (molecular function) |
|---|---|---|---|
| ARHGI_ HUMAN | Q6ZSZ5 | ARHGEF18 KIAA0521 | guanyl-nucleotide exchange factor activity [GO:0005085]; metal ion binding [GO:0046872] |
| CALL5_ HUMAN | Q9NZT1 | CALML5 CLSP | calcium ion binding [GO:0005509]; enzyme regulator activity [GO:0030234] |
| CDC16_ HUMAN | Q13042 | CDC16 ANAPC6 | |

(continued)

| ID | Entry | Gene Names | Gene Ontology (molecular function) |
|---|---|---|---|
| CPNE3_HUMAN | O75131 | CPNE3 CPN3 KIAA0636 | calcium-dependent phospholipid binding [GO:0005544]; calcium-dependent protein binding [GO:0048306]; metal ion binding [GO:0046872]; protein serine/threonine kinase activity [GO:0004674]; receptor tyrosine kinase binding [GO:0030971]; RNA binding [GO:0003723] |
| DCD_HUMAN | P81605 | DCD AIDD DSEP | anion channel activity [GO:0005253]; metal ion binding [GO:0046872]; peptidase activity [GO:0008233]; RNA binding [GO:0003723] |
| DIP2B_HUMAN | Q9P265 | DIP2B KIAA1463 | alpha-tubulin binding [GO:0043014] |
| HTSF1_HUMAN | O43719 | HTATSF1 | **RNA binding [GO:0003723]** |
| IRS2_HUMAN | Q9Y4H2 | IRS2 | 1-phosphatidylinositol-3-kinase regulator activity [GO:0046935]; 14-3-3 protein binding [GO:0071889]; insulin receptor binding [GO:0005158]; phosphatidylinositol 3-kinase binding [GO:0043548]; protein domain specific binding [GO:0019904]; protein phosphatase binding [GO:0019903]; protein serine/threonine kinase activator activity [GO:0043539]; transmembrane receptor protein tyrosine kinase adaptor activity [GO:0005068] |
| NPALP_HUMAN | O60287 | URB1 C21orf108 KIAA0539 NOP254 NPA1 | **RNA binding [GO:0003723]** |
| PRP8_HUMAN | Q6P2Q9 | PRPF8 PRPC8 | K63-linked polyubiquitin modification-dependent protein binding [GO:0070530]; pre-mRNA intronic binding [GO:0097157]; RNA binding [GO:0003723]; U1 snRNA binding [GO:0030619]; U2 snRNA binding [GO:0030620]; U5 snRNA binding [GO:0030623]; U6 snRNA binding [GO:0017070] |
| SSF1_HUMAN | Q9NQ55 | PPAN BXDC3 SSF1 | **RNA binding [GO:0003723]; rRNA binding [GO:0019843]** |
| T2EB_HUMAN | P29084 | GTF2E2 TF2E2 | **DNA binding [GO:0003677]; RNA binding [GO:0003723]; RNA polymerase II general transcription initiation factor activity [GO:0016251]** |
| YLPM1_HUMAN | P49750 | YLPM1 C14orf170 ZAP3 | **RNA binding [GO:0003723]** |
| PTMA_HUMAN | P06454 | PTMA TMSA | **DNA-binding transcription factor binding [GO:0140297]; histone binding [GO:0042393]; ion binding [GO:0043167]** |
| ARPIN_HUMAN | Q7Z6K5 | ARPIN C15orf38 | |
| CCD50_HUMAN | Q8IVM0 | CCDC50 C3orf6 | ubiquitin protein ligase binding [GO:0031625] |
| GSDME_HUMAN | O60443 | GSDME DFNA5 ICERE1 | cardiolipin binding [GO:1901612]; phosphatidylinositol-4,5-bisphosphate binding [GO:0005546]; wide pore channel activity [GO:0022829] |
| K1C14_HUMAN | P02533 | KRT14 | keratin filament binding [GO:1990254]; structural constituent of cytoskeleton [GO:0005200] |
| K1C16_HUMAN | P08779 | KRT16 KRT16A | structural constituent of cytoskeleton [GO:0005200] |

| ID | Entry | Gene Names | Gene Ontology (molecular function) |
|---|---|---|---|
| K1C9_HUMAN | P35527 | KRT9 | structural constituent of cytoskeleton [GO:0005200] |
| K2C1_HUMAN | P04264 | KRT1 KRTA | carbohydrate binding [GO:0030246]; protein heterodimerization activity [GO:0046982]; signaling receptor activity [GO:0038023]; structural constituent of skin epidermis [GO:0030280] |
| K2C5_HUMAN | P13647 | KRT5 | scaffold protein binding [GO:0097110]; structural constituent of cytoskeleton [GO:0005200]; structural constituent of skin epidermis [GO:0030280] |
| NAV1_HUMAN | Q8NEY1 | NAV1 KIAA1151 KIAA1213 POMFIL3 STEERIN1 | |
| PDLI7_HUMAN | Q9NR12 | PDLIM7 ENIGMA | actin binding [GO:0003779]; metal ion binding [GO:0046872]; muscle alpha-actinin binding [GO:0051371] |
| CA198_HUMAN | Q9H425 | C1orf198 | |
| DPH5_HUMAN | Q9H2P9 | DPH5 AD-018 CGI-30 HSPC143 NPD015 | diphthine synthase activity [GO:0004164] |
| FABP5_HUMAN | Q01469 | FABP5 | fatty acid binding [GO:0005504]; identical protein binding [GO:0042802]; lipid binding [GO:0008289]; long-chain fatty acid transporter activity [GO:0005324]; retinoic acid binding [GO:0001972] |
| M3K20_HUMAN | Q9NYL2 | MAP3K20 MLK7 MLTK ZAK HCCS4 | ATP binding [GO:0005524]; JUN kinase kinase kinase activity [GO:0004706]; magnesium ion binding [GO:0000287]; MAP kinase kinase kinase activity [GO:0004709]; protein kinase activator activity [GO:0030295]; protein serine kinase activity [GO:0106310]; protein serine/threonine kinase activity [GO:0004674]; ribosome binding [GO:0043022]; RNA binding [GO:0003723]; small ribosomal subunit rRNA binding [GO:0070181] |
| MAGD2_HUMAN | Q9UNF1 | MAGED2 BCG1 | |
| RBGP1_HUMAN | Q9Y3P9 | RABGAP1 HSPC094 | GTPase activator activity [GO:0005096]; small GTPase binding [GO:0031267]; tubulin binding [GO:0015631] |
| TXNL1_HUMAN | O43396 | TXNL1 TRP32 TXL TXNL | disulfide oxidoreductase activity [GO:0015036]; protein-disulfide reductase activity [GO:0015035] |
| WNK1_HUMAN | Q9H4A3 | WNK1 HSN2 KDP KIAA0344 PRKWNK1 | ATP binding [GO:0005524]; chloride channel inhibitor activity [GO:0019869]; phosphatase binding [GO:0019902]; potassium channel inhibitor activity [GO:0019870]; protein kinase activator activity [GO:0030295]; protein kinase activity [GO:0004672]; protein kinase binding [GO:0019901]; protein kinase inhibitor activity [GO:0004860]; protein serine kinase activity [GO:0106310]; protein serine/threonine kinase activity [GO:0004674] |
| DJC21_HUMAN | Q5F1R6 | DNAJC21 DNAJA5 | **RNA binding [GO:0003723]; zinc ion binding [GO:0008270]** |

(continued)

| ID | Entry | Gene Names | Gene Ontology (molecular function) |
|---|---|---|---|
| HORN_HUMAN | Q86YZ3 | HRNR S100A18 | calcium ion binding [GO:0005509]; transition metal ion binding [GO:0046914] |
| MILK1_HUMAN | Q8N3F8 | MICALL1 KIAA1668 MIRAB13 | cadherin binding [GO:0045296]; identical protein binding [GO:0042802]; metal ion binding [GO:0046872]; phosphatidic acid binding [GO:0070300]; small GTPase binding [GO:0031267] |
| NUDT4_HUMAN | Q9NZJ9 | NUDT4 DIPP2 KIAA0487 HDCMB47P | bis(5'-adenosyl)-hexaphosphatase activity [GO:0034431]; bis(5'-adenosyl)-pentaphosphatase activity [GO:0034432]; diphosphoinositol-polyphosphate diphosphatase activity [GO:0008486]; endopolyphosphatase activity [GO:0000298]; inositol-3,5-bisdiphosphate-2,3,4,6-tetrakisphosphate 5-diphosphatase activity [GO:0052848]; inositol-5-diphosphate-1,2,3,4,6-pentakisphosphate diphosphatase activity [GO:0052845]; m7G(5')pppN diphosphatase activity [GO:0050072]; metal ion binding [GO:0046872]; snoRNA binding [GO:0030515] |
| OCRL_HUMAN | Q01968 | OCRL OCRL1 | GTPase activator activity [GO:0005096]; inositol phosphate phosphatase activity [GO:0052745]; inositol-1,3,4,5-tetrakisphosphate 5-phosphatase activity [GO:0052659]; inositol-1,4,5-trisphosphate 5-phosphatase activity [GO:0052658]; inositol-polyphosphate 5-phosphatase activity [GO:0004445]; phosphatidylinositol phosphate 4-phosphatase activity [GO:0034596]; phosphatidylinositol-3,4,5-trisphosphate 5-phosphatase activity [GO:0034485]; phosphatidylinositol-3,5-bisphosphate 5-phosphatase activity [GO:0043813]; phosphatidylinositol-4,5-bisphosphate 5-phosphatase activity [GO:0004439]; small GTPase binding [GO:0031267] |
| PIMT_HUMAN | P22061 | PCMT1 | cadherin binding [GO:0045296]; protein-L-isoaspartate (D-aspartate) O-methyltransferase activity [GO:0004719] |
| RGPD1_HUMAN | P0DJD0 | RGPD1 RANBP2L6 RGP1 | |
| SPR1B_HUMAN | P22528 | SPRR1B | structural molecule activity [GO:0005198] |
| ZCHC2_HUMAN | Q9C0B9 | ZCCHC2 C18orf49 KIAA1744 | **nucleic acid binding [GO:0003676]; phosphatidylinositol binding [GO:0035091]; zinc ion binding [GO:0008270]** |

Orthogonally, the TENT4 complex that could be obtained by *in vitro* RNA-pulldown experiments using HCMV 1E stem-loop (SL2.7) as a bait was examined. As a result, in addition to TENT4A, TENT4B, ZCCHC14, SAMD4A, and K0355, which are known to interact with 1E, ZCCHC2 was also found (FIG. 9). Although the intensity of ZCCHC2 was low and it is not required for 1E activity, ZCCHC2 was enriched specifically in the pull-down experiment, suggesting that ZCCHC2 may be a previously unrecognized component of the TENT4 complex. Notably, ZCCHC2 was the only protein enriched commonly in both RaPID and RNA-pulldown experiments.

To validate the interaction between ZCCHC2 with eK5, western blotting was performed following the RaPID experiment, which detected ZCCHC2 associated with the eK5 bait (FIG. 5I). TENT4A was also enriched, albeit modestly, implying that TENT4A may be less stably associated with eK5 than ZCCHC2.

## 8. Characterization of ZCCHC2

ZCCHC2 is a poorly characterized protein of 126 kDa with long intrinsically disordered regions, a PX domain, and a CCHC-type zinc finger (ZnF) domain (FIG. 6A). ZCCHC2 is distantly related to ZCCHC14 but lacks the SAM domain, which is known to interact with the CNGGN pentaloop in 1E and PRE. The gls-1 protein from *C. elegans* is also predicted to be related to ZCCHC2, although gls-1 lacks the PX or ZnF domains. Gls-1 has been previously shown to interact with GLD-4 that is a homolog of TENT4.

To test if ZCCHC2 binds to TENT4, co-immunoprecipitation experiments were conducted. As shown in FIG. 6B, ZCCHC2 was co-immunoprecipitated with antibodies against TENT4A and TENT4B in HeLa cells but not in TENT4A/B double knockout cells. These interactions were detected under RNase A-treated conditions, indicating an RNA-independent interaction between TENT4 and ZCCHC2. As shown in FIG. 6C, subcellular fractionation revealed that ZCCHC2 localizes in the cytoplasm, suggesting that ZCCHC2 forms a cytoplasmic complex with TENT4. Notably, the TENT4 proteins distribute in both the nucleus and cytoplasm, with TENT4A mainly localized in the cytoplasm and TENT4B primarily in the nucleus. RT-qPCR (RIP-qPCR) using a HeLa cell line stably expressing EGFP with eK5 in the 3' UTR was performed following RNA immunoprecipitation. As shown in FIG. 6D, ZCCHC2 interacted specifically with eK5-containing EGFP mRNA, further corroborating the RaPID and RNA pull-down results shown in FIGS. 5H and 5I. Based on these results, it was confirmed that ZCCHC2 interacts with both eK5 and TENT4.

Next, to investigate the function of ZCCHC2 in K5-mediated regulation, the ZCCHC2 gene in HeLa cells was ablated with CRISPR-Cas9. Using this KO, Hire-PAT assays were conducted to examine poly(A) tail length distribution. As shown in FIG. 6E, the poly(A) tails of the eK5 reporter mRNAs were shortened in ZCCHC2 KO cells compared with those in the parental cells. In contrast, the K5 mutants have short tails in parental cells with no further shortening in ZCCHC2 KO cells. Similar observations were made with the eK5 constructs, confirming that ZCCHC2 is critical for the tail lengthening effect. Moreover, as shown in FIG. 6F, gene-specific TAIL-seq experiments showed that the ZCCHC2 KO resulted in a reduction in mixed tailing, confirming that ZCCHC2 is necessary for mixed tailing of the K5 reporter mRNAs.

Consistently, luciferase assays and RT-qPCR using the eK5 reporters revealed that eK5 can no longer enhance reporter expression in the absence of ZCCHC2. This result was confirmed using the longer eK5 constructs. As shown in FIG. 6G, RG7834 was found to have no significant effect on the eK5 reporter expression in ZCCHC2 KO cells, unlike in parental cells. Based on these results, it was confirmed that ZCCHC2 is a critical factor for K5 and that this function of ZCCHC2 requires TENT4's activity.

To verify the role of ZCCHC2, rescue experiments were performed by transfecting the ZCCHC2-expression plasmid into ZCCHC2 KO cells. As shown in FIG. 6H, ectopic expression of ZCCHC2 increased luciferase expression from the K5 and eK5 constructs, but not from their mutants. Thus, it was confirmed that ZCCHC2 is indeed a key element mediating the function of K5. When a mutation was introduced into the ZnF domain of ZCCHC2, the mutant failed to rescue the KO cells, demonstrating a critical role of this RNA-binding motif. In addition, as shown in FIG. 6A, a deletion mutant lacking the N-terminal 200 amino acids (ΔN), which contains the high similarity region (referred to here as "HS") among ZCCHC2 and its related proteins ZCCHC14 and gls-1, was generated. As shown in FIG. 6I, this ΔN mutant failed to rescue the defect in ZCCHC2 KO cells, indicating an important function of the N terminus of ZCCHC2.

To further confirm the direct activity of ZCCHC2 on the target RNA, tethering experiments were conducted by utilizing a luciferase reporter containing BoxB elements, instead of K5. As shown in FIG. 6J, when the ZCCHC2 protein was tethered through a λN tag, the reporter expression was specifically upregulated. When the TNRC6B protein was attached as a control, the expression decreased. As shown in FIG. 6I and 6K, it was confirmed that the ZCCHC2 ZnF mutant, which was inactive in the rescue experiment, was fully functional when tethered to the reporter RNA through the λN-BoxB system. Based on these results, it was confirmed that ZnF serves solely as an RNA-binding module and is dispensable for activation function.

Next, the specific region of ZCCHC2 responsible for TENT4 recruitment was identified. As shown in FIG. 6A, two deletion mutants of ZCCHC2 with a FLAG-tag were created: one with a C terminus deletion (ΔC, retaining the N-terminus 1-375 a.a) and another with an N terminus deletion (ΔN, containing 201-1,178 a.a). As shown in FIG. 6L, anti-FLAG antibody co-precipitated both TENT4A and TENT4B from cells expressing the full-length and ΔC ZCCHC2 proteins, confirming the interactions between TENT4 and ZCCHC2. This result confirms that the C-terminal part, including the PX and ZnF domains, is not required for TENT4 binding. In particular, as shown in FIG. 6I and FIG. 6L, ΔN failed to interact with TENT4A or TENT4B, suggesting that ZCCHC2 may recruit TENT4 through its N terminus. This N-terminal part contains a HS region, and it was confirmed that the HS region is similar in sequences to the GLD4-binding region in gls-1, a distant homolog of ZCCHC2 in *C. elegans* (FIG. 6A). Thus, it was confirmed that the HS region may constitute a previously undefined conserved domain that mediates protein-protein interactions.

Based on these results, it was confirmed that ZCCHC2 uses its N terminus and C terminus to interact with TENT4 and K5, respectively. As shown in FIG. 7, it was confirmed that these interactions may mediate the recruitment of TENT4 to K5, resulting in mixed tailing. Further, it was confirmed that the elongated poly(A) tail can promote translation by recruiting cytoplasmic poly(A) binding proteins (PABPCs), which is well established to interact with eIF4G, a component of the eukaryotic translation initiation factor complex (eIF4F). Alternatively, but not mutually exclusively, it was confirmed that additional unknown factors may be involved in translational activation induced by K5 and ZCCHC2.

From the foregoing description, it will be apparent to those skilled in the art that the present invention may be implemented in various specific forms without altering its technical concept or essential features. The experimental examples and embodiments described above should therefore be considered illustrative and not restrictive in any way. The scope of the present invention should be interpreted to encompass all modifications and variations that fall within the meaning and scope of the appended claims and their equivalents, rather than being limited to the detailed description provided above.

**EP 4 549 591 A1**

**Claims**

1.  A method for screening a regulatory element for enhancing mRNA translation, the method comprising:

    (a) preparing a plurality of oligonucleotides by tiling a viral genome;
    (b) preparing a pool of vectors, each including one of the oligonucleotides, wherein each vector includes a reporter gene and includes one of the oligonucleotide in a 3' UTR thereof;
    (c) introducing each vector into a cell;
    (d) fractionating the polysomes of the cell into free mRNA, monosome, light polysome (LP), medium polysome (MP), and heavy polysome (HP), performing sequencing, and calculating, for each oligonucleotide, a value of Equation (1) and a mean ribosome load (MRL):

    $$\text{Equation (1)} = \text{Log2 (HP / free mRNA)}$$

    Mean ribosome load (MRL) = 1 x p(Monosome) + 2.5 x p(LP) + 6 x p(MP) + 11 x p(HP) where p(X) is a proportion of sequencing reads for each fraction X, and
    (e) selecting, as a regulatory element for enhancing mRNA translation, an oligonucleotide for which the value of Equation (1) exceeds 0.2 and the MRL exceeds 4.5.

2.  The method of claim 1, further comprising:

    (d)' isolating DNA and RNA from the cell into which the vector has been introduced in process (c), and obtaining, for each oligonucleotide, a value of Equation (2):

    $$\text{Equation (2)} = \text{Log2(RNA/DNA)};$$

    and
    (e)' selecting an oligonucleotide for which the value of Equation (2) exceeds 0.5 as a regulatory element for enhancing RNA stability,
    wherein the regulatory element is a regulatory element for enhancing RNA stability and mRNA translation.

3.  A regulatory element for enhancing mRNA translation, wherein the Equation (1) value defined in claim 1 exceeds 0.2 and the MRL value defined in claim 1 exceeds 4.5.

4.  The regulatory element of claim 3, wherein the regulatory element comprises:

    (i) any one of the nucleotide sequences of SEQ ID NOs: 79 to 93, or an RNA nucleotide sequence thereof; or
    (ii) a nucleotide sequence having at least 90% identity thereto.

5.  The regulatory element of claim 3, wherein the regulatory element comprises:

    (i) the nucleotide sequence of a segment of the Saffold virus genome (NCBI Reference Sequence: NC_009448.2) or an RNA nucleotide sequence thereof wherein the segment comprises more than 120 and up to 190 consecutive nucleotides in the 5' direction from the nucleotide at position 8060 of the Saffold virus genome;
    (ii) a nucleotide sequence having at least 90% identity thereto; or
    (iii) a homolog thereof, wherein the homolog comprises a nucleotide sequence located in the 3' UTR of a cardiovirus genus and having at least 70% identity to nucleotides 7952 to 7988 of the Saffold virus genome.

6.  The regulatory element of claim 3, wherein the regulatory element is capable of further enhancing RNA stability.

7.  A construct comprising a gene encoding a target protein, and the regulatory element of any one of claims 3 to 6 in a 3' UTR thereof.

8.  The construct of claim 7, wherein the target protein is selected from a reporter, a bioactive peptide, an antigen, or an antibody or a fragment thereof.

9.  The construct of claim 7, wherein the construct is an mRNA construct.

10. A vector, comprising the construct of claim 7.

11. A recombinant host cell, comprising the construct of claim 7, or a vector comprising the construct.

12. A composition, comprising: the construct of claim 7; a vector comprising the construct; or a recombinant host cell comprising the construct or the vector.

13. The composition of claim 12, wherein the composition is for preventing or treating a disease; or for preparing an mRNA construct or the target protein.

【FIG. 1A】

【FIG. 1B】

【FIG. 1C】

C    RNA abundance

(Plot of $\log_2$ RNA/DNA ratio versus Rank, with labeled points: HCMV 1E, WPRE, HCMV 1Em, HCMV miRNA, EBV miRNA, HDV ribozyme)

【FIG. 1D】

【FIG. 1E】

E Representative clusters

Cluster 1
(n= 814)

Cluster 15
(n= 1,591)

Cluster 20
(n= 1,196)

【FIG. 2】

【FIG. 3A】

Secondary screen

| Mutants | Homologs |
|---|---|
| Substitution 1-nt Deletion 2-nt Deletion Compensatory | Picornaviruses including 45 Kobuviruses |

↓                    ↓

3,603 oligos          261 oligos

↓

RNA/DNA count

【FIG. 3B】

【FIG. 3C】

Impact of mutagenesis

EP 4 549 591 A1

【FIG. 3D】

【FIG. 3E】

[FIG. 4]

【FIG. 5】

【FIG. 6】

【FIG. 7】

【FIG. 8】

HCMV 1E
stem-loop
(429-451)

【FIG. 9】

【FIG. 10】

## A Lentivirus constructs

## B FACS data after lentivirus infection

【FIG. 11】

# Polysome fractionation

<div align="center">

**INTERNATIONAL SEARCH REPORT**

</div>

| International application No. |
|---|
| **PCT/KR2023/009153** |

| A. | CLASSIFICATION OF SUBJECT MATTER |
|---|---|

**C12Q 1/70**(2006.01)i; **C12Q 1/6806**(2018.01)i; **C12N 15/10**(2006.01)i

According to International Patent Classification (IPC) or to both national classification and IPC

| B. | FIELDS SEARCHED |
|---|---|

Minimum documentation searched (classification system followed by classification symbols)

C12Q 1/70(2006.01); C12N 15/10(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 조절 엘리멘트(regulatory elements), 번역(translation), 안정성(stability), mRNA, 스크리닝(screening), 바이러스(virus), 타일링(tiling), 폴리솜(polysome), 유리 mRNA(free mRNA), 분획(fraction), 평균 리보솜 부하(mean ribosome load)

| C. | DOCUMENTS CONSIDERED TO BE RELEVANT |
|---|---|

| Category* | Citation of document, with indication, where appropriate, of the relevant passages | Relevant to claim No. |
|---|---|---|
| A | US 2022-0064631 A1 (THE BOARD OF TRUSTEES OF THE LELAND STANFORD JUNIOR UNIVERSITY) 03 March 2022 (2022-03-03)<br>See entire document. | 1-13 |
| A | KAROLLUS, A. et al. Predicting mean ribosome load for 5'UTR of any length using deep learning. PLoS Computational Biology. 10 May 2021, vol. 17, no. 5, e1008982, pp. 1-24.<br>See entire document. | 1-13 |
| A | CASTILLO-HAIR, S. et al. Machine learning for designing next-generation mRNA therapeutics. Accounts of Chemical Research. 14 December 2021, vol. 55, pp. 24-34.<br>See entire document. | 1-13 |
| A | LEPPEK, K. et al. Combinatorial optimization of mRNA structure, stability, and translation for RNA-based therapeutics. Nature communications. 22 March 2022, vol. 13, thesis no. 1536, pp. 1-22.<br>See entire document. | 1-13 |

| ☑ Further documents are listed in the continuation of Box C. | ☑ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **25 September 2023** | **25 September 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

International application No.

**PCT/KR2023/009153**

**C.      DOCUMENTS CONSIDERED TO BE RELEVANT**

| Category* | Citation of document, with indication, where appropriate, of the relevant passages | Relevant to claim No. |
|---|---|---|
| A | ZRIMEC, J. et al. Learning the regulatory code of gene expression. Frontiers in Molecular Biosciences. 10 June 2021, vol. 8, thesis no. 673363, pp. 1-28.<br>     See entire document. | 1-13 |

Form PCT/ISA/210 (second sheet) (July 2022)

**INTERNATIONAL SEARCH REPORT**

International application No.

**PCT/KR2023/009153**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

   a. ☑ forming part of the international application as filed.

   b. ☐ furnished subsequent to the international filing date for the purposes of international search (Rule 13*ter*.1(a)),

   ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
Information on patent family members

International application No.

**PCT/KR2023/009153**

| Patent document cited in search report | | | Publication date (day/month/year) | Patent family member(s) | | | Publication date (day/month/year) |
|---|---|---|---|---|---|---|---|
| US | 2022-0064631 | A1 | 03 March 2022 | EP | 4204015 | A2 | 05 July 2023 |
| | | | | US | 11492611 | B2 | 08 November 2022 |
| | | | | US | 2022-0010299 | A1 | 13 January 2022 |
| | | | | US | 2022-0135964 | A1 | 05 May 2022 |
| | | | | US | 2022-0162588 | A1 | 26 May 2022 |
| | | | | US | 2023-0159915 | A1 | 25 May 2023 |
| | | | | WO | 2022-015513 | A2 | 20 January 2022 |
| | | | | WO | 2022-015513 | A3 | 24 February 2022 |
| | | | | WO | 2022-015513 | A4 | 14 April 2022 |
| | | | | WO | 2022-015514 | A1 | 20 January 2022 |
| | | | | WO | 2022-047427 | A2 | 03 March 2022 |
| | | | | WO | 2022-047427 | A3 | 07 April 2022 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **VAISHNAV et al.** *New Biol.*, 1991, vol. 3, 142-150
- **DINGWALL et al.** *EMBO J.*, 1990, vol. 9, 4145-4153
- **KIM et al.** *Nat. Struct. Mol. Biol.*, 2020, vol. 27, 581-588
- **HUANG et al.** *Mol. Cell. Biol.*, 1993, vol. 13, 7476-7486
- **O'LEARY et al.** *Nucleic Acids Res.*, 2016, vol. 44, D733-D745
- **NERI et al.** *Cell*, 2022, vol. 185, 4023-4037
- **PEARSON et al.** *Proc. Natl. Acad. Sci. USA*, 1988, vol. 85, 2444

- **RICE et al.** EMBOSS: The European Molecular Biology Open Software Suite. *Trends Genet.*, 2000, vol. 16, 276-277
- **DEVEREUX et al.** *Nucleic Acids Research*, 1984, vol. 12, 387
- **ATSCHUL et al.** *J. Mol. Biol.*, 1990, vol. 215, 403
- Guide to Huge Computers. Academic Press, 1994
- **CARILLO et al.** *SIAM J. Applied Math*, 1988, vol. 48, 1073